# EUROPEAN PATENT SPECIFICATION

(11) **EP 0 804 456 B1**
(45) Date of publication and mention of the grant of the patent: **21.08.2002**
(21) Application number: 95938726.7
(22) Date of filing: 06.10.1995
(51) Int. Cl.: C07K 14/00, A61K 47/48

(54) **PEPTIDE NUCLEIC ACID CONJUGATES**
PEPTID-NUKLEINSÄURE-KONJUGATE
CONJUGUES D'ACIDES NUCLEIQUES PEPTIDIQUES

(30) Priority: 06.10.1994 US 319411
(43) Date of publication of application: 05.11.1997
(73) Proprietor: ISIS PHARMACEUTICALS, INC., Carlsbad, CA 92008 (US); BUCHARDT, Dorte, 3500 Vaerlose (DK); NIELSEN, Peter Eigil, 2980 Kokkedal (DK); EGHOLM, Michael, Lexington, Massachusetts 02173 (US)
(72) Inventor: NIELSEN, Peter, DK-2980 Kokkedal (DK); EGHOLM, Michael, Lexington, MA 02173 (US); BUCHARDT, Ole +di, . (DK); SONNECHSEN, Soren, Holst, DK-2630 Tastrup (DK); LOHSE, Jesper, DK-2400 Copenhagen N (DK); MANOHARAN, Muthiah, Carlsbad, CA 92009 (US); KIELY, John, San Diego, CA 92130 (US); GRIFFITH, Michael, San Diego, CA 92130 (US); SPRANKLE, Kelly, Vista, CA 92083 (US)
(74) Representative: Hallybone, Huw George
(86) International application number: US9512931
(87) International publication number: WO96011205

(56) References cited:
- WO-A-92/20702
- WO-A-92/20703
- WO-A-93/12129
- WO-A-95/16202
- WO-A-95/32305
- US-A- 5 142 047
- JOURNAL OF THE AMERICAN CHEMICAL SOCIETY, Volume 114, issued 1992, EGHOLM et al., "Recognition of Guanine and Adenine in DNA by Cytosine and Thymine Containing Peptide Nucleic Acids (PNA)", pages 9677-9678.
- JOURNAL OF THE AMERICAN CHEMICAL SOCIETY, Volume 114, issued 1992, EGHOLM et al., "Peptide Nucleic Acids (PNA). Oligonucleotide Analogues with an Achiral Peptide Backbone", pages 1895-1897.
- SCIENCE, Volume 254, issued 06 December 1991, NIELSEN et al., "Sequence-Selective Recognition of DNA by Strand Displacement with a Thymine-Substituted Polyamide", pages 1497-1500.
- SCIENCE, Volume 258, issued 27 November 1992, HANVEY et al., "Antisense and Antigene Properties of Peptide Nucleic Acids", pages 1481-1485.
- JOURNAL OF ORGANIC CHEMISTRY, Volume 56, issued 1991, HUANG et al., "Acyclic Nucleic Acid Analogues: Synthesis and Oligomerization of Gamma,4-Diamino-2-Oxo-1(2H)-Pyrimidinepenta noic Acid and eta,4-Diamino-2-oxo-1(2H)-Pyrimidinehexanoi c Acid", pages 6007-6018.
- JOURNAL OF POLYMER SCIENCE: Part A: Polymer Chemistry, Volume 27, issued 1989, NAGAE et al., "Functional Monomers and Polymers. CLIV.* Application of Nucleic Acid Base Containing Polymers to High Performance Liquid Chromatography", pages 2593-2609.

## Description

### FIELD OF THE INVENTION

This invention is directed to compounds that are not polynucleotides yet which bind to complementary DNA and RNA strands more strongly than the corresponding DNA. In particular, the invention concerns peptide nucleic acids (PNAs) which are functionalized to include covalently bound conjugates.

### BACKGROUND OF THE INVENTION

Oligonucleotides and their analogs have been developed and used in molecular biology in certain procedures as probes, primers, linkers, adapters, and gene fragments. Modifications to oligonucleotides used in these procedures include labeling with non isotopic labels, e.g. fluorescein, biotin, digoxigenin, alkaline phosphatase, or other reporter molecules. Other modifications have been made to the ribose phosphate backbone to increase the resistance to nucleases. These modifications include use of methyl phosphonates, phosphorothioates, phosphorodithioate linkages, and 2'-O-methyl ribose sugar units. Further modifications include those made to modulate uptake and cellular distribution. Phosphorothioate oligonucleotides are presently being used as antisense agents in human clinical trials for the treatment of various disease states, and as antiviral agents. With the success of these oligonucleotides for both diagnostic and therapeutic uses, there exists an ongoing demand for improved oligonucleotide analogs.

Oligonucleotides can interact with native DNA and RNA in several ways. For example, an oligonucleotide may form a duplex with a single stranded nucleic acid, or form a triplex structure by binding to double stranded DNA. However, to form a triplex structure with a double stranded DNA, the cytosine bases of the oligonucleotide must be protonated. Triplexing is therefore pH dependent. P.O.P. Ts'o and associates have used pseudo isocytosine as a permanently protonated analogue of cytosine in DNA triplexing (see Ono, *et al., J. Am. Chem. Soc*., **1991,** *113*, 4032-4033; Ono, *et. al., J. Org. Chem*., **1992**, *57*, 3225-3230). Trapane and Ts'o have also suggested the use of pseudo isocytosine for triplex formation with single-stranded nucleic acid targets. (see, Trapane, *et. al., J. Biomol. Strul. Struct*., **1991**, *8*, 229; Trapane, *et. al., Biophys. J*., **1992,** *61*, 2437; and Trapane, *et. al., Abstracts Conference on Nucleic Acids Medical Applications*, Cancun, Mexico, January 1993). WO 93/05180 discloses substitution of 8-Oxoadenine for protonated cytosine in triplex formation.

Peptide nucleic acids (PNAs) are compounds that in some respects are analogous to oligonucleotides, but which differ in structure. In peptide nucleic acids, the deoxyribose backbone of oligonucleotides has been replaced with a backbone having peptide linkages. Each subunit has attached a naturally occurring or non-naturally occurring base. One such backbone is constructed of repeating units of N-(2-aminoethyl)glycine linked through amide bonds.

PNA binds both DNA and RNA to form PNA/DNA or PNA/RNA duplexes. The resulting PNA/DNA or PNA/RNA duplexes are bound with greater affinity than corresponding DNA/DNA or DNA/RNA duplexes as evidence by their higher melting temperatures (Tm). This high thermal stability has been attributed to the neutrality of the PNA backbone, which does not encounter the charge repulsion present in DNA or RNA duplexes. The neutral backbone of the PNA also renders the Tms of PNA/DNA(RNA) duplexs practically independent of salt concentration. Thus the PNA/DNA duplex offers a further advantage over DNA/DNA duplex interactions which are highly dependent on ionic strength. Homopyrimidine PNAs have been shown to bind complementary DNA or RNA forming (PNA)2/DNA(RNA) triplexes of high thermal stability (*see*, *e.g.*, Egholm, *et al.*, Science, **1991,** *254*, 1497; Egholm, et *al.*, J. Am. Chem. Soc., **1992,** *114*, 1895; Egholm, *et al., J.* Am. Chem. Soc., **1992,** *114*, 9677).

In addition to increased affinity, PNA has also been shown to bind to DNA with increased specificity. When a PNA/DNA duplex mismatch is melted relative to the DNA/DNA duplex there is seen an 8 to 20 °C drop in the Tm. This magnitude of a drop in Tm is not seen with the corresponding DNA/DNA duplex with a mismatch present. *See* Egholm, M., et al., *Nature* **1993** *365* p. 566.

The binding of a PNA strand to a DNA or RNA strand can occur in one of two orientations. The orientation is said to be anti-parallel when the DNA or RNA strand in a 5' to 3' orientation binds to the complementary PNA strand such that the carboxyl end of the PNA is directed towards the 5' end of the DNA or RNA and amino end of the PNA is directed towards the 3' end of the DNA or RNA. In the parallel orientation the carboxyl end and amino end of the PNA are in reverse orientation with respect to the 5'-3' direction of the DNA or RNA.

PNAs bind to both single stranded DNA and double stranded DNA. It has been observed that two strands of PNA can bind to dsDNA. While PNA/DNA duplexes are stable in the antiparallel configuration, it was previously believed that the parallel orientation is preferred for (PNA)₂/DNA triplexes.

The binding of two single stranded pyrimidine PNAs to a double stranded DNA has been shown to occur via strand displacement, rather than by conventional triple helix formation as observed with triplexing oligonucleotides. When PNA strands invade double stranded DNA, one strand of the DNA is displaced and forms a loop on the side of the PNA₂/DNA complex area. The other strand of the DNA is part of the (PNA)₂/DNA triplex structure. The single stranded loop area (known as a D loop) is susceptible to cleavage by enzymes that can cleave single stranded DNA.

A further advantage of PNA compared to oligonucleotides is that their polyamide backbone is not recognized by either nucleases or proteases, and are therefore resistant to degradation by enzymes.

Because of their properties, PNAs are known to be useful in several different applications. Since PNAs have stronger binding and greater specificity than oligonucleotides, they are of great utility as probes in cloning, blotting procedures, and in applications such as fluorescence *in situ* hybridization (FISH). Homopyrimidine PNAs are used for strand displacement in homopurine targets. The restriction sites that overlap with or are adjacent to the D-loop will not be cleaved by restriction enzymes. Additionally, the local triplex inhibits gene transcription. The binding of PNAs to specific restriction sites within a DNA fragment can inhibit cleavage at those sites. This inhibition is useful in cloning and subcloning procedures. Labeled PNAs are also used to directly map DNA molecules by hybridizing PNA molecules having a fluorescent label to complementary sequences in duplex DNA using strand invasion.

PNAs also have been used to detect point mutations in PCR-based assays (PCR clamping). In PCR clamping, PNA is used to detect point mutations in a PCR-based assay, e.g. the distinction between a common wild type allele and a mutant allele, in a segment of DNA under investigation. Typically, a PNA oligomer complementary to the wild type sequence is synthesized and included in the PCR reaction mixture with two DNA primers, one of which is complementary to the mutant sequence. The wild type PNA oligomer and the DNA primer compete for hybridization to the target. Hybridization of the DNA primer and subsequent amplification will only occur if the target is a mutant allele. With this method, the presence and exact identity of a mutant can be determined.

Considerable research is being directed to the application of oligonucleotides and oligonucleotide analogs that bind complementary DNA and RNA strands for use as diagnostics, research reagents and potential therapeutics. For many uses, the oligonucleotides and oligonucleotide analogs must be transported across cell membranes or taken up by cells to express activity.

PCT/EP/01219 (EP-A-618923) describes novel peptide nucleic acid (PNA) compounds which bind complementary DNA and RNA more tightly than the corresponding DNA. It is desirable to append to these compounds groups which modulate or otherwise influence their activity or their membrane or cellular transport. One method for increasing such transport is by the attachment of a pendant lipophilic group. United States application serial number 117,363, filed Sept. 3, 1993, entitled (WO-A-9 506 659) "Amine-Derivatized Nucleosides and Oligonucleosides", describes several alkylamino functionalities and their use in the attachment of such pendant groups to oligonucleosides.

Additionally, United States application serial number 07/943,516, filed Sept. 11, 1992, entitled "Novel Amines and Methods of Making and Using the Same" and corresponding published PCT application WO 94/06815 describe other novel amine-containing compounds and their incorporation into oligonucleotides for, inter alia, the purposes of enhancing cellular uptake, increasing lipophilicity, causing greater cellular retention and increasing the distribution of the compound within the cell.

United States application serial number 08/116,801, filed Sept. 3, 1993, (WO-A-9 506 474) entitled "Thiol-Derivatized Nucleosides and Oligonucleosides" describes nucleosides and oligonucleosides derivatized to include thiolalkyl functionality, through which pendant groups are attached.

There remains a need in the art for stable compounds that bind complementary DNA and RNA to form double-stranded, helical structures which mimic double-stranded DNA, and which are capable of being derivatized to bear pendant groups to further enhance or modulate their binding, cellular uptake, or other activity.

### OBJECTS OF THE INVENTION

It is an object of the invention to provide PNAs having at least one conjugate attached thereto.

It is a further object of this invention to provide PNAs which include alkylamino chemical functionality.

It is another object of this invention to provide PNAs which include alkylthio chemical functionality.

It is a further object of the invention to provide PNAs having improved transfer properties across cellular membranes.

It is another object to provide peptide nucleic acids that include intercalators, nucleic acid cleaving agents, cell surface phospholipids, and/or diagnostic agents.

These and other objects will become apparent from the following description and accompanying claims.

### SUMMARY OF THE INVENTION

The present invention provides PNA conjugates comprising a PNA backbone and a conjugate bound to the PNA either directly or through a linking moiety. The PNA backbone has an amino end, a carboxyl end, and a plurality of amido groups. Nucleobases are tethered to the backbone through internally located amino groups.

The conjugate is bound through the linking moiety to at least one of the backbone, the tether, or the nucleobase. Preferebly, the conjugate is bound to the backbone.

The conjugate can be a terpene, an aromatic lipophilic molecule, a phospholipid, a cell receptor binding molecule, a crosslinking agent, a water soluble vitamin, a lipid soluble vitamin, an RNA/DNA cleaving complex, a porphyrin, or a polymeric compound selected from polymeric amines, polymeric glycols and polyethers provided that the conjugate is not acridine. The conjugate is prefereably bound to the peptide nucleic acid backbone, a tether or a nucleobase through a linking moiety.

Also provided are PNA conjugates of the formula: 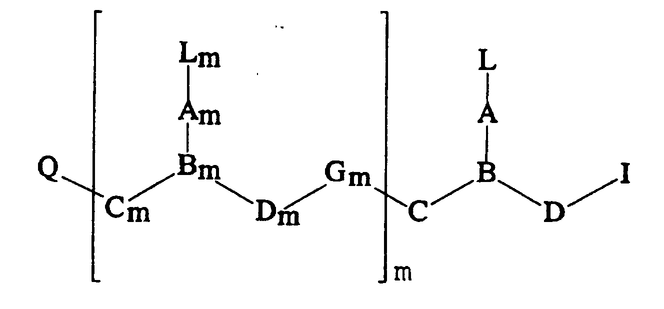 wherein:
m is an ineteger from 1 to about 50;
L and Lₘ independently are R¹²(R¹³)ₐ; wherein:
   R¹² is hydrogen, hydroxy, (C₁-C₄)alkanoyl, a naturally occurring nucleobase, a non-naturally occurring nucleobase, an aromatic moiety, a DNA intercalator, a nucleobase-binding group, a heterocyclic moiety, a reporter ligand, or a conjugate;
   provided that at least one of R¹² is a naturally occurring nucleobase, a non-naturally occurring nucleobase, a DNA intercalator, or a nucleobase-binding group;
   R¹³ is a conjugate; and
   a is 0 or 1;
C and Cₘ independently are (CR⁶R⁷)_{y}; wherein:
   R⁶ and R⁷ independently are hydrogen, a side chain of a naturally occurring alpha amino acid, (C₂-C₆) alkyl, aryl, aralkyl, heteroaryl, hydroxy, (C₁-C₆) alkoxy, (C₁-C₆) alkylthio, a conjugate, NR³R⁴, SR⁵ or R⁶ and R⁷ taken together complete an alicyclic or heterocyclic system;
      wherein R⁵ is hydrogen, a conjugate, (C₁-C₆)alkyl, hydroxy-, alkoxy-, or alkylthio-substituted (C₁-C₆)alkyl; and
      R³ and R⁴ independently are hydrogen, a conjugate, (C₁-C₄)alkyl, hydroxy- or alkoxy- or alkylthio-substituted (C₁-C₄)alkyl, hydroxy, alkoxy, alkylthio or amino;
D and Dₘ independently are (CR⁶R⁷)_{z};
each of y and z is zero or an integer from 1 to 10, wherein the sum y + z is greater than 2 but not more than 10;
Gₘ is independently -NR³CO-, -NR³CS-, -NR³SO-, or -NR³SO₂- in either orientation;
each pair of A-Aₘ and B-Bₘ are selected such that:
   (a) A or Aₘ is a group of formula (IIa), (IIb) or (IIc) and B or Bₘ is N or R³N⁺; or
   (b) A or Aₘ is a group of formula (IId) and B or Bₘ is CH; 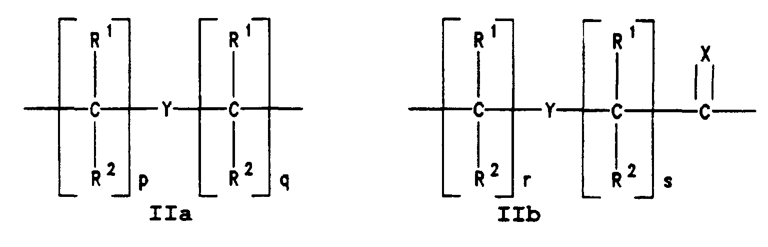 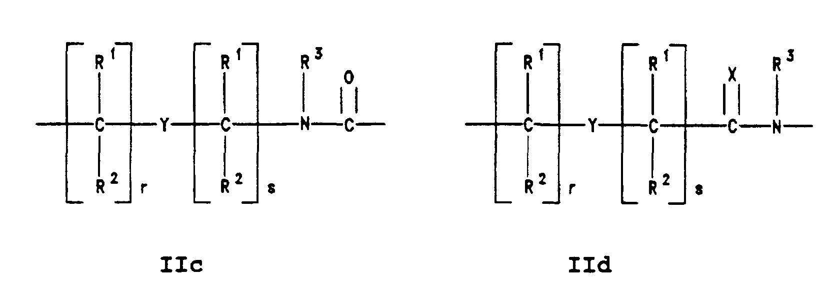
wherein:
X is O, S, Se, NR³, CH₂ or C(CH₃)₂;
Y is a single bond, O, S or NR⁴;
each of p and q is zero or an integer from 1 to 5, the sum p+q being not more than 10;
each of r and s is zero or an integer from 1 to 5, the sum r+s being not more than 10;
R¹ and R² independently are hydrogen, (C₁-C₄)alkyl, hydroxy-substituted (C₁-C₄)alkyl, alkoxy-substituted (C₁-C₄)alkyl, alkylthio-substituted (C₁-C₄)alkyl, hydroxy, alkoxy, alkylthio, amino, halogen or a conjugate;
I is -NR⁸R⁹ or -NR¹⁰C(O)R¹¹; wherein:
   R⁸, R⁹, R¹⁰ and R¹¹ independently are hydrogen, alkyl, an amino protecting group, a reporter ligand, an intercalator, a chelator, a peptide, a protein, a carbohydrate, a lipid, a steroid, a nucleoside, a nucleotide, a nucleotide diphosphate, a nucleotide triphosphate, an oligonucleotide, an oligonucleoside, a soluble polymer, a non-soluble polymer or a conjugate;
Q is -CO₂H, -CO₂R⁸, -CO₂R⁹, -CONR⁸R⁹, -SO₃H, -SO₂NR¹⁰R¹¹ or an activated derivative of -CO₂H or -SO₃H; and
wherein at least one of R¹, R², R³, R⁴, R⁵, R⁶, R⁷, R⁸, R⁹, R¹⁰, R¹¹, R¹² and R¹³ is a conjugate wherein said conjugate is a terpene, an aromatic lipophilic molecule, a phospholipid, a cell receptor binding molecule, a crosslinking agent, a water soluble vitamin, a lipid soluble vitamin, an RNA cleaving complex, a porphyrin, or a polymeric compound selected from polymeric amines, a polymeric glycols and polyethers provided that the conjugate is not acridine; and wherein the conjugate optionally includes a linking moiety. The conjugate is preferably bond to the PNA through a linking moiety.

In preferred embodiments at least one group R¹, R², R³, R⁴, R⁵, R⁶, R⁷, R⁸, R⁹, R¹⁰, R¹¹ R¹² or R¹³ is a conjugate. In other preferred embodiments at least one of group A-Aₘ includes at least one of group R¹, R² or R³.

At least one of groups B-Bₘ or groups G-Gₘ preferably include at least one group R³, and at least one of said groups Q or I preferably include at least one of groups R⁸, R⁹, R¹⁰ and R¹¹. At least one of said groups D-Dₘ, or C-Cₘ preferably include at least one of R³, R⁴, R⁵, R⁶ and R⁷,

In preferred embodiments m is from 1 to about 200, preferably from 1 to about 50, and more preferably from 1 to about 20.

Also provided are compounds having one of the following formulas: 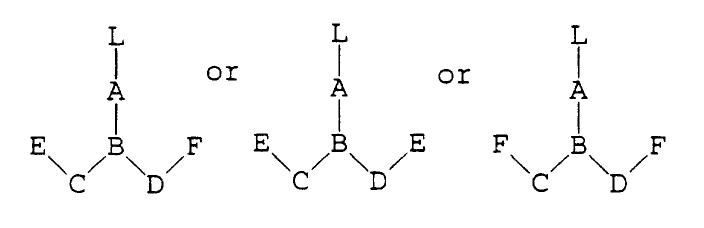 wherein:
L, A, B, C and D are as defined above.
E independently is COOH, CSOH, SOOH, SO₂OH or an activated or protected derivative thereof;
F independently is NHR³ or NPgR³, where Pg is an amino protecting group; and
at least one of R¹, R², R³, R⁴, R⁵, R⁶, R⁷, R¹², and R¹³ is a conjugate as defined above, and wherein the conjugate is preferably bound through a linking moiety.

In preferred embodiments at least one of R³, R⁴, R⁵, R⁶, and R⁷ is a conjugate. In other preferred embodiments at least one group R³ is a conjugate. Preferably, R¹² is a conjugate or R¹³ is a conjugate. In some preferred embodiments at least one group A or group B includes a conjugate, or at least one of said groups C or said groups D include a conjugate.

Also provided are peptide nucleic acid conjugates comprising a plurality of PNA monomers wherein at least one of the PNA monomers has the formula: 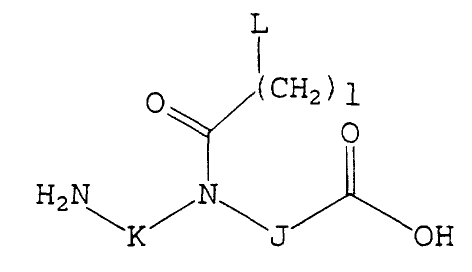 or formula: 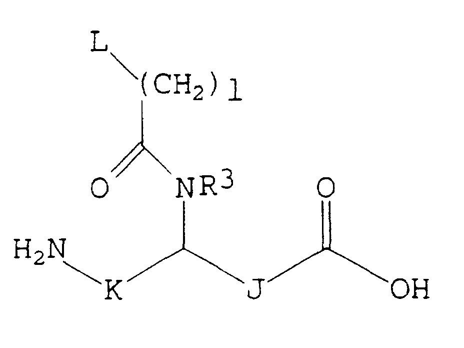 or formula: 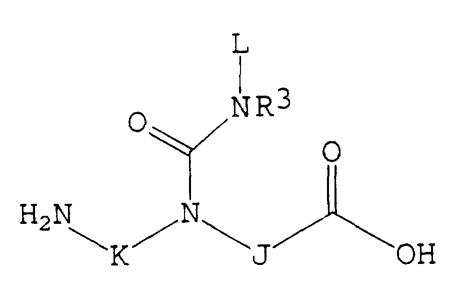 wherein:
L is defined as above;
K is (CR⁶R⁷)_{z} and J is (CR⁶R⁷)_{y} wherein R⁶, R⁷, y and z are defined above; l is an integer from 1 to 5; and
at least one of R¹, R², R³, R⁴, R⁵, R⁶, R⁷, R¹², and R¹³ is a conjugate as defined above. Preferably, at least one of group K, group J or R³ includes a conjugate, preferably bound through a linking moiety.

### BRIEF DESCRIPTION OF THE FIGURES

The numerous objects and advantages of the present invention may be better understood by those skilled in the art by reference to the accompanying figures, in which:
Figure 1 is a reaction schematic depicting the exemplary functionalization of the 2-aminoethyl portion of a PNA backbone at the 1-position.
Figure 2 is a reaction schematic depicting the exemplary functionalization of the 2-aminoethyl portion of a PNA backbone at the 2-position.
Figure 3 depicts exemplary routes for chemical alteration of monomer synthons of the invention.
Figure 4 is a reaction schematic depicting alternate exemplary routes useful in the practice of the invention.
Figure 5 is a reaction schematic depicting the exemplary functionalization of the OH group of an L-serine portion of a PNA backbone.
Figure 6 is a reproduction of an electrophoresis gel pertinent to Example 111A, showing that PNA NTA Fe2⁺ is an effective oligonucleotide cleavage agent.
Figure 7 is a reproduction of an electrophoresis gel pertinent to Example 111B, showing that PNA NTA Fe2⁺ binds to the target by PNA-DNA triplex strand displacement.

### DETAILED DESCRIPTION OF THE INVENTION

This invention is directed to novel peptide nucleic acids having at least one conjugate group. Each PNA monomer or polymer has a backbone which is the linear portion of the PNA. The backbone is normally defined from an amino (N) terminus or deravative thereof to a carboxyl (C) terminus or deravative thereof. In some preferred embodiments the backbone has an N terminal end, a C terminal end, a number of amide linkages which is equal to one less than the number of monomer units in the PNA, and a number of internal substituted amine groups which is equal to the number of monomers in the PNA.

The N terminal or an internal amide nitrogen of the PNA backbone is separated from the nearest substituted amino group by a number of methylene groups which may bear substituent groups. In preferred embodiments there are two such methylene groups. The substituted amino group is bound to a tether, which is in turn bound to a ligand. The ligand is typically a nucleobase.

The substituted amino group is separated from the C terminal carboxyl group or an internal peptidyl carbonyl group of the PNA backbone by one or more methylene groups which may bear substituent groups. In preferred embodiments there is one such methylene group. Thus, in these preferred embodiments the PNA backbone of n monomeric units has the structure:

H-[HN-(CH₂)₂-N(tether-nucleobase)-(CH₂)-CO]ₙ-OH

wherein the methylene groups optionally bear substituent groups in place of hydrogen.

Monomers and oligomers of the present invention include at least one conjugate attached to the N terminal end, C terminal end, a backbone methylene (inclusive of all methylenes in the backbone), an internal substituted amine, a tether, a ligand or an amide linkage between monomers in an oligomer.

As used herein the term nucleobase includes naturally occurring heterocyclic bases such as adenine, guanine, thymine, cytosine and uracil, and also non-naturally occurring nucleobase analogs, homologs and modified nucleobases such as those bearing removable protecting groups. Some representative nucleobase ligands and illustrative synthetic ligands are shown in Figure 2 of WO 92/20702.

As will be recognized, a variety of PNA conjugates can be prepared according to the present invention. Representative conjugates can be formed from homopolymeric PNA strands or heteropolymeric PNA strands (*e.g.*, chimeras of PNA-DNA or PNA-RNA). Each PNA strand or PNA portion of a chimeric strand preferably includes a plurality of ligands, L (typically nucleobases) linked to a backbone via attachment at the position found in nature, *i.e.*, position 9 for adenine or guanine, and position 1 for thymine or cytosine. Alternatively, the ligand can be a non-naturally occurring nucleobase (a nucleobase analog), another base-binding moiety, an aromatic moiety, (C₁-C₄)alkanoyl, hydroxy or hydrogen. L also can be further substituted with a conjugate attached through an optional linking group. In some preferred embodmients L is a conjugate. In monomer synthons, L can be blocked with protecting groups, as illustrated in Figure 4 of WO 92/20702.

The term "conjugate" as used herein includes a terpene, an aromatic lipophilic molecule, a phospholipid, a cell receptor binding molecule, a crosslinking agent, a water soluble vitamin, a lipid soluble vitamin, an RNA cleaving complex, a porphyrin, and polymeric compounds such as polymeric amines, polymeric glycols and polyethers provided that the conjugate is not acridine. PNAs of the present invention include at least one conjugate attached directly or through an optional linking moiety. When so derivatized, the PNA is useful, for example, as a diagnostic or therapeutic agent, to render other properties to a complementary nucleic acid in a test structure or to transfer a therapeutic or diagnostic agent across cellular membranes. Such a diagnostic or therapeutic agent is formed from an oligomeric compound of the invention wherein the oligomeric compound includes monomeric units bearing natural or non-natural occurring nucleobases in a sequence that is complementary to and will specifically hybridize with a region of an RNA or DNA of interest. Thus, an oligomeric compound of the invention is "functionalized" to include a conjugate attached to the oligomeric compound via an optional linking moiety. For the purpose of identification, such a functionalized oligomeric compound can be characterized as a substituent-bearing (*e.g*., steroid-bearing) oligomeric compound. Such oligomeric compounds will have at least one conjugate attached thereto to modulate their activity.

The oligomeric compounds of the present invention may be useful in binding to various other target molecules. Target molecules of the present invention can include any of a variety of biologically significant molecules. Target molecules can include but are not limited to nucleic acids, carbohydrates, glycoproteins or other proteins.

The PNA oligomers and monomer synthons of the invention are constructed such that conjugates may be covalently bound thereto. In one aspect of the invention conjugates are molecular entities appended to PNAs which serve to modulate, optimize, alter or otherwise affect the activity, transport, uptake or longevity of the desired PNA.

For the purposes of this invention the term "modify" shall mean to change properties by addition to, substraction from, cleavage of or otherwise, such that which results is intrinsically different from that which is modified. The term "modulate" shall mean to change (*i.e.*, increase or decrease) the magnitude of a property.

A PNA monomer can be functionalized and derivatized to include a conjugate, and the resulting conjugated monomer can be incorporated into a PNA oligomer. Alternatively, one or more conjugates can be incorporated into an oligomeric PNA that has been functionalized at predetermined positions. It will be apparent to those skilled in the art that a wide range of chemistries for the attachment of conjugates are amenable to the present invention.

Tether A can be a wide variety of groups such as -CR¹R²C(O)-, -CR¹R²C(S)-, -CR¹R²C(Se)-, -CR¹R²C(O)N(R³)-, -CR¹R²C(CH₂)- and -CR¹R²C[C(CH₃)₂]-, where R¹, R² and R³ are as defined above. Preferably, A is methylenecarbonyl (-CH₂CO-), amido (-CONR³-), or, taken together with B when B is R³N⁺, ureido (-N⁺R³CONR³-). A also can be a longer chain moiety such as propanoyl, butanoyl or pentanoyl, or a corresponding derivative wherein O is replaced by another moiety for X or the chain is substituted with R¹R², or contains Y where Y is a heteroatom. Further, A can be a (C₂-C₆)alkylene chain, a (C₂-C₆)alkylene chain substituted with R¹R² or contains Y where Y is a heteroatom. In some embodiments A is a single bond.

In one preferred embodiment B is a nitrogen atom, thereby presenting the possibility of an achiral backbone. B can also be CH or R³N⁺, where R³ is as defined above.

In a preferred embodiment C is -CR⁶R⁷- or a two carbon unit, *i.e.* -CHR⁶CHR⁷- or -CR⁶R⁷CH₂-, where R⁶ and R⁷ are as defined above. R⁶ and R⁷ also can be a heteroaryl group such as, for example, pyrrolyl, furyl, thienyl, imidazolyl, pyridyl, pyrimidinyl, indolyl, or can be taken together to complete an alicyclic system such as, for example, 1,2-cyclobutanediyl, 1,2-cyclopentanediyl or 1,2-cyclohexanediyl. C can also be used as a point of attachment of a conjugate e.g. at R⁶ and or R⁷. In some preferred embodiments G is -CONR³-.

In monomeric embodiments F can be NHR³ or NPgR³ where Pg is an amino protecting group, and E can be CSOH, SOOH, SO₂OH or an activated derivative thereof. In polymeric embodiments groups E and F are activated and chemically combined to produce group G, which therefore can be -CSNR³-, -SONR³- or -SO₂NR³-. The activation can, for example, be achieved using an acid anhydride or an active ester derivative, wherein hydroxy in the groups represented by E is replaced by a leaving group suited for generating the growing backbone.

In some cases it may be useful to attach conjugates at either terminus (Q, I) to modulate the binding characteristics of the resulting PNAs. Representative conjugates include DNA intercalators, which improve dsDNA binding and increase cellular uptake, and basic groups such as lysine or polylysine, which strengthen the binding of the PNA by electrostatic interaction. Other moieties can be located on non-terminal positions. Oligonucleotides and/or oligonucleosides can be covalently bound to terminal positions Q or I to form chimeras containing PNA portions and oligonucleotide and/or oligonucleoside portions. Nucleosides and/or nucleotides (mono, di or tri-phosphates) also can be attached to the terminal positions.

Figure 1 is a reaction schematic depicting the exemplary functionalization of the 2-aminoethyl portion of a PNA backbone at the 1 position. Starting with the commercially available (O-benzyl)Boc-Serine, the acid is protected as the methyl ester using diazomethane and MeOH, yielding the fully protected Compound **30A**. Compound **30A** is reduced at the ester to give the Compound **30B** having a free hydroxyl which is converted to the protected amine Compound **30C** using standard Mitsunobo chemistry. Compound **30C** is reacted with methyl bromoacetate to form a monomeric backbone unit which is further reacted with a chlorocarbonyl functionalized nucleobase or other ligand to form the functionalized monomer with sidechain protected as an O-benzyl. The phthalimido group is removed and the free amine is protected with a BOC group, and the monomer is attached to MBHA Lysine resin to yield the initial monomer attached to the solid support. The support-bound monomer can then be deprotected using TFA, and reacted with a second functionalized monomer or unfunctionalized monomer to give the dimer. The process is repeated in an iterative fashion until the desired PNA is assembled.

Figure 2 is a reaction schematic depicting the exemplary functionalization of the 2-aminoethyl portion of a PNA backbone at the 2-position. Compound **30C** is deprotected with hydrazine and ethanol to give Compound **34A**, which is further reacted with methyl bromoacetate to yield the functionalized backbone Compound **34B.** Compound **34B** is reacted with an acetyl chloride activated nucleobase or other ligand to form the functionalized monomer with the sidechain protected as a benzyl ether. Compound **34** results when 1-thyminyl-acetylchloride is used as the acetyl chlorocarbonyl functionalized nucleobase. Compound **34** is then attached to a suitable resin as shown in Figure 1. The support-bound monomer is then deprotected with TFA, and reacted with additional monomers to yield the desired PNA chain.

Figure 3 depicts the transformation of PNA monomer sidechains. Compound **30** is converted from the phthalimido protected amine to the Boc protected amine using hydrazine and Boc₂O/NaOH, and the benzyl protected hydroxyl is deprotected by hydrogenolysis to yield Compound **35**, which may participate in activation and displacement reactions typical of hydroxyl groups. Thus, PNA oligomer and monomer synthons of the invention may be functionalized with the many chemical species which are useful in the derivitization of DNA and RNA 2'- and 3'-hydroxyls.

Thus, linking moieties attached to PNA monomers of the invention can be modified through a variety of processes, and may participate in linkages with a wide variety of conjugates or conjugates that have themselves been functionalized with a linking moiety.

Compound **35** may also be oxidized to the carbonyl (aldehyde) compound **37A**. Compound **37A** may participate in Schiff's base linkages, which may be stabilized by reductive amination with, for example, sodium borohydride. Compound **37A** may also be further functionalized via enolate condensations, organometallic reactions, stabilized Wittig reactions, and other reactions of active carbonyl groups which will be readily apparent to those skilled in the art.

Figure 4 depicts an alternate route to the functionalized backbone. The commercially available BOC-serine is reacted with triphenylphosphine/DEAD and then TFA/TsOH to produce Compound **46A.** Compound **46A** is treated with Gabriel's reagent to give Compound **46B**. Protection of the amine function as the phthalimide and esterification with ethanol gives Compound **46C**. Deprotection with TFA gives Compound **46D**, which can be used in the preparation of PNA monomers functionalized at the 2 position of the 2-aminoethyl portion of the monomer, as shown in Figure 1. Deprotection with hydrazine gives Compound **46E**, which can be used in the preparation of PNA monomers functionalized at the 1 position of the 2-aminoethyl portion of the monomer, as shown in Figure 2.

It will be seen that selective deprotection of Compound **46C** with either hydrazine or TFA allows the synthesis of monomers functionalized at position 1 or 2 of the 2-aminoethyl portion of the monomer.

Functionalization of the backbone at another position is illustrated in Figure 5. 3-(Boc-amino)-1,3-propanediol is converted to N-Boc-aminoacetaldehyde Compound **39A** via periodate oxidation. Treatment of Compound **39A** according to the procedures of Method A, with glacial acetic acid, and sodium triacetoxyborohydride will give the intermediate Compound **39B**. Compound **39B** can have a variety of different functionalities attached to the secondary amino group in a like manner to Compound **30D** and **34B**. Compound **39B** is further reacted with 1-carboxymethylthyminyl (compound **3**) in 1N NaOH to give Compound **39.** The benzyl-protected serine hydroxyl of compound **39** is deprotected according ot the procedures of Example **42**, and oxidized to a formyl group as per the procedures of Example **43**. The formyl compound is further functionalized to yield the aminoethyl compound as per the procedures of Example **44**. Compound **44** can be treated in a number of different ways to yield other compounds of the invention.

In one aspect of the present invention Compound **44** is treated with a protecting group, incorporated into a PNA oligomer, deprotected and further functionalized with a conjugate that may include an optional linking moiety. In another aspect of the present invention Compound **44** is functionalized with a tether and then incorporated into a PNA oligomer. After oligomerization the protecting group is removed and the tether is reacted with a conjugate that may include an optional linking moiety. In a further aspect of the invention the conjugate which may include an optional linking moiety is attached to Compound **44** prior to oligomerization. It will be understood by those skilled in the arts that many variations of the methods and techniques described above are amenable to the present invention.

It will also be seen that for the PNAs of the invention it may be advantageous to incorporate one or more functionalized monomers into the chain. The monomers may be independently functionalized at position 1 or 2 of the 2-aminoethyl portion of the monomer as illustrated above or at some other position e.g. O-lysine as illustrated above.

The term PNA oligomer as used herein means linked PNA monomers. The monomers that make up the oligomers are further referred to as synthons. A preferred linkage is an amide linkage between consecutive PNA monomers. Conjugates may be attached to a given PNA by a chemical bond to the PNA backbone as illustrated above, to ligand L, or to tether A. The term "PNA backbone" as used herein is intended to mean the linear polymeric chain of the PNA.

The amino acids which form the backbone can be identical or different. Those based on 2-aminoethylglycine are especially well suited to the purpose of the invention. Another backbone is the 2-aminoethylserine benzyl ether. Preferred are those backbones which are functionalized to include linking moieties capable of participating in linkages to conjugates, or to other linking moieties which are in turn attached to conjugates.

Thus, monomer synthons according to the invention may be prepared with linking moieties contained therein, and the desired conjugate group may be bound to the linking moiety either before or after assembly into a PNA oligomer. It will be seen that not every monomer synthon need be functionalzed. However, if advantageous, all the monomers in a given PNA oligomer may be so functionalized.

Conjugates are attached to a linking moiety, a PNA monomer, or a PNA oligomer by the formation of a chemical bond from the reaction of two functional groups. Representative functional groups for formation of the chemical bond include but are not limited to hydroxy, formyl, carboxy or other active carbon species, amino, substituted amino, thio, sulfoxo, sulfono, sulfinic or sulfate ester, or other species similarly able to participate in a chemical linkage with a conjugate group. Functional groups also include those which are protected or "masked." Protecting groups are known per se as chemical functional groups that can be selectively appended to and removed from functionalities such as amine groups and thiol groups. These groups are present in a chemical compound to render such functionality inert to chemical reaction conditions to which the compound is exposed. *See, e.g.*, Greene and Wuts, *Protective Groups in Organic Synthesis*, 2d ed, John Wiley & Sons, New York, 1991.

In some instances it is advantageous to link conjugate groups to PNA oligomers or monomer synthons through a linking moiety. Representative linking moieties useful in the practice of the invention are disclosed in United States application serial number 08/116,801, filed Sept. 3, 1993, entitled "Thiol-Derivatized Nucleosides and Oligonucleosides" and United States application serial number 117,363, filed Sept. 3, 1993, entitled "Amine-Derivatized Nucleosides and Oligonucleosides", the disclosures of which are hereby incorporated by reference.

Thus, the monomer synthons (and therefore ultimately the PNA oligomers) of the invention may be constructed containing any one of several functionalities for the linking of conjugate groups, or linkers attached to conjugate groups (i.e., carboxy, formyl, thio, hydroxy, amino and the like). Those skilled in the art will appreciate that the PNA monomer and oligomers of the invention are capable of conjugation to a wide variety of linking functionalities. For example, to attach a conjugate containing a free amino group to a PNA monomer synthon or PNA oligomer, one may functionalize monomers to contain a carboxyl group, and create an amide linkage using standard chemistries. Similarly, in some instances it might be advantageous to attach the amino group through a PNA monomeric unit functionalized with a formyl moiety by reductive amination as described in the Example **85**. In general, the choice of a particular functional group on a monomer synthon will be driven by the available functional groups on the conjugate group or linking molecule. Those skilled in the art will be able to determine the optimal match of functionalities by application of standard principles.

One aspect of the present invention is directed to PNAs that bear at least one thiol-containing linking moiety at a PNA backbone, tether or ligand position previously described. Representative thiol linking moieties useful for attaching a conjugate to PNA backbone, ligand or tether positions are -NH-(CH₂)₆-S-H and -CH₂-O- (CH₂)₆-S-H where H can be optionally replaced with a protecting group. It can readily be seen that a large number of variations on these linking moieties are possible. Numerous thiol protecting groups are known in the art, including, but not limited to, the triphenylmethyl (trityl; Tr) and S-t-butyl, S-p-nitrobenzyl, and S-p-methoxy-benzyl *(see, e.g.,* Greene and Wuts, Protective Groups in Organic Synthesis, 2d edition, John Wiley & Sons, New York, 1991).

Preferred linker groups according to the invention include an alkyl moiety independently selected to having 1 to about 12 carbon atoms or alkyl groups interspaced with suitable heteroatoms including oxygen, sulfur and nitrogen to form polyethers, polythioethers or polyalkylamines. The term "alkyl" is intended to include straight chain and branched hydrocarbons. In such linkers, the preferred length of these hydrocarbons is 1 to about 7 carbon atoms. Other linkers include heterobifunctional and homobifunctional linkers.

For the purposes of this invention the terms "reporter molecule" and "reporter enzyme" are inclusive of those molecules or enzymes that have physical or chemical properties that allow them to be identified in gels, fluids, whole cellular systems, broken cellular systems and the like utilizing physical properties such as spectroscopy, radioactivity, colorimetric assays, fluorescence, and specific binding. Particularly useful as reporter molecules are biotin, rhodamine, coumarin and dye molecules including fluorescein dyes. Particularly useful as reporter enzymes are alkaline phosphatase and horseradish peroxidase.

Proteins and peptides are utilized in their usual sense as polymers of amino acids. Normally peptides comprise such polymers that contain a smaller number of amino acids per unit molecule than do the proteins. Particularly useful as peptides and proteins are sequence-specific peptides and proteins including phosphodiesterase, peroxidase, phosphatase and nuclease proteins. Such peptides and proteins include but are not limited to SV40 peptide, RNase A, RNase H and Staphylococcal nuclease.

Lipophilic molecules include naturally-occurring and synthetic aromatic moieties such as aromatic hydrocarbons such as benzene, perylene, phenanthrene, anthracene, naphthalene, pyrene, chrysene, and naphthacene.

Intercalators are polycyclic aromatic moieties that can insert between adjacent base pairs without affecting normal Watson-Crick base pairing. Representative intercalators are pyrene, phenanthroline, phenazine, phenanthridine, anthraquinone, acridine, and these and other examples are further disclosed by Manoharan, M., Antisense *Research and Applications,* Crooke and Lebleu, eds., CRC Press, Boca Raton, 1993. Pyrenes include pyrene and other pyrene-based carboxylic acids that can be conjugated using standard protocols. Intercalators of the present invention also include hybrid intercalators. One example of a hybrid intercalator is the photonuclease/intercalator 6-[[[9-[[6-(4-nitrobenzamido)hexyl]amino]acridin-4-yl]carbonyl]amino]hexanoyl-pentafluorophenyl ester. This compound has two noteworthy features: an acridine moiety that is an intercalator and a p-nitro benzamido group that is a photonuclease.

Cell receptor binding molecules according to the invention are vitamins and carbohydrate moieties for which specific receptors exist within a cell. Representative cell receptor binding molecules are disclosed by Application Serial No. PCT/US92/09196, filed October 23, 1992, the contents of which are incorporated herein by reference.

Crosslinking agents are moieties that can effect intrastrand or interstrand covalent binding of RNA and/or DNA. Representative crosslinking agents are disclosed in International Patent Application Serial No. PCT/US93/02059, filed March 5, 1993, which is incorporated herein by reference. Peptide nucleic acids are disclosed herein and by International Patent Application WO 92/20702, published November 26, 1992.

Crosslinking agents of the present invention include photo-crosslinking agents. One group of photo-crosslinking agents is the aryl azides e.g. N-hydroxysuccinimidyl-4-azidobenzoate (HSAB) and N-succinimidyl-6(-4'-azido-2'-nitrophenyl-amino)hexanoate (SANPAH). Aryl azides conjugated to PNA will effect crosslinking with nucleic acids and proteins upon irradiation. They will also crosslink with carrier proteins (such as KLH or BSA), raising an antibody against the oligonucleotides.

Vitamins according to the invention generally can be classified as water soluble or lipid soluble. Water soluble vitamins include thiamine, riboflavin, nicotinic acid or niacin, the vitamin B₆ pyridoxal group, pantothenic acid, biotin, folic acid, the B₁₂ cobamide coenzymes, inositol, choline and ascorbic acid. Lipid soluble vitamins include the vitamin A family, vitamin D, the vitamin E tocopherol family and vitamin K (and phytols). The vitamin A family, including retinoic acid and retinol, are absorbed and transported to target tissues through their interaction with specific proteins such as cytosol retinol-binding protein type II (CRBP-II), Retinol-binding protein (RBP), and cellular retinol-binding protein (CRBP). These proteins, which have been found in various parts of the human body, have molecular weights of approximately 15 kD. They have specific interactions with compounds of vitamin-A family, especially, retinoic acid and retinol.

The vitamin A family of compounds can be attached to PNA oligomers of the invention via acid or alcohol functionalities found in the various family members. For example, conjugation of an N-hydroxy succinimide ester of an acid moiety of retinoic acid to an amine function of a PNA or a linker attached to a PNA results in linkage of vitamin A to the PNA via an amide bond.

α-Tocopherol (vitamin E) and the other tocopherols (beta through zeta) can be conjugated to PNAs to enhance uptake because of their lipophilic character. Also, the lipophilic vitamin, vitamin D, and its ergosterol precursors can be attached to PNAs through their hydroxyl groups by first activating the hydroxyl groups to, for example, hemisuccinate esters. Conjugation then is effected to an aminolinker from the PNA, or to the PNA backbone through the other suitable functional groups described herein. Other vitamins that can be conjugated to PNAs or PNA aminolinkers through hydroxyl groups on the vitamins include thiamine, riboflavin, pyridoxine, pyridoxamine, pyridoxal, deoxypyridoxine. Lipid soluble vitamin K's and related quinone-containing compounds can be conjugated via carbonyl groups on the quinone ring. The phytol moiety of vitamin K may also serve to enhance binding of the PNA to cells.

Pyridoxal (vitamin B₆) has specific B₆-binding proteins. The role of these proteins in pyridoxal transport has been studied by Zhang and McCormick, *Proc. Natl. Acad. Sci. USA,* **1991** *88,* 10407. Zhang and McCormick also have shown that a series of N-(4'-pyridoxyl)amines, in which several synthetic amines were conjugated at the 4'-position of pyridoxal, are able to enter cells by a process facilitated by the B6 transporter. They also demonstrated the release of these synthetic amines within the cell. Other pyridoxal family members include pyridoxine, pyridoxamine, pyridoxal phosphate, and pyridoxic acid. Pyridoxic acid, niacin, pantothenic acid, biotin, folic acid and ascorbic acid can be conjugated to PNAs using N-hydroxysuccinimide esters that are reactive with the PNA or aminolinkers located on the PNA, as described above for retinoic acid.

Other groups for modifying properties of PNAs include RNA/DNA cleaving complexes. RNA/DNA cleavers include o-phenanthroline/metal complexes and Ru(bipyridine)₃²⁺ complexes. The Ru(bpy)₃²⁺ complexes interact with nucleic acids and cleave nucleic acids photochemically. Metal chelators include EDTA, DTPA, nitrilo-triacetate(NTA), and o-phenanthroline. Porphyrins include porphine, its substituted forms, and metal complexes.

A polyamine or polymeric amine species as used herein refers to species that have a plurality of nitrogen atoms thereon. Polyamines include primary amines, hydrazines, semicarbazines, thiosemicarbazines and similar nitrogenous species. Such species can be symmetrical species such as polyamine containing polymers or they can be unsymmetrical wherein the amine functionalities of the polyamine are separated in space by different moieties. In addition to carbon atoms other atomic species such as nitrogen and sulfur may also be incorporated into the polyamine species. In some preferred embodiments of the invention, at least one nitrogen atom of the polyamine has a free electron pair.

Preferred as polyamines are species that range in length from about 3 to about 20 units. More preferably species having at least one nitrogen atom have the general formula H₂N[(CH₂)ₙNH]ₘ- wherein n is an integer between 2 and 8 and m is an integer between 1 and 10. These species can be linear or cyclic. Cyclic amines would include crown amines and mixed crown amines/crown ethers.

Other suitable nitrogen-containing compounds suitable for the formation of polyamines include C₁-C₂₀ straight chain alkylamine, C₁-C₂₀ straight chain substituted alkylamine, C₂-C₅₀ branched chain alkylamine, C₂-C₅₀ branched chain substituted alkylamine, C₃-C₅₀ cyclic alkylamine, C₃-C₅₀ cyclic substituted alkylamine, C₂-C₂₀ straight chain alkenylamine, C₂-C₂₀ straight chain substituted alkenylamine, C₃-C₅₀ branched chain alkenylamine, C₃-C₅₀ branched chain substituted alkenylamine, C₃-C₅₀ cyclic alkenylamine, C₃-C₅₀ cyclic substituted alkenylamine, C₂-C₂₀ straight chain alkynylamine, C₂-C₂₀ straight chain substituted alkynylamine, C₃-C₅₀ branched chain alkynylamine, C₃-C₅₀ branched chain substituted alkynylamine, C₃-C₅₀ cyclic alkynylamine, C₃-C₅₀ cyclic substituted alkynylamine, C₁-C₂₀ straight chain alkylhydrazine, C₁-C₅₀ straight chain substituted alkylhydrazine, C₂-C₅₀ branched chain alkylhydrazine, C₂-C₅₀ branched chain substituted alkylhydrazine, C₃-C₅₀ cyclic hydrazoalkane, C₃-C₅₀ cyclic substituted hydrazoalkane, C₂-C₂₀ straight chain alkenylhydrazine, C₂-C₂₀ straight chain substituted alkenylhydrazine, C₃-C₅₀ branched chain alkenylhydrazine, C₃-C₅₀ branched chain substituted alkenylhydrazine, C₃-C₅₀ cyclic hydrazoalkene, C₃-C₅₀ cyclic substituted hydrazoalkene, C₂-C₂₀ straight chain alkynylhydrazine, C₂-C₂₀ straight chain substituted alkynylhydrazine, C₃-C₅₀ branched chain alkynylhydrazine, C₃-C₅₀ branched chain substituted alkynylhydrazine, C₃-C₅₀ cyclic hydrazoalkyne, C₃-C₅₀ cyclic substituted hydrazoalkyne, C₁-C₂₀ straight chain alkylhydroxyamine, C₁-C₂₀ straight chain substituted alkylhydroxyamine, C₂-C₅₀ branched chain alkylhydroxyamine, C₂-C₅₀ branched chain substituted alkylhydroxyamine, C₃-C₅₀ cyclic oxyalkylamine, C₃-C₅₀ cyclic substituted oxyalkylamine, C₂-C₂₀ straight chain alkenylhydroxyamine, C₂-C₂₀ straight chain substituted alkenylhydroxyamine, C₃-C₅₀ branched chain alkenylhydroxyamine, C₃-C₅₀ branched chain substituted alkenylhydroxyamine, C₃-C₅₀ cyclic oxyalkenylamine, C₃-C₅₀ cyclic substituted oxyalkenylamine, C₂-C₂₀ straight chain alkynylhydroxyamine, C₂-C₂₀ straight chain substituted alkynylhydroxyamine, C₃-C₅₀ branched chain alkynylhydroxyamine, C₃-C₅₀ branched chain substituted alkynylhydroxyamine, C₃-C₅₀ cyclic oxyalkynylamine, C₃-C₅₀ cyclic substituted oxyalkynylamine, C₁-C₂₀ straight chain alkylsemicarbazide, C₁-C₂₀ straight chain substituted alkylsemicarbazide, C₂-C₅₀ branched chain alkylsemicarbazide, C₂-C₅₀ branched chain substituted alkylsemicarbazide, C₃-C₅₀ cyclic alkylsemicarbazide, C₃-C₅₀ cyclic substituted alkylsemicarbazide, C₂-C₂₀ straight chain alkenylsemicarbazide, C₂-C₂₀ straight chain substituted alkenylsemicarbazide, C₃-C₅₀ branched chain alkenylsemicarbazide, C₃-C₅₀ branched chain substituted alkenylsemicarbazide, C₃-C₅₀ cyclic alkenylsemicarbazide, C₃-C₅₀ cyclic substituted alkenylsemicarbazide, C₂-C₂₀ straight chain alkynylsemicarbazide, C₂-C₂₀ straight chain substituted alkynylsemicarbazide, C₃-C₅₀ branched chain alkynylsemicarbazide, C₃-C₅₀ branched chain substituted alkynylsemicarbazide, C₃-C₅₀ cyclic alkynylsemicarbazide, C₃-C₅₀ cyclic substituted alkynylsemicarbazide, C₁-C₂₀ straight chain alkylthiosemicarbazide, C₁-C₂₀ straight chain substituted alkylthiosemicarbazide, C₂-C₅₀ branched chain alkylthiosemicarbazide, C₂-C₅₀ branched chain substituted alkylthiosemicarbazide, C₃-C₅₀ cyclicalkylthiosemicarbazide, C₃-C₅₀ cyclic substituted alkylthiosemicarbazide, C₂-C₂₀ straight chain alkenylthiosemicarbazide, C₂-C₂₀ straight chain substituted alkenylthiosemicarbazide, C₃-C₅₀ branched chain alkenylthiosemicarbazide, C₃-C₅₀ branched chain substituted alkenylthiosemicarbazide, C₃-C₅₀ cyclic alkenylthiosemicarbazide, C₃-C₅₀ cyclic substituted alkenylthiosemicarbazide, C₂-C₂₀ straight chain alkynylthiosemicarbazide, C₂-C₂₀ straight chain substituted alkynylthiosemicarbazide, C₃-C₅₀ branched chain alkynylthiosemicarbazide, C₃-C₅₀ branched chain substituted alkynylthiosemicarbazide, C₃-C₅₀ cyclic alkynylthiosemicarbazide, C₃-C₅₀ cyclic substituted alkynylthiosemicarbazide, C₁-C₂₀ straight chain alkylhydrazone, C₁-C₂₀ straight chain substituted alkylhydrazone, C₂-C₅₀ branched chain alkylhydrazone, C₂-C₅₀ branched chain substituted alkylhydrazone, C₃-C₅₀ cyclic hydrazoalkane, C₃-C₅₀ cyclic substituted hydrazoalkane, C₂-C₂₀ straight chain alkenylhydrazone, C₂-C₂₀ straight chain substituted alkenylhydrazone, C₃-C₅₀ branched chain alkenylhydrazone, C₃-C₅₀ branched chain substituted alkenylhydrazone, C₃-C₅₀ cyclic hydrazoalkene, C₃-C₅₀ cyclic substituted hydrazoalkene, C₂-C₂₀ straight chain alkynylhydrazone, C₂-C₂₀ straight chain substituted alkynylhydrazone, C₃-C₅₀ branched chain alkynylhydrazone, C₃-C₅₀ branched chain substituted alkynylhydrazone, C₃-C₅₀ cyclic hydrazoalkyne, C₃-C₅₀ cyclic substituted hydrazoalkyne, C₁-C₂₀ straight chain alkylhydrazide, C₁-C₂₀ straight chain substituted alkylhydrazide, C₃-C₅₀ branched chain alkylhydrazide, C₃-C₅₀ branched chain substituted alkylhydrazide, C₃-C₅₀ cyclic alkylhydrazide, C₃-C₅₀ cyclic substituted alkylhydrazide, C₂-C₂₀ straight chain alkenylhydrazide, C₂-C₂₀ straight chain substituted alkenylhydrazide, C₃-C₅₀ branched chain alkenylhydrazide, C₃-C₅₀ branched chain substituted alkenylhydrazide, C₃-C₅₀ cyclic alkenylhydrazide, C₃-C₅₀ cyclic substituted alkenylhydrazide, C₂-C₂₀ straight chain alkynylhydrazide, C₂-C₂₀ straight chain substituted alkynylhydrazide, C₃-C₅₀ branched chain alkynylhydrazide, C₃-C₅₀ branched chain substituted alkynylhydrazide, C₃-C₅₀ cyclic alkynylhydrazide and C₃-C₅₀ cyclic substituted alkynylhydrazide.

In accordance with preferred embodiments of the present invention polyamines are linear or cyclic non-aromatic polyamines. A useful class of cyclic non-aromatic polyamines are crown amines which are disclosed by Studer, *et al., Helv. Chim. Acta* **1986,** 69, 2081 and Smith-Jones, *et al., Bioconjugate Chem.* **1991,** 2, 415. In still more preferred embodiments of the present invention polyamines are linear or cyclic non-aromatic comprising non-amide nitrogen atoms. By non-amide is meant a nitrogen which is not adjacent to a carbonyl group (i.e. C=O or C=S).

Representative polyethylene glycol (PEG)-containing groups are disclosed by Ouchi, *et al., Drug Design and Discovery* **1992,** *9,* 93, Ravasio, *et al., J. Org. Chem.* **1991,** *56,* 4329, and Delgardo *et. al., Critical Reviews in Therapeutic Drug Carrier Systems* **1992,** 9, 249. Polyethylene glycols and polyethers include both linear and cyclic compounds. The cyclic polyethers include crown ethers.

As used in this specification the term alkyl includes but is not limited to C₁-C₁₂ straight and branched chained alkyls such as methyl, ethyl, propyl, butyl, pentyl, hexyl, heptyl, octyl, nonyl, decyl, undecyl, dodecyl, isopropyl, 2-butyl, isobutyl, 2-methylbutyl, isopentyl, 2-methyl-pentyl, 3-methylpentyl, 2-ethylhexyl and 2-propylpentyl. Alkenyl groups include but are not limited to unsaturated moieties derived from the above alkyl groups including but not limited to vinyl, allyl and crotyl. Alkynyl groups include unsaturated moieties having at least one triple bond that are derived from the above alkyl groups including but are not limited to ethynyl and propargyl. Aryl groups include but are not limited to phenyl, tolyl, benzyl, naphthyl, anthracyl, phenanthryl, pyrenyl, and xylyl. Halogens include fluorine, chlorine, iodine and bromine. Suitable heterocyclic groups include but are not limited to imidazole, tetrazole, triazole, pyrrolidine, piperidine, piperazine and morpholine. Amines include amines of all of the above alkyl, alkenyl and aryl groups including primary and secondary amines and "masked amines" such as phthalimide. Alkanoyl compounds are those which contain an alkyl portion and are linked through a carbonyl group; i.e., groups which have the structure alkyl-C(O)-. Aralkyl groups have both aryl and alkyl portions, and are attached through their alkyl portions. A benzyl group is an example of an aralkyl group. Alkaryl groups also have both aryl and alkyl portions, but are attached through their aryl portions. Alkoxy groups are alkyl, alkenyl or alkynyl groups which are attached dthrough an oxygen atom.

Heteroaryl groups are aromatic heretocycles, such as pyrrolyl, furyl, thienyl, imidazolyl, pyridyl, pyrimidinyl, and indolyl.

Terpenes are known in the art as oligomers of isoprene, particularly the dipentenes, pinenes, and myrcenes. Included within the definition of terpene molecules are terpene derivatives such as camphor and menthol.

The term phospholipid as used herein includes those compounds which upon hydrolysis yield phosphoric acid, an alcohol and one or more fatty acids. Representative examples of phospholipids include lecithin, cephalin and sphingomyelin.

Amines are also meant to include polyalkylamino compounds and aminoalkylamines such as aminopropylamine and further heterocyclo-alkylamines such as imidazol-1, 2 or 4-yl-propylamine.

Substituent groups for the above as well as for other moieties listed below include but are not limited to other alkyl, haloalkyl, alkenyl, alkynyl, alkoxy, thioalkoxy, haloalkoxy and aryl groups as well as halogen, hydroxyl, amino, azido, carboxy, cyano, nitro, mercapto, sulfides, sulfones, sulfoxides, keto, carboxy, nitrates, nitrites, nitroso, nitrile, trifluoromethyl, O-alkyl, S-alkyl, NH-alkyl, amino, silyl, amides, ester, ethers, carbonates, carbamates, ureas, imidazoles, intercalators, conjugates, polyamines, polyamides, polyethylene glycols, polyethers, groups that enhance the pharmacodynamic properties of oligonucleotides, and groups that enhance the pharmacokinetic properties of oligonucleotides. Other suitable substituent groups also include rhodamines, coumarins, acridones, pyrenes, stilbenes, oxazolo-pyridocarbazoles, anthraquinones, phenanthridines, phenazines, azidobenzenes, psoralens, porphyrins and cholesterols. One particularly preferred group is CF₃. Typical intercalators and conjugates include cholesterols, phospholipids, biotin, phenanthroline, phenazine, phenanthridine, anthraquinone, acridine, fluoresceins, rhodamines, coumarins, and dyes. Halogens include fluorine, chlorine, bromine, and iodine.

One method for preparing compounds according to the invention is to react PNA oligomers or monomeric units bearing at least one free hydroxyl group under basic conditions with a compound having formula Lᵥ-(CH₂)ₙ-S-PG where Lᵥ is a leaving group, PG is a protecting group and n is from 1 to about 20. Displacement of the leaving group through nucleophilic attack of an oxygen anion produces the desired thiol derivative. Suitable leaving groups include but are not limited to halogen, alkylsulfonyl, substituted alkylsulfonyl, arylsulfonyl, substituted arylsulfonyl, hetercyclcosulfonyl, trichloroacetimidate, and pentafluorophenol. A more preferred group includes chloro, fluoro, bromo, iodo, p-(2,4-dinitroanilino)benzenesulfonyl, benzenesulfonyl, methylsulfonyl (mesylate), p-methylbenzenesulfonyl (tosylate), p-bromobenzenesulfonyl, trifluoromethylsulfonyl (triflate), trichloroacetimidate, acyloxy, 2,2,2-trifluoroethanesulfonyl, imidazolesulfonyl, and 2,4,6-trichlorophenyl, with bromo being preferred.

In embodiments wherein thiol-containing functionality is appended to PNA backbone hydroxyl positions, amine functionality in the base portion of the PNA oligomer or monomeric units preferably is protected under non-acidic conditions with protecting groups known in the art, including benzoyl and isobutyryl groups. Alternatively, base protection can precede reaction with thiol reagent Lᵥ-(CH₂)ₙ-S-PG. Suitably protected PNA monomeric units can be assembled into a PNA oligomer according to techniques disclosed in Example 26 of this specification.

Compounds according to the invention can also be prepared by reacting 5-halogen substituted pyrimidine-bearing PNA monomer synthons or 2- or 8-halogen substituted purine PNA monomer synthons with an acetylenic reagent having formula HC≡C-CH₂-Qₐ-PG where Qₐ is O, S, or NH, under conditions effective to couple the pyrimidine or purine base with the acetylenic reagent and form a PNA monomer synthon bearing a substituent having formula -C≡C-CH₂-Qₐ-PG at the pyrimidine 5-position or at the purine 2- or 8-position. Numerous suitable protecting groups are known in the art, including, but not limited to: amine protecting groups such as trifluoroacetate, allyloxycarbonyl (Alloc), carbobenzyloxy-(CBZ), chloroCbz, t-butyloxycarbonyl (Boc), fluorenylmethoxycarbonyl (Fmoc), isonicotinyloxycarbonyl (i-Noc) groups; hydroxyl protecting groups such as t-butyldiphenylsilyl, *t*-butyldimethylsilyl, and dimethoxytrityl groups; and thiol protecting groups such as S-trityl, S-*p*-methoxybenzylthioether, S-*p*-nitrobenzylthioether, and S-*t*-butylthioether. *(see, e.g.,* Veber and Hirschmann, et *al., J. Org. Chem.* **1977,** 42, 3286 and Atherton, *et al.*, The Peptides, Gross and Meienhofer, Eds, Academic Press; New York, 1983; Vol. 9 pp. 1- 38). Coupling preferably is mediated by a metal selected from palladium, nickel, platinum and iridium under conditions generally in accordance with Haralambidis, *et al*., *Nucleic Acids Research* **1987,** *15*, 4857. Once coupling is effected, the protecting group is removed and the resultant free hydroxy, thio, or amino compound is condensed with a suitable thiol derivative having formula R₄-(CH₂)ₙ-S-R₁ where R₄ is R₅OOC-, HS, or -NCS where R₅ is H, chloro, alkyl having 1-12 carbon atoms, or an active ester.

Compounds according to the invention can also be prepared by reacting metal-substituted pyrimidine bearing PNA monomer synthons or purine bearing PNA monomer synthons with an acrylate having formula H₂C=C-C(O)OR₆ (R₆ = alkyl having 1-12 carbon atoms) under conditions effective to couple the pyrimidine or purine base with the acrylate and form a PNA monomer synthon bearing a substituent having formula -CH=CH-C(O)OH at the pyrimidine 5-position or at the purine 2- or 8-position. Coupling is effected under conditions generally in accordance with Dreyer, *et al., Proc. Natl. Acad. Sci. USA* **1985,** *82,* 968. Once coupling is effected, the acid is condensed with an amino thiol derivative having formula H₂N-(CH₂)ₙ-S-PG.

Compounds according to the invention also can be prepared by reacting PNA monomer synthons bearing leaving groups, Lᵥ as defined above, at 5-pyrimidine positions or at 2-, 6-, or 8-purine positions with, for example, thiol derivatives having formula HQₐ-(CH₂)ₙ-S-PG wherein Qₐ and PG are as described above, under conditions effective to displace the leaving group. Such displacement preferably occurs at room temperature in solvents such as dimethylformamide (DMF) or dimethylsulfoxide (DMSO).

PNA oligomers according to the invention can be prepared by assembling a PNA oligomer and appending thiol functionality thereto. For example, PNA oligomers having free hydroxyl groups can be assembled according to the techniques of Examples **26** and **31**-**37** of this specification, and then reacted with a reagent having formula Lᵥ-(CH₂)ₙ-S-PG. As will be recognized, however, greater selectivity can be achieved in terms of placement of thiol functionality within a PNA oligomer by introducing such functionality, as discussed above, on selected PNA monomer synthons and then using both the selected PNA monomer synthons and other PNA monomer synthons to construct the desired PNA oligomer.

Once assembled, a PNA bearing one or more linking moieties ending with -S-PG is treated with a Lewis acid under conditions to remove protecting group PG. Representative acids include silver cation and mercuric cation. Once deprotected, the PNA can be contacted with a thiol-containing steroid molecule, reporter molecule, lipophilic molecule, reporter enzyme, peptide, protein or other thiol containing conjugate in the presence of a thiol-based coupling reagent. Useful coupling reagents include 2,2'-dithiobis(5-nitropyridine) and other pyridyl disulfides or Ellman reagent.

Alternatively, a PNA bearing one or more linking moieties ending with -S-H can be contacted with electrophillic moieties having formula (maleimido)-conjugate or Lᵥ-CH₂C(O)-R₂ where Lᵥ is a leaving group as defined above and R₂ is also defined above. As will be recognized, the sulfur atom on the PNA bonds with the former electrophilic moiety via 1,4-addition and with the latter via nucleophilic displacement. Preferred electrophilic moieties include phospholipid maleimide, o-phenanthroline-5-iodoacetamide, fluorescein maleimide, and pyrene maleimide however many alternative electrophilic moieties can be used.

Thus, in one aspect of the invention the desired PNA sequence is assembled using standard methods and techniques as set forth in Example **26**. At least one PNA monomer including a masked or protected linking moiety is included in the sequence at a predetermined position. A conjugate is then chemically bonded to the linking moiety directly or via a linking moiety attached to the conjugate.

Additional representative thiol groups amenable to the present invention are cysteine, glutathione, penicillamine, 2-pyridylmercaptyl, Br-CH₂-CO-NH-CH₂-CH₂-STr, SH-C-(CH₃)₂CH₂-NH-CH₂-CH₂-NH-CH₂-C-(CH₃)₂SH, HOOC-CH₂-CH₂-S-S-CH₃OOCS, CH₃-CO-S-C(CH₃)-CH₂-C-NH-(CH₂)₂-COOH), (*see*, *e.g*., Dizio, *et al., Bioconjugate Chem*. **1991,** *2*, 353 and Greenfield, *et al., Bioconjugate Chem.* **1990,** *1*, 400).

Numerous amine protecting groups are known in the art, including, but not limited to: phthalimide (Phth), trifluoroacetate, allyloxycarbonyl (Alloc), carbobenzyloxy (CBZ), chloroCBZ, t-butyloxycarbonyl (Boc), fluorenylmethoxycarbonyl (Fmoc), and isonicotinyloxycarbonyl (i-Noc) groups. (*see, e.g.,* Veber and Hirschmann, *et al., J. Org. Chem.* **1977,** *42,* 3286 and Atherton, *et al.,* The Peptides, Gross and Meienhofer, Eds, Academic Press; New York, 1983; Vol. 9 pp. 1-38).

Conjugate bearing PNA oligomers according to the invention may be prepared by either functionalizing the PNA monomeric synthons and assembly into the desired oligomer, or by assembly of PNA monomeric synthons which do not contain conjugate groups, and subsequently attaching the desired conjugate or conjugates thereto. For example, PNA monomer synthons bearing at least one free hydroxyl group may be reacted under basic conditions with a compound having formula Lᵥ-(CH₂)ₙ-NH-PGA where Lᵥ is a leaving group as defined above and PGA is an amine protecting group. Displacement of the leaving group through nucleophilic attack of an oxygen anion produces the desired amine derivative. Suitable leaving groups according to the invention include but are not limited to halogen, alkylsulfonyl, substituted alkylsulfonyl, arylsulfonyl, substituted arylsulfonyl, hetercyclcosulfonyl or trichloroacetimidate. A more preferred group includes chloro, fluoro, bromo, iodo, p-(2,4-dinitroanilino)benzenesulfonyl, benzenesulfonyl, methylsulfonyl (mesylate), p-methylbenzenesulfonyl (tosylate), p-bromobenzenesulfonyl, trifluoromethylsulfonyl (triflate), trichloroacetimidate, acyloxy, 2,2,2-trifluoroethanesulfonyl, imidazolesulfonyl, and 2,4,6-trichlorophenyl, with bromo being preferred.

PNA oligomers according to the invention can also be prepared by assembling a PNA oligomer and appending alkylamino functionality thereto. For example, PNA oligomers having free hydroxyl groups can be assembled according to known techniques see Examples **26** and **31**-**37** and then reacted with a reagent having formula Lᵥ-(CH₂)ₙ-NH-PGA. As will be recognized, however, greater selectivity can be achieved in terms of placement of alkylamino functionality within a PNA oligomer by introducing such functionality, as discussed above, on selected PNA monomeric synthons and then using both the selected PNA monomeric synthons and other PNA monomeric synthons to construct a desired PNA oligomer.

Once assembled, a PNA bearing one or more groups having a linking moiety ending with a protected amine group is treated with reagents effective to remove the protecting group. Once deprotected, the PNA can be contacted with electrophilic moieties such as, for example, succinimidyl esters and other activated carboxylic acids including C(=O)-O-succinimide and C ( = O ) - O - pentafluorophenyl, isothiocyanates, sulfonyl chlorides, haloacetamides, phospholipid carbocyclic acid active esters, o-phenanthroline-5-iodoacetamide,fluoresceinisothiocyanate, 1-pyrene butyric acid-N-hydroxy succinimide ester and carboxylic acid derivatives of peptide nucleic acids. Preferred electrophilic moieties include cholesteryl-3-hemisuccinate-N-hydroxy succinimide ester, pyrene-1-butyric acid-N-hydroxy succinimide ester and polyethylene glycol-propionic acid-N-hydroxy succimide ester.

In other aspects of the invention a conjugate is attached to at least one monomeric unit of a PNA in a diagnostic or therapeutic agent to assist in the transfer of therapeutic or diagnostic agent across cellular membranes. Such a diagnostic or therapeutic agent is formed from a plurality of linked PNA monomeric units bearing natural or non-natural occurring bases and including at least one conjugate which, can include an optional linking moiety, appended thereto, in a sequence that is complementary to and will specifically hybridize with a region of an RNA or DNA of interest. Thus, one or more of the linked PNA monomeric units are "functionalized" to include a conjugate linked to the PNA monomeric unit. This linkage may include an optional linking moiety. Linked PNA monomer units having at least one functionalized PNA monomer unit within their sequence are expected to demonstrate enhanced activity and transport across cellular membranes.

A variety of linking groups can be used to connect conjugates to PNA oligomers or monomer synthons. Certain linking groups, such as Ω-aminoalkoxy moieties and Ω-aminoalkylamino moieties, are particularly useful for linking steroid molecules or reporter molecules to PNA hydroxyl groups. Many linking groups are commercially available, including heterobifunctional and homobifunctional linking moieties available from the Pierce Co. (Rockford, Il). Heterobifunctional and homobifunctional linking moieties are particularly useful in conjunction with the Ω-aminoalkoxy and Ω-aminoalkylamino moieties to form extended linkers that connect peptides and proteins to PNA oligomers. It is intended that the PNA monomer synthons of the invention include adenine bearing PNA synthons functionalized with linkers on their N-6 purine amino groups, guanine bearing PNA monomer synthons functionalized with linkers at their exocyclic N-2 purine amino groups, and cytosine bearing PNA monomer synthons functionalized with linkers on either their N-4 pyrimidine amino groups or 5 pyrimidine positions. This type of linkage is illustrated by the attachment of the conjugate biotin in a PNA 5-mer to the N-4 position of cytosine in Example **47**.

A PNA oligomer of the invention may be reacted with an active ester derivative of a conjugate *(e.g.,* cholic acid). Active ester derivatives include N-hydroxysuccinimide esters, tetrafluorophenolic esters, pentafluorophenolic esters and pentachlorophenolic esters. For cholic acid, the reaction of the amino group and the active ester produces a PNA oligomer in which cholic acid is attached to the N-terminal position through a linking group. Cholic acid can be attached to the carboxy terminal of a PNA oligomer by conversion of the cholic acid to the N-hydroxysuccinimide ester thereof, and then by further reaction with the PNA of interest. Cholic acid can be attached to both ends of a PNA oligomer by applying both methods above. As will be seen by those skilled in the art, the above methods are applicable to a large number of conjugates of the present invention.

In further embodiments of the invention, a PNA oligomer bearing an aminolinker at one or more selected non-terminal monomeric units is prepared according to the procedures of Examples **26** and **65**. A conjugate such as cholic acid is then attached using an active ester or an isothiocyanate thereof. This approach allows the introduction of a large number of conjugates into a single PNA oligomeric sequence. Indeed each of the PNA monomers in the oligomer can be so substituted.

Attachment of conjugates such as reporter enzymes, peptides, and proteins to PNAs is achieved by reaction of the enzyme, peptide or protein with the above-described amino linking group on the PNA by any of several methods. For example, a peptide or protein can be attached to a functionalized PNA monomer by treatment of the peptide or protein with EDC/sulfo-NHS (*i.e.*, 1-ethyl-3(3-dimethylaminopropylcarbodiimide/N-hydroxysulfosuccinimide), which will facilitate linkage of the carboxyl end of the reporter enzyme, peptide, or protein to the amino terminal end of the PNA, or to an amine-containing linker bound to the PNA. Further, a PNA oligomeric conjugate can be prepared using EDC/sulfo-NHS to attach a carboxyl group of an aspartic or glutamic acid residue in the reporter enzyme, peptide or protein to the amino terminus of a PNA.

Preferably a reporter enzyme-, peptide- or protein-functionalized PNA can be prepared by treating the reporter enzyme, peptide or protein with the PNA oligomer functionalized with a heterobifunctional linker such as m-maleimidobenzoyl-N-hydroxysulfosuccinimide ester (MBS) or succinimidyl4-(N-maleimidomethyl)cyclohexane-1-carboxylate (SMCC). Thus a thiol function on the reporter enzyme, peptide or protein is linked to the amino function of the PNA. A PNA oligomer-maleimide conjugate is formed by reaction of the amino group of the PNA-bound linker with the MBS or SMCC maleimide linker. The resulting conjugate is then reacted with peptides or proteins having free sulfhydryl groups.

In a second preferred method, a reporter enzyme-, peptide- or protein-functionalized linked PNA oligomer can be prepared by treatment of the peptide or protein with the PNA using a homobifunctional linker such as disuccinimidyl suberate (DSS) to link an amino function on the peptide or protein to the amino group of a linker on the PNA. By this mechanism, a succinimidyl functionality can be attached to a PNA oligomer by reaction of the amino group of the PNA-bound linker with a disuccinimidyl suberate linker. The disuccinimidyl suberate linker couples with the amine linker on the sequence to extend the size of the linker. The extended linker is then reacted with amine groups such as, for example, the amine of lysine or other available N-terminus amines on reporter enzymes, peptides and proteins.

PNA oligomers of the present invention may range in size from a dimer to a 200-mer. In preferred embodiments of the present invention the PNA oligomers range in size from a dimer to a 50-mer. In more preferred embodiments the PNA oligomers range in size from a dimer to about a 20-mer.

In further embodiments a 13- to 14-mer PNA is used following standard methods and techniques to identify a region of a DNA coding for a message that is present for the expression of a specific mRNA.

Although longer PNA molecules are envisioned by the present invention it can redily be seen that PNAs having from from 1 to about 20 monomeric units will be useful for a number of diagnostic applications.

The compounds of the present invention also will be useful as research reagents for the modulation of the production of a protein by an organism. Modulation may be accomplished by contacting the organism with compounds of the present invention. Preferably the compounds are hybridizable with RNA or DNA coding for the protein of interest.

The compounds of the present invention will additionally be useful as diagnostic reagents. Diagnostic applications include the detection of the presence or absence of a particular DNA or RNA in a sample. The sample of interest is contacted with a compound of the invention wherein the compound is specifically hybridizable with the RNA of interest. Methods of detecting the hybridization of the diagnostic reagent to the DNA or RNA of interest or alternativly the absence of such hybridization include but are not limited to detection by fluorescence, radiolabeling or capillary gel electrophoresis. PNAs of the present invention are labeled by attachment of the appropriate conjugate thereto. The sample of interest is contacted with the PNA having at least one conjugate attached thereto. The determination of the presence or absence of the nucleic acid of interest will be facilitated by the detection of the presence or absence of hybridization to the sample.

In a further aspect of the invention, the PNAs are conjugated to peptides or proteins, where the peptides have signaling activity and the proteins are, for example, enzymes, transcription factors or antibodies. Also, the PNAs can be attached to water-soluble or water-insoluble polymers.

In another aspect of the invention, the PNAs are conjugated to oligonucleotides or carbohydrates. When desired, a PNA oligomer can be synthesized onto a moiety (*e.g.*, a peptide chain, reporter, intercalator or other type of ligand-containing group) which is attached to a solid support.

As a further aspect of the present invention, PNAs can be used to target RNA and ssDNA to produce hybridization probes for the identification and purification of nucleic acids. Furthermore, the PNAs can be modified to form strand displacement triple helices with dsDNA. Thus, these reagents may be used for research and in diagnostics for detection and isolation of specific nucleic acids.

Triple helix formation, wherein an oligonucleotide is triplexed to a dsDNA, is believed to be the only means known in the art for sequence-specific recognition of dsDNA. However, triple helix formation is largely limited to recognition of homopurine-homopyrimidine sequences. Triplexing with strand displacement using PNAs of this invention is superior to oligonucleotide-dsDNA triple helix recognition in that it may allow for recognition of any sequence by use of the four natural bases.

PNAs of the present invention may be useful as research and diagnostic reagents for the detection of a gene. PNAs are designed with a nucleobase sequence containing from about 12 to about 18 sub-units that are complementary to the regulatory region (the promoter) of the target gene. Samples (e.g. cells, tissue, or other) of interest are prepared following standard methods and techniques and are treated in vitro with a PNA (in a suitable carrier) of the present invention having a specific sequence and at least one conjugate attached to at least one sub-unit. The PNA will bind to the promoter and block access thereto by RNA polymerase. Consequently, no mRNA, and thus no gene product (protein), is produced. If the target is within a vital gene for a virus, no viable virus particles will be produced. Alternatively, if the target is downstream from the promoter, RNA polymerase will terminate at this position, thus forming a truncated mRNA/protein which is nonfunctional.

Sequence-specific recognition of ssDNA by base complementary hybridization can be exploited to target specific genes and viruses. In this case, the target sequence is contained in the mRNA such that binding of the drug to the target hinders the action of ribosomes and, consequently, translation of the mRNA into protein. The peptide nucleic acids of the invention are superior to prior reagents in that they have significantly higher affinity for complementary ssDNA. Additionally, because PNAs donot posses a charged backbone, lysine or other similar charged moieties can be present without interfering with PNA activity. Additionally, PNAs are water soluble (which should facilitate cellular uptake), and contain amides of non-biological amino acids (which should make them biostable and resistant to enzymatic degradation by, for example, proteases), and are capable of triplex formation with mRNA.

Binding of PNA compounds to double-stranded DNA accompanied by strand displacement is shown in United States application serial number 08/088,658, filed July 2, 1993, entitled "Higher Order Structure and Binding of Peptide Nucleic Acids". Binding of PNA and bis PNA to double stranded DNA accompanied by strand invasion is also shown in United States application serial number 08/275,951, filed July 15, 1994, entitled "Linked Peptide Nucleic Acids". The illustrative examples therein show that PNA compounds bind via Watson-Crick binding to their complementary strand and extrudes the other strand in a virtually single-stranded conformation.

The chimeric structure between PNAs and DNA or RNA are used in place of, or in addition to a normal PNA strand to effect duplexing, triplexing, nucleic acid binding or protein binding. The RNA or DNA nucleic acid portion of such chimeric structures include but are not limited to a nucleic acid connected via phosphodiester, phosphorothioate, phosphorodithioate, alkylphosphonate, hydrogen phosphonate, phosphotriester, phosphoramidite and other like phosphorus linkages. They further can include but are not limited to other substitutions such as substitution at the 2' position of a ribose sugar. Particularly preferred are 2'-fluoro's since they increase affinity of the nucleic acid portion of the chimera to other nucleic acids and 2'-O-alkyl, particularly 2'-O-propyl, 2'-O-allyl and the like since they confer nuclease resistance to the nucleic acid strand.

The compounds of the present invention may be useful to bind to other target molecules. Target molecules of the present invention can include any of a variety of biologically significant molecules. Such other target molecules can be carbohydrates, glycoproteins or other proteins. In some preferred embodiments of the present invention, the target molecule is a protein such as an immunoglobulin, receptor, receptor binding ligand, antigen or enzyme and more specifically can be a phospholipase, tumor necrosis factor, endotoxin, interleukin, plasminogen activator, protein kinase, cell adhesion molecule, lipoxygenase, hydrolase or transacylase. In other embodiments of the invention the target molecules can be important regions of the human immunodeficiency virus, *Candida*, herpes viruses, papillomaviruses, cytomegalovirus, rhinoviruses, hepatitis viruses, or influenza viruses. In yet other embodiments of the present invention the target molecules can be regions of an oncogene. In still further embodiments, the target molecule is ras 47-mer stem loop RNA, the TAR element of human immunodeficiency virus, rev response element (RRE) or the gag-pol stem loop of human immunodeficiency virus (HIV). Still other targets can induce cellular activity. For example, a target can induce interferon.

In binding to transcription factors or other target molecules, the transcription factor or other target molecule need not be purified. It can be present, for example, in a whole cell, in a humoral fluid, in a crude cell lysate, in serum or in other humoral or cellular extract. Of course, purified transcription factor or a purified form of another target molecule is also useful in some aspects of the invention.

In still other embodiments of the present invention, synthetically prepared transcription factor or other target molecules can be useful. These synthetically prepared molecules need only contain at least one monomeric unit having a conjugate attached thereto. A transcription factor or other target molecule also can be modified, such as by biotinylation or radiolabeling. For example, synthetically prepared transcription factor can incorporate one or more biotin molecules during synthesis or can be modified post-synthesis.

Transcription factors, as the term is used herein, are DNA- or RNA-binding proteins that regulate the expression of genes. HIV tat and c-rel are examples of transcription factors which regulate the expression of genes. Also encompassed by the term are DNA and RNA binding proteins which are not strictly considered transcription factors, but which are known to be involved in cell proliferation. These transcription factors include c-myc, fos, and jun. Methods of the present invention are particularly suitable for use with transcription factor as target molecules since transcription factors generally occur in very small cellular quantities.

The utility of PNA-containing duplex structures can be illustrated by constructing PNA sequences which correspond to various sequences of the HIV TAR element that have the potential to form duplex structures either as stem-loop structures or two PNAs forming a duplex structure. In a competition assay, PNA structures that bind the tat transcription factor prevent binding of the competitor TAR sequence present in the incubation mixture. As the TAR RNA sequence is biotinylated only that proteins available to bind to TAR will remain on the microtiter plate after washing away unbound molecules and tat protein complexed to a PNA sequence. The concentration dependence of the competition between the TAR PNA structures and biotinylated TAR structure will serve to define those sequences capable of effectively competing for tat and thus useful as HIV modulatory agents.

Additional objects, advantages, and novel features of this invention will become apparent to those skilled in the art upon examination of the following examples thereof.

### EXAMPLE 1

### tert-Butyl 4-nitrophenyl carbonate (1)

Sodium carbonate (29.14 g; 0.275 mol) and 4-nitrophenol (12.75 g; 91.6 mmol) were mixed with dioxane (250 ml). Boc-anhydride (20.0 g; 91.6 mmol) was transferred to the mixture with dioxane (50 ml). The mixture was refluxed for 1 h, cooled to 0°C, filtered and concentrated to 1/3, and then poured into water (350 ml) at 0°C. After stirring for 1/2 h., the product was collected by filtration, washed with water, and then dried over an appropriate drying agent, *in vacuo.* Yield 21.3 g (97%). M.p. 73.0-74.5°C (litt. 78.5-79.5°C). Anal. for C₁₁H₁₃NO₅ found(calc.) C: 55.20(55.23) H: 5.61(5.48) N: 5.82(5.85).

### EXAMPLE 2

### (N-Boc-2-aminoethyl)glycine (2)

The title compound was prepared by a modification of the procedure by Heimer, *et al. Int. J. Pept.,* **1984,** *23,* 203-211 N-(2-Aminoethyl)glycine (3.00 g; 25.4 mmol) was dissolved in water (50 ml), dioxane (50 ml) was added, and the pH was adjusted to 11.2 with 2 N sodium hydroxide. tert-Butyl-4-nitrophenyl carbonate (**1**, 7.29 g; 30.5 mmol) was dissolved in dioxane (40 ml) and added dropwise over a period of 2 h, during which time the pH was maintained at 11.2 with 2 N sodium hydroxide. The pH was adjusted periodically to 11.2 for three more hours and then the solution was left overnight. The solution was cooled to 0°C and the pH was carefully adjusted to 3.5 with 0.5 M hydrochloric acid. The aqueous solution was washed with chloroform (3 x 200 ml), the pH adjusted to 9.5 with 2N sodium hydroxide and the solution was evaporated to dryness, *in vacuo* (14 mmHg). The residue was extracted with DMF (25+2x10 ml) and the extracts filtered to remove excess salt. This results in a solution of the title compound in about 60% yield and greater than 95% purity by tlc (visualized by ninhydrin, Rf=0.3). The solution was used in the following preparations of Boc-aeg (aeg=aminoethylglycine) derivates without further purification.

### EXAMPLE 3

### N-1-Carboxymethylthymine (3)

This procedure is different from the literature synthesis, but is easier, gives higher yields, and leaves no unreacted thymine in the product. To a suspension of thymine (40.0 g; 0.317 mol) and potassium carbonate (87.7 g; 0.634 mmol) in DMF (900 ml) was added methyl bromoacetate (30.00 ml; 0.317 mmol). The mixture was stirred vigorously overnight under nitrogen. The mixture was filtered and evaporated to dryness, in *vacuo.* The solid residue was treated with water (300 ml) and 4 N hydrochloric acid (12 ml), stirred for 15 min at 0°C, filtered, and washed with water (2 x 75 ml). The precipitate was treated with water (120 ml) and 2N sodium hydroxide (60 ml), and was boiled for 10 minutes. The mixture was cooled to 0°C, filtered, and the pure title compound was precipitated by the addition of 4 N hydrochloric acid (70 ml). Yield after drying, *in vacuo* over an appropriate drying agent: 37.1 g (64%). ¹H-NMR: (90 MHz; DMSO-d₆) : 11.33 ppm (s,1H,NH); 7.49(d,J=0.92Hz,1H,ArH) 4.38 (s,2H,CH₂); 1.76 (d,J=0.92Hz,T-CH₃).

### EXAMPLE 4

### N-1-Carboxymethylthymine pentafluorophenyl ester (4)

N-1-Carboxymethylthymine (**3**, 10.0g; 54.3 mmol) and pentafluorophenol (10.0 g; 54.3 mmol) were dissolved in DMF (100 ml) and cooled to 5°C in ice water. DCC (13.45 g; 65.2 mmol) then was added. When the temperature passed below 5°C, the ice bath was removed and the mixture was stirred for 3 h at ambient temperature. The precipitated DCU was removed by filtration and washed twice with DMF (2 x 10 ml). The combined filtrate was poured into ether (1400 ml) and cooled to 0°C. Petroleum ether (1400 ml) was added and the mixture was left overnight. The title compound was isolated by filtration and was washed thoroughly with petroleum ether. Yield: 14.8 g(78%). The product was pure enough to carry out the next reaction, but an analytical sample was obtained by recrystallization from 2-propanol. M.p. 200.5-206°C Anal. for C₁₃H₇F₅N₂O₄. Found(calc.) C: 44.79(44.59); H: 2.14(2.01) N: 8.13(8.00). FAB-MS: 443 (M+1+glycerol), 351 (M+1). ¹H-NMR (90 MHz; DMSO-d₆): 11.52 ppm (s,1H,NH); 7.64 (s,1H,ArH); 4.99 (s,2H,CH₂); 1.76 (s,3H,CH₃).

### EXAMPLE 5

### N-(N-Boc-2-aminoethyl)-N-[(1-thyminyl)acetyl]glycine (5)

To the DMF-solution from Example **2** was added triethyl amine (7.08 ml; 50.8 mmol) followed by N-1-carboxymethylthymine pentafluorophenyl ester (**4**, 4.45 g; 12.7 mmol). The resultant solution was stirred for 1 h. The solution was cooled to 0°C and treated with cation exchange material ("Dowex 50W X-8", 40 g) for 20 min. The cation exchange material was removed by filtration, washed with dichloromethane (2 x 15 ml), and dichloromethane (150 ml) was added. The resulting solution was washed with saturated sodium chloride, dried over magnesium sulfate, and evaporated to dryness, *in vacuo*, first by a water aspirator and then by an oil pump. The residue was shaken with water (50 ml) and evaporated to dryness. This procedure was repeated once. The residue then was dissolved in methanol (75 ml) and poured into ether (600 ml) and petroleum ether (1.4 L). After stirring overnight, the white solid was isolated by filtration and was washed with petroleum ether. Drying over an appropriate drying agent, *in vacuo*, gave 3.50 g (71.7%). M.p. 142-147°C. Anal. for C₁₆H₂₄N₄O₇. Found(calc.) C: 49.59(50.00) H: 6.34(6.29) N: 14.58(14.58). ¹H-NMR (250 MHz, DMSO-d₆): Due to the limited rotation around the secondary amide bond several of the signals were doubled in the ratio 2:1,(indicated in the list by mj. for major and mi. for minor). 12.73 ppm (b,1H, -CO₂H); 11.27 ppm (s, mj., imide); 11.25 ppm (s, mi., imide); 7.30 ppm (s, mj., ArH); 7.26 ppm (s, mi., ArH); 6.92 ppm (unres. t, mj., BocNH); 6.73 ppm (unres. t; mi., BocNH); 4.64 ppm (s, mj., T-CH₂-CO-); 4.47 ppm (s, mi., T-CH₂-CO-); 4.19 ppm (s, mi., CONRCH₂CO₂H); 3.97 ppm (s, mj., CONRCH₂CO₂H); 3.41-2.89 ppm (unres. m, -CH₂CH₂- and water) ; 1.75 ppm (s,3H, T-CH₃); 1.38 ppm (s, 9H, t-Bu). ¹³C-NMR: 170.68 ppm (CO); 170.34 (CO); 167.47 (CO); 167.08 (CO); 164.29 (CO); 150.9 (C5''); 141.92 (C6''); 108.04 (C2'); 77.95 and 77.68 (Thy-CH₂CO); 48.96, 47.45 and 46.70 (-CH₂CH₂- and NCH₂CO₂H); 37.98 (Thy-CH₃) ; 28.07 (t-Bu). FAB-MS: 407 (M+Na⁺) ; 385 (M+H⁺).

### EXAMPLE 6

### N- (N-Boc-2-aminoethyl)-N-[(1-thyminyl)acetyl]glycine pentafluorophenyl ester (6, Boc-Taeg.OPfp)

N-(N-Boc-2-aminoethyl)-N-[(1-thyminyl)acetyl]glycine (5) (2.00 g; 5.20 mmol) was dissolved in DMF (5 ml) and methylene chloride (15 ml) was added. Pentafluorophenol (1.05 g; 5.72 mmol) was added and the solution was cooled to 0°C in an ice bath. DDC then was added (1.29 g; 6.24 mmol) and the ice bath was removed after 2 min. After 3 h with stirring at ambient temperature, the precipitated DCU was removed by filtration and washed with methylene chloride. The combined filtrate was washed twice with aqueous sodium hydrogen carbonate and once with saturated sodium chloride, dried over magnesium sulfate, and evaporated to dryness, *in vacuo*. The solid residue was dissolved in dioxane (150 ml) and poured into water (200 ml) at 0°C. The title compound was isolated by filtration, washed with water, and dried over an appropriate drying agent, *in vacuo*. Yield: 2.20 g (77%). An analytical sample was obtained by recrystallisation from 2-propanol. M.p. 174-175.5°C. Analysis for C₂₂H₂₃N₄O₇F₅, found(calc.): C: 48.22(48.01); H: 4.64(4.21); N: 9.67(10.18). ¹H-NMR (250 MHz, CDCl₃) :Due to the limited rotation around the secondary amide bond several of the signals were doubled in the ratio 6:1 (indicated in the list by mj. for major and mi. for minor). 7.01 ppm (s, mi., ArH); 6.99 ppm (s, mj., ArH); 5.27 ppm (unres. t, BocNH); 4.67 ppm (s, mj., T-CH₂-CO-); 4.60 ppm (s, mi., T-CH₂-CO-); 4.45 ppm (s, mj., CONRCH₂CO₂Pfp); 4.42 ppm (s, mi., CONRCH₂CO₂Pfp); 3.64 ppm (t, 2H, BocNHCH₂CH₂-); 3.87 ppm ("q",2H,BocNHCH₂CH₂-); 1.44(s,9H,t-Bu). FAB-MS: 551 (10; M+1); 495 (10; M+l-tBu); 451 (80; -Boc).

### EXAMPLE 7

### N-(N-Boc-2-aminoethyl)-N-(Cbz)glycine (7)

Aminoethyl glycine (52.86 g; 0.447 mol) was dissolved in water (900 ml) and dioxane (900 ml) was added. The pH was adjusted to 11.2 with 2N NaOH. While the pH was kept at 11.2, tert-butyl-p-nitrophenyl carbonate (128.4 g; 0.537 mol) was dissolved in dioxane (720 ml) and added dropwise over the course of 2 hours. The pH was kept at 11.2 for at least three more hours and then left with stirring overnight. The yellow solution was cooled to 0°C and the pH was adjusted to 3.5 with 2 N HCl. The mixture was washed with chloroform (4x100 ml), and the pH of the aqueous phase was readjusted to 9.5 with 2 N NaOH at 0°C. Cbz chloride (73.5 ml; 0.515 mol) was added over half an hour, while the pH was kept at 9.5 with 2 N NaOH. The pH was adjusted frequently over the next 4 hours, and the solution was left with stirring overnight. On the following day the solution was washed with ether (3x600 ml) and the pH of the solution was afterwards adjusted to 1.5 with 2 N HCl at 0°C. The title compound was isolated by extraction with ethyl acetate (5x1000 ml). The ethyl acetate solution was dried over magnesium sulfate and evaporated to dryness, *in vacuo.* This afforded 138 g, which was dissolved in ether (300 ml) and precipitated by the addition of petroleum ether (1800 ml). Yield 124.7 g (79%). M.p. 64.5-85 °C. Anal. for C₁₇H₂₄N₂O₆ found(calc.) C: 58.40(57.94); H: 7.02(6.86); N: 7.94(7.95). ¹H-NMR (250 MHz, CDCl₃) 7.33 & 7.32 (5H, Ph); 5.15 & 5.12 (2H, PhCH₂); 4.03 & 4.01 (2H, NCH₂CO₂H); 3.46 (b, 2H, BocNHCH₂CH₂); 3.28 (b, 2H, BocNHCH₂CH₂); 1.43 & 1.40 (9H, ^{t}Bu). HPLC (260 nm) 20.71 min. (80.2%) and 21.57 min. (19.8%). The UV-spectra (200 nm - 300 nm) are identical, indicating that the minor peak consists of Bis-Z-AEG.

### EXAMPLE 8

### N-(N-Boc-2-aminoethyl)glycine ethyl ester (8)

N-(N-Boc-2-aminoethyl)-N-(Cbz)glycine (**7,** 60.0 g; 0.170 mol) and N,N-dimethyl-4-aminopyridine (6.00 g) were dissolved in absolute ethanol (500 ml), and cooled to 0°C before the addition of DCC (42.2 g; 0.204 mol). The ice bath was removed after 5 minutes and stirring was continued for 2 more hours. The precipitated DCU (32.5 g dried) was removed by filtration and washed with ether (3x100 ml). The combined filtrate was washed successively with diluted potassium hydrogen sulfate (2x400 ml), diluted sodium hydrogencarbonate (2x400 ml) and saturated sodium chloride (1x400 ml). The organic phase was filtered, then dried over magnesium sulfate, and evaporated to dryness, in vacuo, which yielded 66.1 g of an oily substance which contained some DCU.

The oil was dissolved in absolute ethanol (600 ml) and was added 10% palladium on carbon (6.6 g) was added. The solution was hydrogenated at atmospheric pressure, where the reservoir was filled with 2 N sodium hydroxide. After 4 hours, 3.3 L was consumed out of the theoretical 4.2 L. The reaction mixture was filtered through celite and evaporated to dryness, *in vacuo*, affording 39.5 g (94%) of an oily substance. A 13 g portion of the oily substance was purified by silica gel (600 g SiO₂) chromatography. After elution with 300 ml 20% petroleum ether in methylene chloride, the title compound was eluted with 1700 ml of 5% methanol in methylene chloride. The solvent was removed from the fractions with satisfactory purity, *in vacuo* and the yield was 8.49 g. Alternatively 10 g of the crude material was purified by Kugel Rohr distillation. ¹H-NMR (250 MHz, CD₃OD) ; 4.77 (b. s, NH); 4.18 (q, 2H, MeCH₂-) ; 3.38 (s, 2H, NCH₂CO₂Et); 3.16 (t, 2H, BocNHCH₂CH₂) ; 2.68 (t, 2H, BocNHCH₂CH₂) ; 1.43 (s, 9H, ^{t}Bu) and 1.26 (t, 3H, CH₃) ¹³C-NMR 171.4 (COEt) ; 156.6 (CO) ; 78.3 ((CH₃)₃C) ; 59.9 (CH₂) ; 49.0 (CH₂); 48.1 (CH₂); 39.0 (CH₂); 26.9 (CH₂) and 12.6 (CH₃).

### EXAMPLE 9

### N-(N-Boc-2-aminoethyl)glycine methyl ester (9)

The procedure of Example **8** was used, with methanol being substituted for ethanol. The final product was purified by flash column chromatography.

### EXAMPLE 10

### N-(N-Boc-2-aminoethyl)-N-[(1-thyminyl)acetyl]glycine ethyl ester (10)

N-(N-Boc-2-aminoethyl)glycine ethyl ester (**8**, 13.5 g; 54.8 mmol), DhbtOH (9.84 g; 60.3 mmol) and N-1-carboxymethyl thymine (**4**, 11.1 g; 60.3 mmol) were dissolved in DMF (210 ml). Methylene chloride (210 ml) then was added. The solution was cooled to 0°C in an ethanol/ice bath and DCC (13.6 g; 65.8 mmol) was added. The ice bath was removed after 1 hour and stirring was continued for another 2 hours at ambient temperature. The precipitated DCU was removed by filtration and washed twice with methylene chloride (2 x 75 ml). To the combined filtrate was added more methylene chloride (650 ml). The solution was washed successively with diluted sodium hydrogen carbonate (3 x 500 ml), diluted potassium hydrogen sulfate (2 x 500 ml), and saturated sodium chloride (1 x 500 ml). Some precipitate was removed from the organic phase by filtration, The organic phase was dried over magnesium sulfate and evaporated to dryness, in *vacuo.* The oily residue was dissolved in methylene chloride (150 ml), filtered, and the title compound was precipitated by the addition of petroleum ether (350 ml) at 0°C. The methylene chloride/petroleum ether procedure was repeated once. This afforded 16.0 g (71%) of the title compound which was more than 99% pure by HPLC.

### EXAMPLE 11

### N-(N-Boc-2-aminoethyl)-N-[(1-thyminyl)acetyl]glycine (6a)

The material from Example **10** was suspended in THF (194 ml, gives a 0.2 M solution), and 1 M aqueous lithium hydroxide (116 ml) was added. The mixture was stirred for 45 minutes at ambient temperature and then filtered to remove residual DCU. Water (40 ml) was added to the solution which was then washed with methylene chloride (300 ml). Additional water (30 ml) was added, and the alkaline solution was washed once more with methylene chloride (150 ml). The aqueous solution was cooled to 0°C and the pH was adjusted to 2 by the dropwise addition of 1 N HCl (approx. 110 ml). The title compound was extracted with ethyl acetate (9 x 200 ml), the combined extracts +were dried over magnesium sulfate and were evaporated to dryness, *in vacuo.* The residue was evaporated once from methanol, which after drying overnight afforded a colorless glassy solid. Yield 9.57 g (64 %). HPLC > 98% R_{T}=14.8 min . Anal. for C₁₆H₂₄N₄O₇/0.25 H₂O Found (calc.) C: 49.29(49.42); H: 6.52(6.35); N: 14.11(14.41). Due to the limited rotation around the secondary amide, several of the signals were doubled in the ratio 2:1 (indicated in the list by mj. for major and mi. for minor). ¹H-NMR (250 MHz, DMSO-d₆) : 12.75 (b.s., 1H, CO₂H); 11.28 (s, "1H", mj., imide NH); 11.26 (s, "1H", mi., imide NH); 7.30 (s, "1H", mj., T H-6); 7.26 (s, "1H", mi., T H-6); 6.92 (b.t., "1H", mj., BocNH); 6.73 (b.t., "1H", mi., BocNH); 4.64 (s, "2H", mj., CH₂CON); 4.46 (s, "2H", mj., CH₂CON); 4.19 (s, "2H", mi., CH₂CO₂H); 3.97 (s, "2H"_{,} mj., CH₂CO₂H); 3.63-3.01 (unresolved m, includes water, CH₂CH₂); 1.75 (s, 3H, CH₃) and 1.38 (s, 9H, ^{t}Bu).

### EXAMPLE 12

### N-4-Cbz cytosine (12)

Over a period of about 1 h, Cbz chloride (52 ml; 0.36 mol) was added dropwise to a suspension of cytosine (8, 20.0 g;0.18 mol) in dry pyridine (1000 ml) at 0°C under nitrogen in oven-dried equipment. The solution then was stirred overnight, after which the pyridine suspension was evaporated to dryness, in vacuo. Water (200 ml) and 4 N hydrochloric acid were added to reach pH ∼1. The resulting white precipitate was filtered off, washed with water and partially dried by air suction. The still-wet precipitate was boiled with absolute ethanol (500 ml) for 10 min, cooled to 0°C, filtered, washed thoroughly with ether, and dried, in vacuo. Yield 24.7 g (54%). M.p.>250°C. Anal. for C12H11N3O3. Found(calc.); C: 58.59(58.77); H: 4.55(4.52); N: 17.17(17.13). No NMR spectra were recorded since it was not possible to get the product dissolved.

### EXAMPLE 13

### N-4-Cbz-N-1-carboxymethyl cytosine (13)

In a three necked round bottomed flask equipped with mechanical stirring and nitrogen coverage was placed methyl bromacetate (7.82 ml;82.6 mmol) and a suspension of N-4-Cbz cytosine (12, 21.0 g;82.6 mmol) and potassium carbonate (11.4 g;82.6 mmol) in dry DMF (900 ml). The mixture was stirred vigorously overnight, filtered, and evaporated to dryness, *in vacuo*. Water (300 ml) and 4 N hydrochloric acid (10 ml) were added, the mixture was stirred for 15 minutes at 0°C, filtered, and washed with water (2 x 75 ml). The isolated precipitate was treated with water (120 ml), 2N sodium hydroxide (60 ml), stirred for 30 min, filtered, cooled to 0°C, and 4 N hydrochloric acid (35 ml) was added. The title compound was isolated by filtration, washed thoroughly with water, recrystallized from methanol (1000 ml) and washed thoroughly with ether. This afforded 7.70 g (31%) of pure compound. The mother liquor from the recrystallization was reduced to a volume of 200 ml and cooled to 0°C. This afforded an additional 2.30 g of a material that was pure by tlc but had a reddish color. M.p. 266-274°C. Anal. for C₁₄H₁₃N₃O₅. Found(calc.); C: 55.41(55.45); H: 4.23(4.32); N: 14.04(13.86). ¹H-NMR (90 MHz; DMSO-d₆): 8.02 ppm (d,J=7.32Hz,1H,H-6); 7.39 (s,5H,Ph); 7.01 (d,J=7.32Hz,1H,H-5); 5.19 (s,2H,PhCH₂-); 4.52 (s,2H).

### EXAMPLE 14

### N-4-Cbz-N-1-carboxymethyl cytosine pentafluorophenyl ester (14)

N-4-Cbz-N-1-carboxymethyl-cytosine (**13**, 4.00 g; 13.2 mmol) and pentafluorophenol (2.67 g; 14.5 mmol) were mixed with DMF (70 ml), cooled to 0°C with ice-water, and DCC (3.27 g; 15.8 mmol) was added. The ice bath was removed after 3 min and the mixture was stirred for 3 h at room temperature. The precipitated DCU was removed by filtration, washed with DMF, and the filtrate was evaporated to dryness, *in vacuo* (0.2 mmHg). The solid residue was treated with methylene chloride (250 ml), stirred vigorously for 15 min, filtered, washed twice with diluted sodium hydrogen carbonate and once with saturated sodium chloride, dried over magnesium sulfate, and evaporated to dryness, *in vacuo*. The solid residue was recrystallized from 2-propanol (150 ml) and the crystals were washed thoroughly with ether. Yield 3.40 g (55%). M.p. 241-245°C. Anal. for C₂₀H₁₂N₃F₅O₅. Found(calc.); C: 51.56(51.18); H: 2.77(2.58); N: 9.24(8.95) .¹H-NMR (90 MHz; CDCl₃) : 7.66 ppm (d,J=7.63Hz,1H,H-6); 7.37 (s,5H,Ph); 7.31 (d,J=7.63Hz,1H,H-5); 5.21 (s,2H,PhCH₂-); 4.97 (s,2H,NCH₂-). FAB-MS: 470 (M+1)

### EXAMPLE 15

### N-(N-Boc-2-aminoethyl)-N-[(N-4-Cbz-1-cytosinyl)acetyl]glycine (15)

To a solution of (N-Boc-2-aminoethyl)glycine **2**, in DMF, prepared as described in Example **2**, was added triethyl amine (7.00 ml; 50.8 mmol) and N-4-Cbz-N-1-carboxymethyl-cytosine pentafluorophenyl ester (**14**, 2.70 g; 5.75 mmol). After stirring the solution for 1 h at room temperature, methylene chloride (150 ml), saturated sodium chloride (250 ml), and 4 N hydrochloric acid to pH ∼1 were added. The organic layer was separated and washed twice with saturated sodium chloride, dried over magnesium sulfate, and evaporated to dryness, *in vacuo*, first with a water aspirator and then with an oil pump. The oily residue was treated with water (25 ml) and was again evaporated to dryness, *in vacuo*. This procedure then was repeated. The oily residue (2.80 g) was then dissolved in methylene chloride (100 ml), petroleum ether (250 ml) was added, and the mixture was stirred overnight. The title compound was isolated by filtration and washed with petroleum ether. Tlc (system 1) indicated substantial quantities of pentafluorophenol, but no attempt was made to remove it. Yield: 1.72 g (59%). M.p. 156°C(decomp.). ¹H-NMR (250 MHz, CDCl₃): Due to the limited rotation around the secondary amide bond several of the signals were doubled in the ratio 2:1, (indicated in the list by mj. for major and mi. for minor). 7.88 ppm (dd,1H,H-6); 7.39 (m,5H,Ph); 7.00 (dd,1H,H-5); 6.92 (b,1H,BocNH); 6.74 (b,1H,ZNH)-?; 5.19 (s,2H,Ph-CH₃); 4.81 ppm (s, mj., Cyt-CH₂-CO-); 4.62 ppm (s, mi., Cyt-CH₂-CO-); 4.23 (s, mi., CONRCH₂CO₂H); 3.98 ppm (s, mj., CONRCH₂CO₂H); 3.42-3.02 (unres. m, -CH₂CH₂- and water);1.37 (s,9H,tBu). FAB-MS: 504 (M+1); 448 (M+1-tBu).

### EXAMPLE 16

### N-(N-Boc-2-aminoethyl)-N-[(N-4-Cbz-1-cytosinyl)acetyl]glycine pentafluorophenyl ester (16)

N-(N-Boc-2-aminoethyl)-N-[(N-4-Cbz-1-cytosinyl)acetyl]-glycine (**15**, 1.50 g; 2.98 mmol) and pentafluorophenol (548 mg; 2.98 mmol) was dissolved in DMF (10 ml) Methylene chloride (10 ml) was added, the reaction mixture was cooled to 0°C in an ice bath, and DCC (676 mg; 3.28 mmol) was added. The ice bath was removed after 3 min and the mixture was stirred for 3 h at ambient temperature. The precipitate was isolated by filtration and washed once with methylene chloride. The precipitate was dissolved in boiling dioxane (150 ml) and the solution was cooled to 15°C, whereby DCU precipitated. The DCU was removed by filtration and the resulting filtrate was poured into water (250 ml) at 0°C. The title compound was isolated by filtration, was washed with water, and dried over an appropriate drying agent, *in* *vacuo*. Yield 1.30 g (65%). Analysis for C₂₉H₂₈N₅O₈F₅. Found(calc.); C: 52.63(52.02); H: 4.41(4.22); N: 10.55(10.46). ¹H-NMR (250 MHz; DMSO-d₆) : showed essentially the spectrum of the above acid, most probably due to hydrolysis of the ester. FAB-MS: 670 (M+1); 614 (M+1-tBu)

### EXAMPLE 17

### N-(N-Boc-2-aminoethyl)-N-[(N-4-Cbz-1-cytosinyl)acetyl]glycine (17)

N-(N-Boc-2-aminoethyl)glycine ethyl ester (**8**, 5.00 g; 20.3 mmol), DhbtOH (3.64 g; 22.3 mmol) and N-4-Cbz-N-1-carboxymethyl cytosine (**13**, 6.77 g; 22.3 mmol) were suspended in DMF (100 ml). Methylene chloride (100 ml) was then added. The solution was cooled to 0°C and DCC (5.03 g; 24.4 mmol) was added. The ice bath was removed after 2 h and stirring was continued for another hour at ambient temperature. The reaction mixture was evaporated to dryness, *in vacuo*. The residue was suspended in ether (100 ml) and stirred vigorously for 30 min. The solid material was isolated by filtration and the ether wash procedure was repeated twice. The material was then stirred vigorously for 15 min with dilute sodium hydrogencarbonate (aprox. 4% solution, 100 ml), filtered and washed with water. This procedure was then repeated once, which after drying left 17.0 g of yellowish solid material. The solid was then boiled with dioxane (200 ml) and filtered while hot. After cooling, water (200 ml) was added. The precipitated material was isolated by filtration, washed with water, and dried. According to HPLC (observing at 260 nm) this material has a purity higher than 99%, besides the DCU. The ester was then suspended in THF (100 ml), cooled to 0°C, and 1 N LiOH (61 ml) was added. After stirring for 15 minutes, the mixture was filtered and the filtrate was washed with methylene chloride (2 x 150 ml). The alkaline solution then was cooled to 0°C and the pH was adjusted to 2.0 with 1 N HCl. The title compound was isolated by filtration and was washed once with water, leaving 11.3 g of a white powder after drying. The material was suspended in methylene chloride (300 ml) and petroleum ether (300 ml) was added. Filtration and wash afforded 7.1 g (69%) after drying. HPLC showed a purity of 99% R_{T}= 19.5 min, and a minor impurity at 12.6 min (approx. 1%) most likely the Z-deprotected monomer. Anal. for C₂₃H₂₉N₅O₈ found(calc.) C: 54.16(54.87); H: 5.76(5.81) and N: 13.65(13.91). ¹H-NMR (250 MHz, DMSO-d₆). 10.78 (b.s, 1H, CO₂H) ; 7.88 (2 overlapping dublets, 1H, Cyt H-5); 7.41-7.32 (m, 5H, Ph); 7.01 (2 overlapping doublets, 1H, Cyt H-6); 6.94 & 6.78 (unres. triplets, 1H, BocNH); 5.19 (s, 2H, PhCH₂); 4.81 & 4.62 (s, 2H, CH₂CON); 4.17 & 3.98 (s, 2H, CH₂CO₂

### EXAMPLE 18

### 9-Carboxymethyl adenine ethyl ester (18)

Adenine (10.0 g, 74 mmol) and potassium carbonate (10.29 g, 74.0 mmol) were suspended in DMF and ethyl bromoacetate (8.24 ml, 74 mmol) was added. The suspension was stirred for 2.5 h under nitrogen at room temperature and then filtered. The solid residue was washed three times with DMF (10 ml). The combined filtrate was evaporated to dryness, *in vacuo*. The yellow-orange solid material was poured into water (200 ml) and 4 N HCl was added to pH≈6. After stirring at 0°C for 10 min, the solid was filtered off, washed with water, and recrystallized from 96% ethanol (150 ml). The title compound was isolated by filtration and washed thoroughly with ether. Yield 3.4 g (20%). M.p. 215.5-220°C. Anal. for C₉H₁₁N₅O₂ found(calc.): C: 48.86(48.65); H: 5.01(4.91); N: 31.66(31.42). ¹H-NMR (250 MHz; DMSO-d₆) : (s, 2H, H-2 & H-8), 7.25 (b. s., 2H, NH₂), 5.06 (s, 2H, NCH₂), 4.17 (q, 2H, J=7.11 Hz, OCH₂) and 1.21 (t, 3H, J=7.13 Hz, NCH₂). ¹³C-NMR. 152.70, 141.30, 61.41, 43.97 and 14.07. FAB-MS. 222 (MH+). IR: Frequency in cm⁻¹ (intensity). 3855 (54.3), 3274(10.4), 3246(14.0), 3117(5.3), 2989(22.3), 2940(33.9), 2876(43.4), 2753(49.0), 2346(56.1), 2106(57.1), 1899(55.7), 1762(14.2), 1742(14.2), 1742(1.0), 1671(1.8), 1644(10.9), 1606(0.6), 1582(7.1), 1522(43.8), 1477(7.2), 1445(35.8) and 1422(8.6). The position of alkylation was verified by X-ray crystallography on crystals, which were obtained by recrystallization from 96% ethanol.

Alternatively, 9-carboxymethyl adenine ethyl ester **18**, can be prepared by the following procedure. To a suspension of adenine (50.0 g, 0.37 mol) in DMF (1100 ml) in 2 L three-necked flask equipped with a nitrogen inlet, a mechanical stirrer and a dropping funnel was added 16.4 g (0.407 mol) haxane washed sodium hydride- mineral oil dispersion. The mixture was stirred vigorously for 2 hours, then ethyl bromacetate 75 ml, 0.67 mol) was added dropwise over the course of 3 hours. The mixture was stirred for one additional hour, whereafter tlc indicated complete conversion of adenine. The mixture was evaporated to dryness at 1 mmHg and water (500 ml) was added to the oily residue which caused crystallization of the title compound. the solid was recrystallized from 60% ethanol (600 ml). Yield after drying 53.7 (65.6%). HPLC (215 nm) purity > 99.5%.

### EXAMPLE 19

### N-6-Cbz-9-carboxymethyl adenine ethyl ester (19)

9-Carboxymethyladenine ethyl ester (**18,** 3.40 g, 15.4 mmol) was dissolved in dry DMF (50 ml) by gentle heating, cooled to 20°C, and added to a solution of N-ethyl-Cbz-imidazole tetrafluoroborate (62 mmol) in methylene chloride (50 ml) over a period of 15 min with ice-cooling. Some precipitation was observed. The ice bath was removed and the solution was stirred overnight. The reaction mixture was treated with saturated sodium hydrogen carbonate (100 ml). After stirring for 10 min, the phases were separated and the organic phase was washed successively with one volume of water, dilute potassium hydrogen sulfate (twice), and with saturated sodium chloride. The solution was dried over magnesium sulfate and evaporated to dryness, *in vacuo,* which afforded 11 g of an oily material. The material was dissolved in methylene chloride (25 ml), cooled to 0°C, and precipitated with petroleum ether (50 ml). This procedure was repeated once to give 3.45 g (63%) of the title compound. M.p. 132-35°C. Analysis for C₁₇H₁₇N₅O₄ found (calc.): C: 56.95(57.46); H: 4.71(4.82); N: 19.35(19.71). ¹H-NMR (250 MHz; CDCl₃) : 8.77 (s, 1H, H-2 or H-8); 7.99 (s, 1H, H-2 or H-8); 7.45-7.26 (m, 5H, Ph); 5.31 (s, 2H, N-CH₂) ; 4.96 (s, 2H, Ph-CH₂) ; 4.27 (q, 2H, J=7.15 Hz, CH₂CH₃) and 1.30 (t, 3H, J=7.15 Hz, CH₂CH₃). ¹³C-NMR: 153.09; 143.11; 128.66; 67.84; 62.51; 44.24 and 14.09. FAB-MS: 356 (MH+) and 312 (MH+-CO₂). IR: frequency in cm⁻¹ (intensity) . 3423 (52.1); 3182 (52.8); 3115(52.1); 3031(47.9); 2981(38.6); 1747(1.1); 1617(4.8); 15.87(8.4); 1552(25.2); 1511(45.2); 1492(37.9); 1465(14.0) and 1413(37.3).

### EXAMPLE 20

### N-6-Cbz-9-carboxymethyl adenine (20)

N-6-Cbz-9-carboxymethyladenine ethyl ester (**19**, 3.20 g; 9.01 mmol) was mixed with methanol (50 ml) cooled to 0°C. Sodium Hydroxide Solution (50 ml; 2N) was added, whereby the material quickly dissolved. After 30 min at 0°C, the alkaline solution was washed with methylene chloride (2x50ml). The aqueous solution was brought to pH 1.0 with 4 N HCl at 0°C, whereby the title compound precipitated. The yield after filtration, washing with water, and drying was 3.08 g (104%). The product contained salt and elemental analysis reflected that. Anal. for C₁₅H₁₃N₅O₄ found(caic.): C: 46.32(55.05); H: 4.24(4.00); N: 18.10(21.40) and C/N: 2.57(2.56). ¹H-NMR(250 MHz; DMSO-d₆): 8.70 (s, 2H, H-2 and H-8); 7.50-7.35 (m, 5H, Ph); 5.27 (s, 2H, N-CH₂); and 5.15 (s, 2H, Ph-CH₂). ¹³C-NMR. 168.77, 152.54, 151.36, 148.75, 145.13, 128.51, 128.17,127.98, 66.76 and 44.67.IR (KBr) 3484(18.3); 3109(15.9); 3087(15.0); 2966(17.1); 2927(19.9); 2383(53.8); 1960(62.7); 1739(2.5); 1688(5.2); 1655(0.9); 1594(11.7); 1560(12.3); 1530(26.3); 1499(30.5); 1475(10.4); 1455(14.0); 1429(24.5) and 1411(23.6). FAB-MS: 328 (MH+) and 284 (MH+-CO₂). HPLC (215 nm, 260 nm) in system 1: 15.18 min, minor impurities all less than 2%.

### EXAMPLE 21

### N-(N-Boc-2-aminoethyl]-N-[(N-6-Cbz-9-adeninyl)acetyl]glycine ethyl ester (21)

N-(N-Boc-2-aminoethyl)glycine ethyl ester **(8,** 2.00 g; 8.12 mmol), DhbtOH (1.46 g; 8.93 mmol) and N-6-Cbz-9-carboxymethyl adenine **(20,** 2.92 g; 8.93 mmol) were dissolved in DMF (15 ml). Methylene chloride (15 ml) then was added. The solution was cooled to 0°C in an ethanol/ice bath. DCC (2.01 g; 9.74 mmol) was added. The ice bath was removed after 2.5 h and stirring was continued for another 1.5 hour at ambient temperature. The precipitated DCU was removed by filtration and washed once with DMF (15 ml), and twice with methylene chloride (2 x 15 ml). To the combined filtrate was added more methylene chloride (100 ml). The solution was washed successively with dilute sodium hydrogen carbonate (2 x 100 ml), dilute potassium hydrogen sulfate (2 x 100 ml), and saturated sodium chloride (1 x 100 ml). The organic phase was evaporated to dryness, in vacuo, which afforded 3.28 g (73%) of a yellowish oily substance. HPLC of the raw product showed a purity of only 66% with several impurities, both more and less polar than the main peak. The oil was dissolved in absolute ethanol (50 ml) and activated carbon was added. After stirring for 5 minutes, the solution was filtered. The filtrate was mixed with water (30 ml) and was left with stirring overnight. The next day, the white precipitate was removed by filtration, washed with water, and dried, affording 1.16 g (26%) of a material with a purity higher than 98% by HPLC. Addition of water to the mother liquor afforded another 0.53 g with a purity of approx. 95%. Anal. for C₂₆H₃₃N₇O₇•H₂O found(calc.) C: 55.01(54.44; H: 6.85(6.15) and N: 16.47(17.09). ¹H-NMR (250 MHz, CDCl₃) 8.74 (s, 1H, Ade H-2); 8.18 (b. s, 1H, ZNH); 8.10 & 8.04 (s, 1H, H-8); 7.46-7.34 (m, 5H, Ph); 5.63 (unres. t, 1H, BocNH); 5.30 (s, 2H, PhCH₂); 5.16 & 5.00 (s, 2H, CH₂CON); 4.29 & 4.06 (s, 2H, CH₂CO₂H) ; 4.20 (q, 2H, OCH₂CH₃); 3.67-3.29 (m, 4H, CH₂CH₂); 1.42 (s, 9H, ^{t}Bu) and 1.27 (t, 3H, OCH₂CH₃). The spectrum shows traces of ethanol and DCU.

### EXAMPLE 22

### N-(N-Boc-2-aminoethyl)-N-[(N-6-Cbz-9-adeninyl)acetyl]glycine (22)

N-(N-Boc-2-aminoethyl)-N-[(N-6-Cbz-9-adeninyl)acetyl]-glycine ethyl ester (**21**, 1.48 g; 2.66 mmol) was suspended in THF (13 ml) and the mixture was cooled to 0°C. Lithium hydroxide (8 ml; 1 N) was added. After 15 min of stirring, the reaction mixture was filtered, extra water (25 ml) was added, and the solution was washed with methylene chloride (2 x 25 ml). The pH of the aqueous solution was adjusted to pH 2.0 with 1 N HCl. The precipitate was isolated by filtration, washed with water, and dried, affording 0.82 g (58%). The product reprecipitated twice with methylene chloride/ petroleum ether, 0.77 g (55%) after drying. M.p. 119°C (decomp.) Anal. for C₂₄H₂₉N₇O₇•H₂O found(calc.) C: 53.32(52.84); H: 5.71(5.73); N: 17.68(17.97). FAB-MS. 528.5 (MH+). ¹H-NMR (250 MHz, DMSO-d₆). 12.75 (very b, 1H, CO₂H); 10.65 (b. s, 1H, ZNH); 8.59 (d, 1H, J= 2.14 Hz, Ade H-2); 8.31 (s, 1H, Ade H-8); 7.49-7.31 (m, 5H, Ph); 7.03 & 6.75 (unresol. t, 1H, BocNH); 5.33 & 5.16 (s, 2H, CH₂CON); 5.22 (s, 2H, PhCH₂); 4.34-3.99 (s, 2H, CH₂CO₂H); 3.54-3.03 (m's, includes water, CH₂CH₂) and 1.39 & 1.37 (s, 9H, ^{t}Bu). ¹³C-NMR. 170.4; 166.6; 152.3; 151.5; 149.5; 145.2; 128.5; 128.0; 127.9; 66.32; 47.63; 47.03; 43.87 and 28.24.

### EXAMPLE 23

### 2-Amino-6-chloro-9-carboxymethylpurine (23)

To a suspension of 2-amino-6-chloropurine (5.02 g; 29.6 mmol) and potassium carbonate (12.91 g; 93.5 mmol) in DMF (50 ml) was added bromoacetic acid (4.70 g; 22.8 mmol). The mixture was stirred vigorously for 20 h. under nitrogen. Water (150 ml) was added and the solution was filtered through Celite to give a clear yellow solution. The solution was acidified to a pH of 3 with 4 N hydrochloric acid. The precipitate was filtered and dried, *in vacuo*, over an appropriate drying agent. Yield (3.02 g; 44.8%). ¹H-NMR(DMSO-d6): d = 4.88 ppm (s,2H); 6.95 (s,2H); 8.10 (s,1H).

### EXAMPLE 24

### 2-Amino-6-benzyloxy-9-carboxymethylpurine (24)

Sodium (2.0 g; 87.0 mmol) was dissolved in benzyl alcohol (20 ml) and heated to 130°C for 2 h. After cooling to 0°C, a solution of 2-amino-6-chloro-9-carboxymethylpurine (**23**, 4.05 g; 18.0 mmol) in DMF (85 ml) was slowly added, and the resulting suspension stirred overnight at 20°C. Sodium hydroxide solution (1N, 100 ml) was added and the clear solution was washed with ethyl acetate (3 x 100 ml). The water phase then was acidified to a pH of 3 with 4 N hydrochloric acid. The precipitate was taken up in ethyl acetate (200 ml), and the water phase was extracted with ethyl acetate (2 x 100 ml). The combined organic phases were washed with saturated sodium chloride solution (2 x 75 ml), dried with anhydrous sodium sulfate, and taken to dryness by evaporation, *in vacuo*. The residue was recrystallized from ethanol (300 ml). Yield after drying, *in vacuo*, over an appropriate drying agent: 2.76 g (52%). M.p. 159-65°C. Anal. (calc., found) C(56.18; 55.97), H(4.38; 4.32), N(23.4; 23.10). ¹H-NMR (DMSO-d₆): 4.82 ppm.(s,2H); 5.51 (s,2H); 6.45 (s,2H); 7.45 (m,5H); 7.82 (s,1H).

### EXAMPLE 25

### N-(N-Boc-2-aminoethyl)-N-[(2-Amino-6-benzyloxy-purine-9-yl)acetyl]glycine [BocGaeg monomer] (25)

2-Amino-6-benzyloxy-9-carboxymethyl-purine (**24,** 0.50 g; 1.67 mmol), N-Boc-aminoethyl glycine methyl ester (0.65 g; 2.80 mmol), diisopropylethyl amine (0.54 g; 4.19 mmol), andbromo-tris-pyrrolidino-phosphonium-hexafluoro-phosphate (PyBroP®) (0.798 g; 1.71 mmol) were stirred in DMF (2 ml) for 4 h. The clear solution was poured into an ice-cooled solution of sodium hydrogen carbonate (1 N; 40 ml) and extracted with ethyl acetate (3 X 40 ml). The organic layer was washed with potassium hydrogen sulfate solution (1 N; 2 X 40 ml), sodium hydrogen carbonate (1 N; 1 X 40 ml) and saturated sodium chloride solution (60 ml). After drying with anhydrous sodium sulfate and evaporation, *in vacuo,* the solid residue was recrystallized from ethyl acetate/hexane (20 ml (2:1)) to give the methyl ester in 63% yield (MS-FAB 514 (M+1). Hydrolysis was accomplished by dissolving the ester in ethanol/water (30 ml (1:2)) containing conc. sodium hydroxide (1 ml). After stirring for 2 h, the solution was filtered and acidified to a pH of 3, by the addition of 4 N hydrochloric acid. The title compound was obtained by filtration. Yield: 370 mg (72% for the hydrolysis) . Purity by HPLC was more than 99%. Due to the limited rotation around the secondary amide several of the signals were doubled in the ratio 2:1 (indicated in the list by mj. for major and mi. for minor) . ¹H-NMR(250, MHz, DMSO-d₆) : d = 1.4 ppm. (s,9H); 3.2 (m,2H); 3.6 (m,2H); 4.1 (s, mj., CONRCH₂COOH) ; 4.4 (s, mi., CONRCH₂COOH); 5.0 (s, mi., Gua-CH₂CO-); 5.2 (s, mj., Gua-CH₂CO) ; 5.6 (s,2H); 6.5 (s,2H); 6.9 (m, mi., BocNH); 7.1 (m, mj., BocNH); 7.5 (m.,3H); 7.8 (s,1H); 12,8 (s;1H). ¹³C-NMR. 170.95; 170.52; 167.29; 166.85; 160.03; 159.78; 155.84; 154.87; 140.63; 136.76; 128.49; 128.10; 113.04; 78.19; 77.86; 66.95; 49.22; 47.70; 46.94; 45.96; 43.62; 43.31 and 28.25.

### EXAMPLE 26

### Synthesis of PNA Oligomers by Solid Phase, General Procedure

The functionalized resin is measured out to typically provide 0.1-1.0 millimoles of functionality, (functionalities attached to resins are commercially available through various sources e.g. Peptides International, Kentucky). This weight of resin is suspended in a 1:1 (v:v) dichloromethane:dimethylformamide solution (30mL/1g of resin) and allowed to swell for a period of time if desired. The solvent is then removed by filtration and the resin resuspended in trifluoroacetic acid (1mL/1gm of resin) and shaken for 3 minutes. The trifluoroacetic acid is removed by filtration and this step is repeated twice. The resin is washed three times with a solution of 1:1 (v:v) dichloromethane:dimethylformamide. The resulting resin is resuspended in pyridine solution (5mL/100mg of resin) and vacuum filtered to remove the pyridine. This step is repeated twice. The resin is suspended in 1:1 (v:v) pyridine:dimethylformamide and to this suspension is added the desired PNA monomer (2-10 molar equivalents), TBTU (1.9-9.9 molar equivalents), and di-isopropylethylamine (5-20 molar equivalents) such that the final concentration of PNA monomer is 0.2M. The suspension is shaken for 15-60 minutes and the spent coupling solution is removed by filtration. The resin is washed with pyridine three times, and any unreacted amines are capped using Rapoport's Reagent, 5 equivalents in DMF for 5 minutes. The resin is then washed three times with pyridine followed by three washes with a solution of 1:1 (v:v) dichloromethane: dimethylformamide (5mL/100mg of resin). At this point, the resin is ready for the next coupling reaction and this procedure is repeated until the desired PNA is assembled on the resin.

### Specific Examples of Amino Ethyl Glycine (aeg-) PNAs and aeg-PNA Derivatives Prepared by this General Method

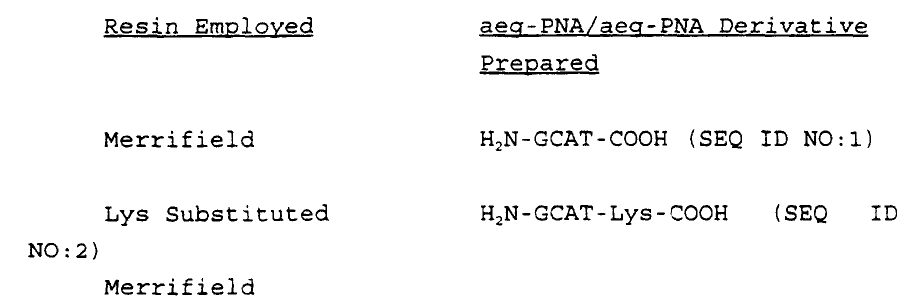

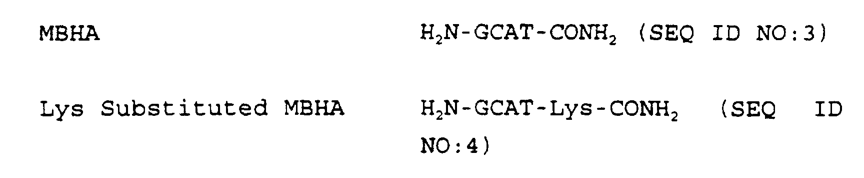

### EXAMPLE 27

### Capping of the PNA

PNA can be capped by a non-PNA moiety on the N terminus by following the procedures described in Example **26** and substituting a desired carboxylic acid-based capping reagent for the PNA monomer in the final coupling step.

### Specific Examples of aeg-PNAs and aeg-PNA Derivatives Prepared by this General Method

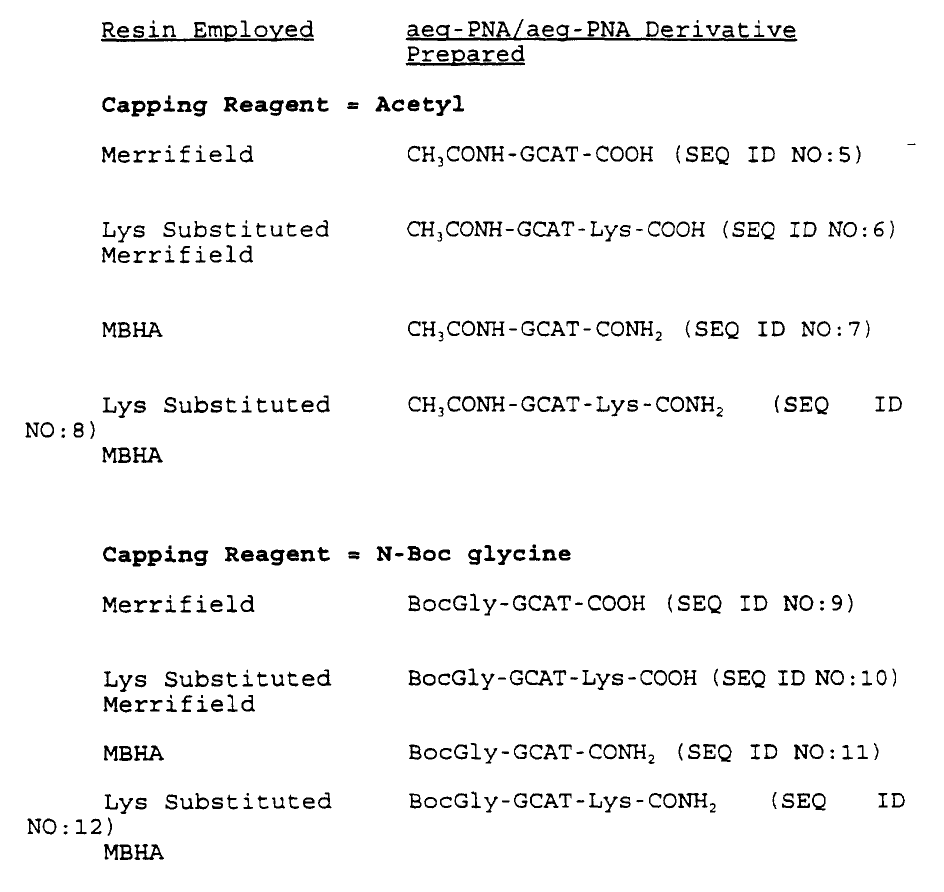

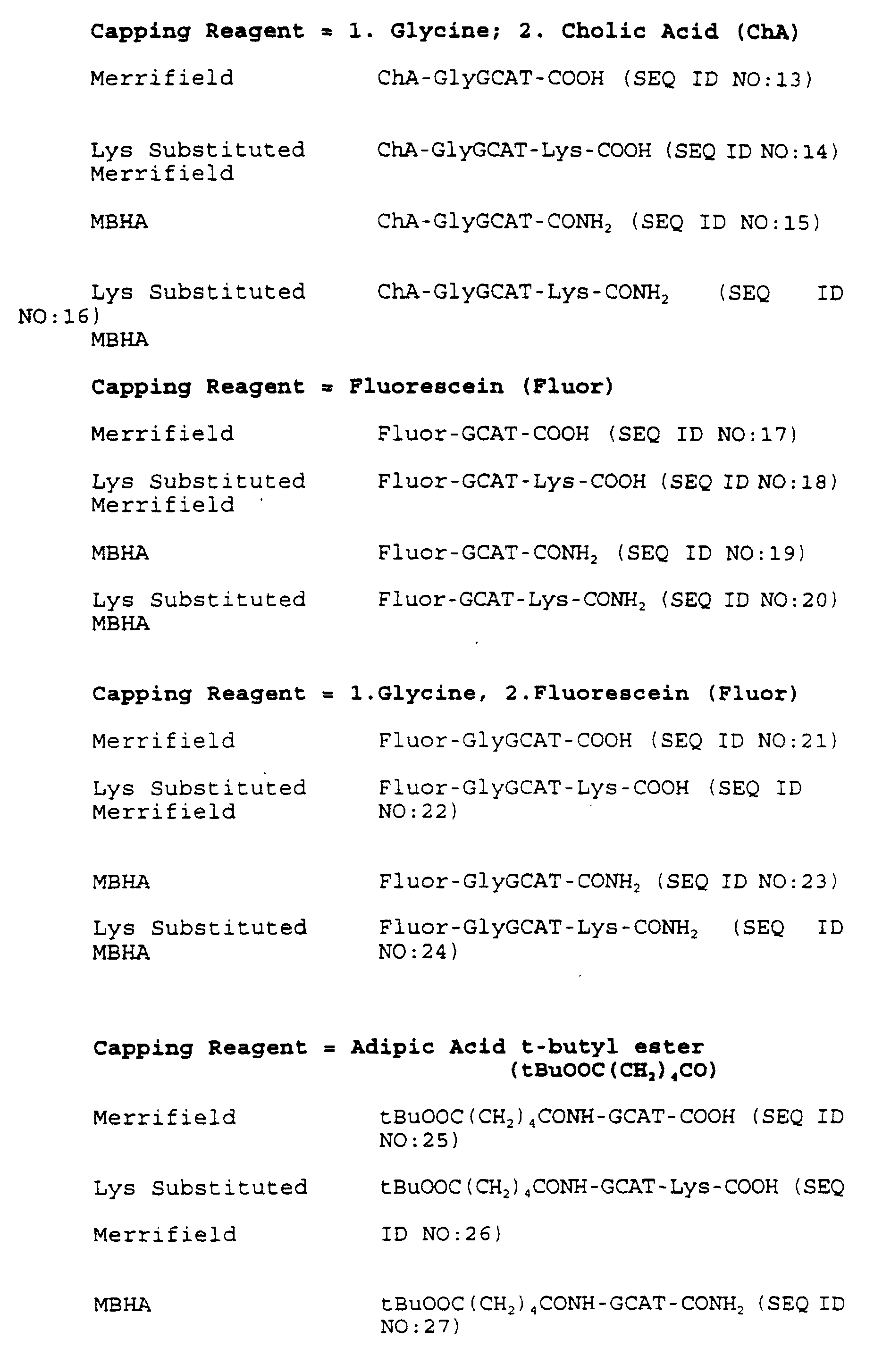

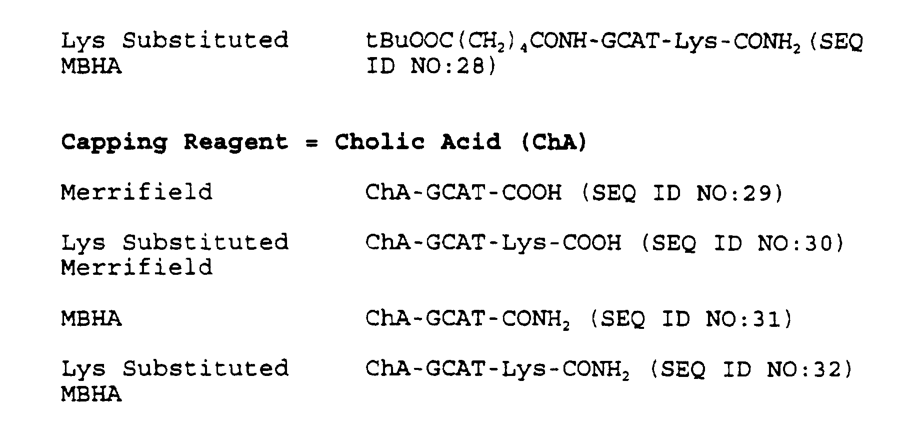

### EXAMPLE 28

### General Cleavage Reaction

The completed PNA or PNA derivative may be cleaved from the resin and isolated via the following. The resin is washed twice with trifluoroacetic acid, then suspended in a mixture of thioanisole, m-cresol, trifluoromethanesulfonic acid, and trifluoroacetic acid in a ratio of 1:1:2:6, 200 µl/l µmole, for 1 hour at room temperature. The cleavage solution is then drained into ether, 1 ml/µmole, and the above process is repeated. The precipitate is then collected by either filtration or centrifugation.

### EXAMPLE 29

### General Purification Procedure

The isolated PNA or PNA derivative is purified by preparative reverse phase HPLC using a Waters µBondapak Phenyl-C18 column (19mmX150mm) and eluting with a gradient comprised of a solution of 9:1 acetonitrile:0.050M NH₄OAc, pH 6, .1% hexafluoroisopropanol added to a solution of 0.050M NH₄OAc, pH 6, .1% hexafluoroisopropanol. The eluent can be adjusted for the particular PNA or PNA derivative being purified.

### EXAMPLE 30

### N-[(N-phthaloyl)(1-methyl-O-benzyl)-2-aminoethyl]-N-[(1-thyminyl) acetyl] glycine (30)

### A. (O-Benzyl) Boc Serine methyl ester (30A)

(O-benzyl) Boc Serine 2.00 g (6.8 mmoles) was dissolved in 50 ml of ether. To this solution was added an ether solution containing freshly prepared diazomethane prepared from 5 g of Diazald using the procedure from Aldrich Technical Bulletin number AL-180. After stirring the solution for 30 minutes, glacial acetic acid was added dropwise until the solution became clear. The resulting solution was concentrated in vacuo to yield 2.00 g, 95 % as a clear oil. ¹H: (CDCl₃); 7.3 (d, 5H), 5.5 (bs, 1H), 4.5 (s, 2H), 3.8 (m, 2 H), 3.7 (s, 3 H), 1.4 (s, 9H)

### B. 1-O-Benzyl-2-N-Boc-2,3-propandiol (30B)

Compound 30A was dissolved in 50 ml of methanol and cooled to 0 °C. To this was added 300 mg of lithium borohydride in small portions, waiting for the gas evolution to cease between additions. When all of the hydride had been added the reaction was refluxed for 1 hour. It was then cooled to 0oC, and quenched by the dropwise addition of saturated sodium bicarbinate. The solution was extracted twice with 50 ml portions of chloroform. The combined organic extracts were washed again with saturated sodium bicarbonate, followed by saturated sodium chloride solutions. The chloroform solution was dried over magnesium sulfate and concentrated to an oil. The oil was chromatographed over silica gel eluting with 7% methanol in dichloromethane, isolating 1.30 g as a clear oil. 1H: (CDC13); 7.3 (s, 5 H), 5.2 (bs, 1 H), 4.5 (s, 2 H), 3.9-3.5 (mm, 5 H), 1.4 (s, 9 H)

### C. 1-O-benzyl-2-N-Boc-3-phthaloyl-2,3-diaminopropanol (30C)

Compound **30B** was dissolved in 100 ml of freshly distilled THF. To this solution was added the following reagents; triphenyl phosphine, (1.26 g 4.82 mmol), diethyl azodicarboxylate (0.839 g 4.82 mmol), and phthalimide (0.710 g 4.82 mmol), and the reaction stirred overnight at room temperature. After stirring the solution was concentrated to a yellow solid and chromatographed over silica gel using 30% ethyl acetate in hexanes. The product was isolated as a white solid weighing 1.60 g, 97 %. 1H: (CDCl₃); 7.9 (m, 2 H), 7.7 (m, 2H), 7.3 (s, 5 H), 5.1 (d, 1 H), 4.5 (s, 2 H), 4.0-3.5 (mm, 5 H), 1.3 (s, 9 H).

### D. N-[(N-phthaloyl)(1-methyl-O-benzyl)-2-aminoethyl]-glycine methyl ester (30D)

Compound 30C (1.00 g, 2.9 mmol) is dissolved in 20 ml dichloromethane and cooled to 0°C. To this is added 20ml TFA and the reaction is allowed to warm to room temperature. Upon completion, the reaction is concentrated in vacuo and then redissolved in dichloromethane and washed with 1 N NaOH and brine, the organic solution is dried and concentrated to give the free amine. This amine is then dissolved in anhydrous THF with 2 eq of triethylamine and cooled to 0°C. Methyl bromoacetate (0.500 g, 3.2 mmol) is added and the reaction is allowed to stir at room temperature until complete. Standard aqueous workup and chromatography yields the title compound.

### E. N-[(N-phthaloyl)(1-methyl-O-benzyl)-2-aminoethyl]-N-[(1-thyminyl)acetyl]glycine (30)

The functionalized backbone monomer of Example **30D** is dissolved in THF and treated with 3 eq of triethyl amine. To this 1 eq of 1-chlorocarbonylmethyl thymine is added dropwise as a THF solution over 30 min. The resulting solution is stirred overnight or until complete. Standard acidic workup and chromatography yield the title compound.

### EXAMPLE 31

### N-[(N-phthaloyl)(1-methyl-O-benzyl)-2-aminoethyl]-N-[(9-adeninyl)acetyl]glycine (31)

### A. 9-Carboxymethyl adenine ethyl ester (31A)

Adenine (10.0 g, 74 mmol) and potassium carbonate (10.29 g, 74.0 mmol) were suspended in DMF and ethyl bromoacetate (8.24 ml, 74 mmol) was added. The suspension was stirred for 2.5 h under nitrogen at room temperature and then filtered. The solid residue was washed three times with DMF (10 ml). The combined filtrate was evaporated to dryness, *in vacuo.* The yellow-orange solid material was poured into water (200 ml) and 4 N HCl was added to pH≈6. After stirring at 0°C for 10 min, the solid was filtered off, washed with water, and recrystallized from 96% ethanol (150 ml). The title compound was isolated by filtration and washed thoroughly with ether. Yield 3.4 g (20%). M.p. 215.5-220°C. Anal. for C₉H₁₁N₅O₂ fcund(calc.) : C: 48.86(48.65); H: 5.01(4.91); N: 31.66(31.42). ¹H-NMR (250 MHz; DMSO-d₆) : (s, 2H, H-2 & H-8), 7.25 (b. s., 2H, NH₂), 5.06 (s, 2H, NCH₂), 4.17 (q, 2H, J=7.11 Hz, OCH₂) and 1.21 (t, 3H, J=7.13 Hz, NCH₂). ¹³C-NMR. 152.70, 141.30, 61.41, 43.97 and 14.07. FAB-MS. 222 (MH+). IR: Frequency in cm⁻¹ (intensity). 3855 (54.3), 3274(10.4), 3246(14.0), 3117(5.3), 2989(22.3), 2940(33.9), 2876(43.4), 2753(49.0), 2346(56.1), 2106(57.1), 1899(55.7), 1762(14.2), 1742(14.2), 1742(1.0), 1671(1.8), 1644(10.9), 1606(0.6), 1582(7.1), 1522(43.8), 1477(7.2), 1445(35.8) and 1422(8.6). The position of alkylation was verified by X-ray crystallography on crystals, which were obtained by recrystallization from 96% ethanol.

Alternatively, 9-carboxymethyl adenine ethyl ester can be prepared by the following procedure. To a suspension of adenine (50.0 g, 0.37 mol) in DMF (1100 ml) in 2 L three-necked flask equipped with a nitrogen inlet, a mechanical stirrer and a dropping funnel was added 16.4 g (0.407 mol) hexane washed sodium hydride- mineral oil dispersion. The mixture was stirred vigorously for 2 hours, whereafter ethyl bromacetate 75 ml, 0.67 mol) was added dropwise over the course of 3 hours. The mixture was stirred for one additional hour, whereafter tlc indicated complete conversion of adenine. The mixture was evaporated to dryness at 1 mmHg and water (500 ml) was added to the oily residue which caused crystallization of the title compound. The solid was recrystallized from 96% ethanol (600 ml). Yield after drying 53.7 (65.6%). HPLC (215 nm) purity > 99.5%.

### B. N-6-Cbz-9-carboxymethyl adenine ethyl ester (31B)

9-Carboxymethyladenine ethyl ester (**31A**, 3.40 g, 15.4 mmol) was dissolved in dry DMF (50 ml) by gentle heating, cooled to 20°C, and added to a solution of N-ethyl-Cbz-imidazole tetrafluoroborate (62 mmol) in methylene chloride (50 ml) over a period of 15 min with ice-cooling. Some precipitation was observed. The ice bath was removed and the solution was stirred overnight. The reaction mixture was treated with saturated sodium hydrogen carbonate (100 ml). After stirring for 10 min, the phases were separated and the organic phase was washed successively with one volume of water, dilute potassium hydrogen sulfate (twice), and with saturated sodium chloride. The solution was dried over magnesium sulfate and evaporated to dryness, *in vacuo,* which afforded 11 g of an oily material. The material was dissolved in methylene chloride (25 ml), cooled to 0°C, and precipitated with petroleum ether (50 ml). This procedure was repeated once to give 3.45 g (63%) of the title compound. M.p. 132-35°C. Analysis for C₁₇H₁₇N₅O₄ found (calc.): C: 56.95(57.46); H: 4.71(4.82); N: 19.35(19.71). ¹H-NMR (250 MHz; CDCl₃): 8.77 (s, 1H, H-2 or H-8); 7.99 (s, 1H, H-2 or H-8); 7.45-7.26 (m, 5H, Ph); 5.31 (s, 2H, N-CH₂); 4.96 (s, 2H, Ph-CH₂); 4.27 (q, 2H, J=7.15 Hz, CH₂CH₃) and 1.30 (t, 3H, J=7.15 Hz, CH₂CH₃). ¹³C-NMR: 153.09; 143.11; 128.66; 67.84; 62.51; 44.24 and 14.09. FAB-MS: 356 (MH+) and 312 (MH+-CO₂). IR: frequency in cm⁻¹ (intensity) . 3423 (52.1); 3182 (52.8); 3115(52.1); 3031(47.9); 2981(38.6); 1747(1.1); 1617(4.8); 15.87(8.4); 1552(25.2); 1511(45.2); 1492(37.9); 1465(14.0) and 1413(37.3).

### C. N-6-Cbz-9-carboxymethyl adenine (31C)

N-6-Cbz-9-carboxymethyladenine ethyl ester (**31B**, 3.20 g; 9.01 mmol) was mixed with methanol (50 ml) cooled to 0°C. Sodium hydroxide solution (50 ml; 2N) was added, whereby the material quickly dissolved. After 30 min at 0°C, the alkaline solution was washed with methylene chloride (2x50ml). The aqueous solution was brought to pH 1.0 with 4 N HCl at 0°C, whereby the title compound precipitated. The yield after filtration, washing with water, and drying was 3.08 g (104%). The product contained salt and elemental analysis reflected that. Anal. for C₁₅H₁₃N₅O₄ found(calc.): C: 46.32(55.05); H: 4.24(4.00); N: 18.10(21.40) and C/N: 2.57(2.56). ¹H-NMR(250 MHz; DMSO-d₆): 8.70 (s, 2H, H-2 and H-8); 7.50-7.35 (m, 5H, Ph); 5.27 (s, 2H, N-CH₂); and 5.15 (s, 2H, Ph-CH₂). ¹³C-NMR. 168.77, 152.54, 151.36, 148.75, 145.13, 128.51, 128.17,127.98, 66.76 and 44.67.IR (KBr) 3484(18.3); 3109(15.9); 3087(15.0); 2966(17.1); 2927(19.9); 2383(53.8); 1960(62.7); 1739(2.5); 1688(5.2); 1655(0.9); 1594(11.7); 1560(12.3); 1530(26.3); 1499(30.5); 1475(10.4); 1455(14.0); 1429(24.5) and 1411(23.6). FAB-MS: 328 (MH+) and 284 (MH+-CO₂). HPLC (215 nm, 260 nm) in system 1: 15.18 min, minor impurities all less than 2%.

### D. N-[(N-phthaloyl)(1-methyl-O-benzyl)-2-aminoethyl]-N-[(9-adeninyl]acetyl]glycine (31)

N-6-Cbz-9-carboxymethyl adenine **(31C)** is converted to the acid chloride using standard techniques, and treated according to the procedure of Example **30D** to yield the title comound.

### EXAMPLE 32

### N-[(N-phthaloyl)(1-methyl-O-benzyl)-2-aminoethyl]-N-[(1-cytosinyl)acetyl]glycine (32)

### A. N-4-Cbz cytosine (32A)

Over a period of about 1 h, Cbz chloride (52 ml; 0.36 mol) was added dropwise to a suspension of cytosine (8, 20.0 g;0.18 mol) in dry pyridine (1000 ml) at 0°C under nitrogen in oven-dried equipment. The solution then was stirred overnight, after which the pyridine suspension was evaporated to dryness, *in vacuo.* Water (200 ml) and 4 N hydrochloric acid were added to reach pH 1. The resulting white precipitate was filtered off, washed with water and partially dried by air suction. The still-wet precipitate was boiled with absolute ethanol (500 ml) for 10 min, cooled to 0°C, filtered, washed thoroughly with ether, and dried, *in vacuo*. Yield 24.7 g (54%). M.p.>250°C. Anal. for C₁₂H₁₁N₃O₃. Found (calc.); C: 58.59(58.77); H: 4.55(4.52); N: 17.17(17.13). No NMR spectra were recorded since it was not possible to get the product dissolved.

### B. N-4-Cbz-N-1-carboxymethyl cytosine (32B)

In a three necked round bottomed flask equipped with mechanical stirring and nitrogen coverage was placed methyl bromacetate (7.82 ml;82.6 mmol) and a suspension of N-4-Cbz-cytosine **(32A,** 21.0 g;82.6 mmol) and potassium carbonate (11.4 g;82.6 mmol) in dry DMF (900 ml). The mixture was stirred vigorously overnight, filtered, and evaporated to dryness, *in vacuo.* Water (300 ml) and 4 N hydrochloric acid (10 ml) were added, the mixture was stirred for 15 minutes at 0°C, filtered, and washed with water (2 x 75 ml). The isolated precipitate was treated with water (120 ml), 2N sodium hydroxide (60 ml), stirred for 30 min, filtered, cooled to 0°C, and 4 N hydrochloric acid (35 ml) was added. The title compound was isolated by filtration, washed thoroughly with water, recrystallized from methanol (1000 ml) and washed thoroughly with ether. This afforded 7.70 g (31%) of pure compound. The mother liquor from the recrystallization was reduced to a volume of 200 ml and cooled to 0°C. This afforded an additional 2.30 g of a material that was pure by tlc but had a reddish color. M.p. 266-274°C. Anal. for C₁₄H₁₃N₃O₅. Found(calc.); C: 55.41(55.45); H: 4.23(4.32); N: 14.04(13.86). ¹H-NMR (90 MHz; DMSO-d₆) : 8.02 ppm (d,J=7.32Hz,1H,H-6); 7.39 (s,5H,Ph); 7.01 (d,J=7.32Hz,1H,H-5); 5.19 (s,2H,PhCH₂-); 4.52 (s,2H).

### C. N-[(N-phthaloyl)(1-methyl-O-benzyl)-2-aminoethyl]-N-[(1-cytosinyl)acetyl]glycine (32)

N-4-Cbz-N-1-carboxymethyl cytosine is converted to the acid chloride according to standard techniques, and treated according to the procedure of Example **30D** to yield the title comound.

### EXAMPLE 33

### N-[(N-phthaloyl)(1-methyl-O-benzyl)-2-aminoethyl]-N-[(2-Amino-6-benzyloxy-purine-9-yl)acetyl]glycine (33)

### A. 2-Amino-6-chloro-9-carboxymethylpurine (33A)

To a suspension of 2-amino-6-chloropurine (5.02 g; 29.6 mmol) and potassium carbonate (12.91 g; 93.5 mmol) in DMF (50 ml) was added bromoacetic acid (4.70 g; 22.8 mmol). The mixture was stirred vigorously for 20 h under nitrogen. Water (150 ml) was added and the solution was filtered through Celite to give a clear yellow solution. The solution was acidified to a pH of 3 with 4 N hydrochloric acid. The precipitate was filtered and dried, *in vacuo*, over an appropriate drying agent. Yield (3.02 g; 44.8%). ¹H-NMR(DMSO-d6) : d = 4.88 ppm (s,2H); 6.95 (s,2H); 8.10 (s,1H).

### B. 2-Amino-6-benzyloxy-9-carboxymethylpurine (33B)

Sodium (2.0 g; 87.0 mmol) was dissolved in benzyl alcohol (20 ml) and heated to 130°C for 2 h. After cooling to 0°C, a solution of 2-amino-6-chloro-9-carboxymethylpurine (**33A**, 4.05 g; 18.0 mmol) in DMF (85 ml) was slowly added, and the resulting suspension stirred overnight at 20°C. Sodium hydroxide solution (1N, 100 ml) was added and the clear solution was washed with ethyl acetate (3 x 100 ml). The water phase then was acidified to a pH of 3 with 4 N hydrochloric acid. The precipitate was taken up in ethyl acetate (200 ml), and the water phase was extracted with ethyl acetate (2 x 100 ml). The combined organic phases were washed with saturated sodium chloride solution (2 x 75 ml), dried with anhydrous sodium sulfate, and taken to dryness by evaporation, *in vacuo.* The residue was recrystallized from ethanol (300 ml). Yield after drying, *in vacuo,* over an appropriate drying agent: 2.76 g (52%). M.p. 159-65°C. Anal. (calc., found) C(56.18; 55.97), H(4.38; 4.32), N(23.4; 23.10). ¹H-NMR (DMSO-d₆): 4.82 ppm.(s,2H); 5.51 (s,2H); 6.45 (s,2H); 7.45 (m,5H); 7.82 (s,1H).

### C. N-[(N-phthaloyl)(1-methyl-O-benzyl)-2-aminoethyl]-N-[(2-Amino-6-benzyloxy-purine-9-yl]acetyl]glycine (33)

2-Amino-6-benzyloxy-9-carboxymethyl-purine (**33B**, 1.67 mmol), Compound **31D** (2.80 mmol), diisopropylethyl amine (4.19 mmol), and bromo-tris-pyrrolidino-phosphonium-hexafluoro-phosphate (PyBroP®) (1.71 mmol) are stirred in DMF (2 ml) for 4 h. The clear solution is poured into an ice-cooled solution of sodium hydrogen carbonate (1 N; 40 ml) and extracted with ethyl acetate (3 X 40 ml). The organic layer is washed with potassium hydrogen sulfate solution (1 N; 2 X 40 ml), sodium hydrogen carbonate (1 N; 1 X 40 ml) and saturated sodium chloride solution (60 ml). The organic layer is separated, dried with anhydrous sodium sulfate and concentrated in *vacuo.* The solid residue is recrystallized from ethyl acetate/hexane (20 ml (2:1)) to give the methyl ester. Hydrolysis is accomplished by dissolving the ester in ethanol/water (30 ml (1:2)) containing conc. sodium hydroxide (1 ml). After stirring for 2 h, the solution is filtered and acidified to a pH of 3, by the addition of 4 N hydrochloric acid. The title compound is obtained by filtration. Purity is judged by HPLC.

### EXAMPLE 34

### N-[(N-Boc)(2-methyl-O-benzyl)-2-aminoethyl]-N-[(1-thyminyl)-acetyl]glycine (34)

### A. (O-benzyl)2-N-Boc-diaminopropanol (34A)

Compound **30C** is dissolved in 200 ml ethanol in a 500 ml flask. Hydrazine is added to the stirring reaction mixture. The mixture is heated to 60-65° in an oil bath and refluxed 14 hours. Solvent is evaporated *in vacuo.* The residue is dissolved in dichloromethane (250 ml) and extracted twice with an equal volume of NH₄OH. The organic layer is evaporated to yield the crude compound **34A.** The product may be used without further purification or can be further purified by HPLC. NMR is used to assay product purity.

### B. N-[(N-Boc)(2-methyl-O-benzyl)-2-aminoethyl]-N-glycine methyl ester (34B)

Compound **34A** is treated with methyl bromoacetate according to the procedure of Example **30B** to yield the title compound.

### C. N-[(N-Boc)(2-methyl-O-benzyl)-2-aminoethyl]-N-[(1-thyminyl)acetyl] glycine (34)

Compound **34B** is treated with chlorocarbonylmethylthymine according to the procedure of Example 30C to yield the title compound.

### EXAMPLE 35

### N-[(N-Boc)(1-hydroxymethyl)-2-aminoethyl]-N-[(1-thyminyl)-acetyl]glycine (35)

Compound 30 is treated with hydrazine according to the procedure of Example **34A** to convert the phthalimido group to the free amine, which is then treated with Boc₂O/NaOH to yield the N-Boc intermediate which is then treated with H₂/Pd/C to yield the title compound.

### EXAMPLE 36

### N-[(N-Boc)(2-hydroxymethyl)-2-aminoethyl]-N-[(1-thyminyl)-acetyl]glycine (36)

Compound **34** is treated according to the procedures of Example **35** to yield the title compound.

### EXAMPLE 37

### N-[(N-Boc)(1-formyl)-2-aminoethyl]-N-[(1-thyminylacetyl] glycine (37A) and N-[(N-Boc)(2-formyl)-2-aminoethyl-N-[(1-thyminyl]acetyl]glycine (37B)

Compound **35** or **36** is treated with oxalyl chloride/DMSO according to the procedure of Omura and Swern, Tetrahedron **34** 1651 (1978) to yield the respective title compound.

### EXAMPLE 38

### N-[(N-Boc)(1-aminomethyl)-2-aminoethyl]-N-(1-thyminylacetyl]glycine (38A) and N-[(N-Boc)(2-aminomethyl)-2-aminoethyl]-N-[(1-thyminyl)acetyl]glycine (38B)

Compound **37A** or **37B** is dissolved in 4 ml methanol. Sodium acetate pH 4.0 (2 ml), sodium cyanoborohydride (0.02 grams, 0.3 mmol) and ammonia in water (300 µl) are added to the reaction mixture, which is stirred 2 hours, after which it is concentrated *in vacuo*. The residue is dissolved in dichloromethane (50 mL) and washed with an equal volume saturated NaHCO₃. The aqueous layer is washed with dichloromethane and the combined organic extracts washed with an equal volume saturated NaCl. The aqueous layer is washed with dichloromethane and the combined organic layers dried over MgSO₄ and concentrated. The residue is chromatographed on a silica gel column, eluting with a gradient of 50% ethyl acetate in hexanes to 100% ethyl acetate. The desired product (0.15 grams, 80%) elutes with 10% Methanol/90% ethyl acetate.

### EXAMPLE 39

### Synthesis of N-(N-Boc-2-aminoethyl)-N-[(1-thyminyl)acetyl]-L-serine(OBn) (39)

### A. N-Boc-aminoacetaldehyde (39A)

3-(Boc-amino)-1,2-propanediol (21g, 110 mmol) was dissolved in water (200ml) and stirred with potassium m-periodate (25.3g, 110 mmol) at room temperature under argon for 2h. The reaction mixture was filtered and washed with methylene chloride (100ml). The aqueous layer was extracted with methylene chloride (3x150ml). The organic extract was washed with brine (50ml), dried and evaporated to dryness to give a light pink oil. This oil was purified by flash column chromatography using silica gel and CH₂Cl₂/ethyl acetate as the eluent. Yield was 14g (80%). ¹HNMR (CDCl₃) d 1.45 (s, 9H, t-Butyl), 4.08 (d, 2H, C*H*₂), 5.24 (bs, 1H, N*H*) and 9.67 (s, 1H, C*H*O).

### B. N-(N-Boc-2-aminoethyl)-L-serine(O-benzyl) methyl ester (39B)

Method A: L-Serine(OBn) methyl ester (16.93g, 81 mmol) and N-Boc-aminoacetaldehyde (**39A**) (12.88g, 81 mmol) were dissolved in dry CH₂Cl₂ (200ml) and allowed to stir at room temperature under an atmosphere of argon. Glacial acetic acid (1ml) was added and the mixture was stirred for an additional 15 minutes. Sodiumtriacetoxyborohydride (18.99g, 90 mmol) was added at room temperature in one lot. After stirring for 3h under argon, the reaction was diluted with CH₂Cl₂ (100ml). The organic layer was washed first with sat. NaHCO₃ (100ml) to pH 7 followed by washing with water (50ml) and then brine (50ml). The organic extract was dried and evaporated to dryness. The residue was purified by flash chromatography using CH₂Cl₂/acetone as the eluent. The fractions having the required product were collected and evaporated to dryness to give 8.0g (28%) of pure product as foam. ¹HNMR (CDCl₃) δ 1.38 (s, 9H, t-Butyl), 1.95 (bs, 1H, *NH*), 2.60 (m, 1H, C*H*₂), 2.80 (m, 1H, C*H*₂), 3.18 (m, 2H, C*H*₂), 3.42 (t, 1H), 3.65 (m, 2H, C*H*₂), 3.72 (s, 3H, OC*H*₃), 4.50 (s, 2H, OC*H*₂), 5.04 (bs, 1H, N*H*) and 7.30 (m, 5H, Ar*H*). Anal. Calcd for C₁₁H₂₂N₂O₅ : C, 50.37; H, 8.45; N, 10.68. Found: C, 50.60; H, 8.43; N, 10.83.

Method B: L-Serine(OBn) methyl ester (2.6g, 12.44 mmol) and N-Boc-aminoacetaldehyde (**39A**) (2.07g, 13 mmol) were dissolved in dry CH₃OH (35ml) and placed in a parr bottle. To this solution anhydrous NaOAc (1.77g, 13 mmol) and Pd/C (10%, 0.6g) were added under an atmosphere of argon. The reaction mixture was hydrogenated at 3psi of hydrogen for 2h. The catalyst was filtered, washed with methanol (20ml) and the combined filtrate was evaporated to dryness. The residue was partitioned between CH₂Cl₂ (150ml) and sat. NaHCO₃(100ml) and extracted with CH₂Cl₂. The organic extract was washed with water (100ml) and brine, dried and evaporated to dryness. The residue was purified by flash column chromatography using CH₂Cl₂/acetone as the eluent. The pure fractions were collected and evaporated to dryness to give 3.9g(89%) of the product as foam.

### C. N-(N-Boc-2-aminoethyl)-N-[(1-thyminyl)acetyl]-L-serine(OBn) methyl ester (39C)

The substrate **39B** (7.0g, 20 mmol) and N-1-carboxymethylthymine (**3,** 3.68g, 20 mmol) were dissolved in dry DMF/CH₂Cl₂ (1:1; 200ml) and cooled to 0°C in an ice bath under an atmosphere of argon. To this cold solution was added DhbtOH (3.62g, 20 mmol) followed by EDC (4.20g, 22 mmol). The reaction mixture was stirred at room temperature under argon overnight and evaporated to dryness. The residue was dissolved in a mixture of CH₂Cl₂ (200 ml) and water (100ml) and extracted in CH₂Cl₂. The organic extract was washed with 5% NaHCO₃ (100ml), water (100ml) and brine(100ml). The CH₂Cl₂ layer was dried over anhydrous Na₂SO₄ and evaporated to dryness. The residue was purified by flash column chromatography over silica gel using CH₂Cl₂/MeOH as the eluent. The pure fractions were collected and evaporated to give 9g (87%) of the product as a foam. ¹HNMR (CDCl₃) d 1.42 (s, 9H, t-Butyl), 1.90 (s, 3H, C*H*₃), 3.32 (m, 2H, C*H*₂), 3.55 (m, 2H, C*H*₂), 3.72 (s, 3H, OC*H*₃), 4.00 (m, 2H, C*H*₂), 4.32 (m, 1H, C*H*), 4.50 (s, 2H, OC*H*₂), 4.52 (m, 2H, C*H*₂), 5.46 (t, 1H, N*H*), 6.85 (s, 1H, C₆*H*), 7.30 (m, 5H, Ar*H*) and 8.8 (s, 1H, N*H*). Anal. Calcd for C₂₅H₃₄N₄O₈: C, 57.90; H, 6.61; N, 10.80. Found: C, 57.83; H, 6.68; N, 10.65.

### D. N-(N-Boc-2-aminoethyl)-N-[(1-thyminyl)acetyl]-L-serine(OBn) (39)

The substrate **39C** (0.52g, 1 mmol) was dissolved in THF (10ml) and cooled to 0°C in an ice bath. To this cold stirred solution NaOH (1N, 5ml, 5 mmol) was added all at once. The reaction mixture was stirred at 0°C for 1h and evaporated to dryness. The residue was dissolved in water (10ml) and the pH was adjusted to 3-4 with solid citric acid. The aqueous solution was extracted with EtOAc (3x30ml). The organic extract was washed with brine, dried and evaporated to dryness to give a foam. The residue was dried over solid NaOH and checked by HNMR. Yield 0.25g (49%). ¹HNMR (DMSO-d₆) d 1.38 (s, 9H, t-Butyl), 1.72 (s, 3H, C*H*₃), 3.00 to 4.00 (m, 4H, 2C*H*₂), 4.40 (m, 5H, C*H* C*H*₂, & OC*H*₂), 6.80 (t, 1H, N*H*), 7.22 (m, 6H, C₆*H* & Ar*H*) and 11.25 (m, 1H, COO*H*).

### EXAMPLE 40

### N-(N-Boc-2-Aminoethyl)-N-(2, 2, 2-trichloro-1, 1-dimethyl-1-ethyoxycarbonyl)-L-serine[(α-methoxy-α-trifluoromethyl-α phenyl)-O-acetate] methyl ester (40)

### A. N-(N-Boc-2-aminoethyl)-L-serine(OH) methyl ester (40a)

The substrate **39b** (0.7g, 2 mmol) was dissolved in methanol (20ml) and treated with Pd/C (10%, 0.2g). The reaction mixture was hydrogenated for 12h at 50psi H₂. The catalyst was filtered and washed with MeOH (20ml). The combined filtrate was evaporated to dryness. The residue was purified by flash column chromatography over silica gel using CH₂Cl₂/MeOH as the eluent. The pure fractions were collected and evaporated to give 0.9g (95%) of the product as a foam. ¹HNMR (CDCl₃) d 1.42 (s, 9H, t-Butyl), 2.70 (m, 1H, C*H*₂), 2.88 (m, 1H, C*H*₂), 3.20 (m, 2H, C*H*₂), 3.45 (m, 1H), 3.62 to 3.90 (m, 6H, C*H*₂, OC*H*₃), 5.15 (bs, 1H, N*H*).

### B. N-(2-Boc-Aminoethyl)-N-(2, 2, 2-trichloro-1, 1-dimethyl-1-ethyoxycarbonyl)-L-serine(OH) methyl ester (40b)

The substrate **40a** (2.62g, 10 mmol) was dissolved in ether (30ml) and mixed with 2N NaHCO₃ solution (20ml). To this stirred solution was added 1,1-dimethyl-2,2,2-trichloroethyl chloroformate (2.39g, 10 mmol, dissolved in 20ml of ether) slowly during 15min period at room temperature. After the addition of TecBocCl, the reaction was stirred for 12h and concentrated to dryness. The residue was suspended in water (50ml) and extracted in CH₂Cl₂ (2x75ml). The organic extract was washed water (50ml) and brine(50ml). The CH₂Cl₂ layer was dried over anhydrous Na₂SO₄ and evaporated to dryness. The residue was purified by flash column chromatography over silica gel using CH₂Cl₂/acetone as the eluent. The pure fractions were collected and evaporated to give 3.9g(85%) of an oily product. ¹HNMR (CDCl₃) d 1.42 (*s*, 9H, t-Butyl), 1.90 (*s*, 6*H*, 2C*H*₃), 3.35 (m, 2H, C*H*₂), 3.50 (m, 2H, C*H*₂), 3.75 (s, 3H, OC*H*₃), 3.90 to 4.30 (m, 3H, *OH* & C*H*₂), 4.45 (m, 1H, C*H*), 5.06 (m, 1H, N*H*).

### C. N-(N-Boc-2-Aminoethyl)-N-(2, 2, 2-trichloro-1, 1-dimethyl-1-ethyoxycarbonyl)-L-serine[(α-methoxy-α trifluoromethyl-α-phenyl)-O-acetate] methyl ester (40)

The substrate **40b** (0.3g, 0.65 mmol) was dissolved in dry CH₂Cl₂ (30ml) and cooled to 0°C under argon. To this cold stirred solution was added dry pyridine (1ml) followed by a-methoxy-a-trifluoromethyl-a-phenyl acetyl chloride (0.18g, 0.7 mmol). After addition of the acid chloride, the reaction was allowed to stir for 12h. The reaction mixture was diluted with CH₂Cl₂ (75ml) and washed with 5%NaHCO₃ (100ml), water (50ml) and brine(50ml). The CH₂Cl₂ layer was dried over anhydrous Na₂SO₄ and evaporated to dryness. The residue was loaded on a column loaded with silica gel using CH₂Cl₂. The excess Moser's acid was eluted first with CH₂Cl₂. Then the column was eluted with CH₂Cl₂/acetone (8:2) to give the product. The product was checked by ¹⁹F for chiral purity. The ¹⁹F showed two peaks at 20°C for the rotamer around the amide bond. But the two peaks became a single peak at 80°C. No raceimization was observed. ¹H NMR (CDCl₃) d 1.42 (s, 9H, t-Butyl), 1.88 (s, 6H, 2C*H*₃), 3.00 (m, 2H, C*H*₂), 3.50 (s, 3H, OC*H*₃), 3.75 (s, 3H, OC*H*₃), 4.28 (m, 1H, C*H*), 4.80 (m, 2H, C*H*₂), 5.10 (m, 1H, N*H*) and 7.45 (m, 5H, Ar*H*) . ¹⁹F NMR (DMSO-d₆) ppm -72.699 & -72.78(20°C), -71.15(80°C) with TFA (-76.53ppm) as internal standard.

### EXAMPLE 41

### N-(N-Boc-2-Aminoethyl)-N-(2, 2, 2-trichloro-1, 1-dimethyl-1-ethyoxycarbonyl)-L-serine[(a-methoxy-a-trifluoromethyl-a-phenyl)-O-acetat methyl ester (41)

### A. N-(N-Boc-2-aminoethyl)-D-serine(O-benzyl) methyl ester (41a)

The titled compound was prepared by following the method B Example **39,** described for the prepartion of L-isomer **39b.** The reactants used: Boc-aminoacetaldehyde (8.27g, 52 mmol), D-serine(0Bn) methyl ester (11.0g, 52.63 mmol), NaOAc (7.07g, 52 mmol), Pd/C (2g) and dry MeOH (100ml). The crude product was purified by flash chromatography using CH₂Cl₂/ethyl acetate as the eluent. Yield 12g (66%). ¹HNMR (CDCl₃) δ 1.44 (s, 9H, t-Butyl), 1.95 (bs, 1H, N*H*), 2.60 (m, 1H, C*H*₂), 2.80 (m, 1H, C*H*₂), 3.18 (m, 2H, C*H*₂), 3.44 (t, 1H), 3.68 (m, 2H, C*H*₂), 3.72 (s, 3H, OC*H*₃), 4.50 (s, 2H, OC*H*₂), 5.06 (bs, 1H, N*H*) and 7.26 (m, 5H, Ar*H*) . Anal. Calcd for C₁₈H₂₈N₂O₅ : C, 61.34; H, 8.01; N, 7.95. Found: C, 61.31; H, 8.12; N, 7.85.

### B. N-(2-Boc-Aminoethyl)-D-serine methyl ester (41b)

The titled compound was prepared by following the procedure described for the prepartion of L-isomer **40a**, Example Y. The reactants used were: The substrate **41a**, (0.71g, 2 mmol), Pd/C (10%, 0.2g) and dry MeOH (20ml). The crude product was purified by flash chromatography using CH₂Cl₂/MeOH as the eluent. Yield 0.9g (95%). ¹HNMR (CDCl₃) δ 1.42 (s, 9H, t-Butyl), 2.70 (m, 1H, C*H*₂), 2.88 (m, 1H. C*H*₂), 3.20 (m, 2H, C*H*₂), 3.45 (m, 1H), 3.62 to 3.90 (m, 6H, C*H*₂, OC*H*₃), 5.15 (bs, 1H, N*H*).

### C. N-(N-Boc-2-aminoethyl)-N-(2, 2, 2-trichloro-1, 1-dimethyl-1-ethyoxycarbonyl)-D-serine(OH) methyl ester (41c)

The titled compound was prepared by following the procedure described for the prepartion of L-isomer **40b**. The reactants used were: The substrate **41b**, (0.5g, 1.91 mmol), 1,1-dimethyl-2,2,2-trichloroethyl chloroforamate (0.72g, 3 mmol), 1N NaHCO₃ (5ml, 5 mmol) and ether (10ml). The crude product was purified by flash chromatography using CH₂Cl₂/acetone as the eluent. Yield 0.5g (56%). ¹HNMR (CDCl₃) δ 1.42 (s, 9H, t-Butyl), 1.90 (s, 6H, 2C*H*₃), 3.35 (m, 2H, C*H*₂), 3.50 (m, 2H, C*H*₂), 3.75 (s, 3H, OC*H*₃), 3.90 to 4.30 (m, 3H, O*H* & C*H*₂), 4.45 (m, 1H, C*H*), 5.06 (m, 1H, N*H*).

### D. N-(N-Boc-2-Aminoethyl)-N-(2, 2, 2-trichloro-1, 1-dimethyl-1-ethyoxycarbonyl)-L-serine[(α-methoxy-α-trifluor omethyl-α-phenyl)-O-acetate] methyl ester (41d)

The substrate **41c** 15 (0.3g, 0.65 mmol) was dissolved in dry CH₂Cl₂ (30ml) and cooled to 0°C under argon. To this cold stirred solution was added dry pyridine (1ml) followed by α-methoxy-α-trifluoromethyl-α-phenyl acetic acid chloride (0.18g, 0.7 mmol). After the addition of acid chloride, the reaction was allowed to stir for 12h. The reaction mixture was diluted with CH₂Cl₂ (75ml) and washed with 5%NaHCO₃ (100ml), water (50ml) and brine (50ml). The CH₂Cl₂ layer was dried over anhydrous Na₂SO₄ and evaporated to dryness. The residue was loaded on a column silica gel using CH₂Cl₂. The excess Moser's acid was eluted first with CH₂Cl₂. Then the column was eluted with CH₂Cl₂/acetone (8:2) to give the product. The product was checked by¹⁹F for chiral purity. The ¹⁹F showed two peaks at 20°C for rotamer around the amide bond. But the two peaks became single peak at 80°C. No raceimization was observed. The L-isomer (**40**) and D-isomer (**41d**) were mixed and checked by ¹⁹F NMR. The mixture showed a triplet (ppm, -70.772, -70.856 & -70.947) at 20°C, but at 80°C a triplet became a doublet (ppm, -70.679 & -70.758). This accounts for the DL mixture and rotamer at 20°C. On the other hand at 80°C they exsist only as the DL isomer. ¹H NMR (DMSO-d₆) δ 1.38 (s, 9H, t-Butyl), 1.80 (m, 6H, 2*CH*₃), 3.00 (m, 2H, C*H*₂), 3.32 (m, 2H, C*H*₂), 3.42 (s, 3H, OC*H*₃), 3.64 (s, 3H, OC*H*₃), 4.76 (m, 3H, C*H* & C*H*₂), 6.64 (m, 1H, N*H*) and 7.45 (s, 5H, Ar*H*). ¹⁹F NMR (DMSO-d₆) ppm -70.86 & -70.96(20°C), -70.75(80°C) with TFA (-76.53ppm) as internal standard.

### E. N- (N-Boc-2-aminoethyl)-N-[(1-thyminyl)acetyl]-D-serine(OBn) methyl ester (41e)

The titled compound was prepared by following the procedure described for the prepartion of L-isomer **39c**. The reactants used were: The substrate **41a** (10.3g, 29.3 mmol), thymin-1-ylacetic acid (6.07g, 33 mmol), DhbtOH (5.4g, 33 mmol), EDC (6.3g, 33 mmol), dry CH₂Cl₂ (150ml) and dry DMF (50ml). The crude product was purified by flash chromatography using CH₂Cl₂/acetone as the eluent. Yield 13.2g (87%). ¹HNMR (CDCl₃) δ 1.42 (s, 9H, t-Butyl), 1.90 (s, 3H, C*H*₃), 3.32 (m, 2H, C*H*₂), 3.55 (m, 2H, C*H*₂), 3.72 (s, 3H, OC*H*₃), 4.00 (m, 2H, C*H*₂), 4.32 (m, 1H, C*H*), 4.50 (s, 2H, OC*H*₂), 4.52 (m, 2H, C*H*₂), 5.46 (t, 1H, N*H*), 6.85 (s, 1H, C₆*H*), 7.30 (m, 5H, Ar*H*) and 8.8 (s, 1H, N*H*). Anal. Calcd for C₂₅H₃₄N₄O₈: C, 57.90; H, 6.61; N, 10.80. Found: C, 57.82; H, 6.67; N, 10.67.

### EXAMPLE 42

### N-(N-Boc-2-aminoethyl)-N-[(1-thyminyl)acetyl]-L(or D)-serine methyl ester (42)

Compound **39** (0.5g) is dissolved in ethanol and palladium on carbon (50mg) is added. The mixture is shaken on a Parr hydrogenation apparatus for 24h. The reaction mixture is filtered and concentrated. The residue is purified by flash column chromatography to give the title compound.

### EXAMPLE 43

### N-(N-Boc-2-aminoethyl)-N-[(1-thyminyl)acetyl]-L(or D)-serine methyl ester (43)

Compound **42** is converted to the title compound using the procedures of Example **37.**

### EXAMPLE 44

### N-(N-Boc-2-aminoethyl)-N-[(1-thyminyl)acetyl]-L-serine methyl ester (44)

Compound **43** is converted to the title compound using the procedures of Example **38.**

### EXAMPLE 45

### N-Methyl PNA Monomers, General Procedures for Synthesis and Oligomerization

### 1. Method A

### A. N-Methyl-1-amino-2,3-propandiol (45A)

Methylamine (172ml of a 40% solution in water, 2 mol) was cooled to 0 °C and 2,3-epoxy-1-propanol (25g, 0.34mol) was added at a rate to maintain the temperature at or below 10 °C. The mixture was stirred for 3 hours at 0 °C. Excess methylamine and water was evaporated in *vacuo* and the product was purified by distillation at 103-105 °C, at 0.5 mm Hg, to give 25.6g (76%) of the title compound.

### B. N-Boc-N-methyl-1-amino-2,3-propandiol (45B)

To a solution of N-methyl-1-amino-2,3-propandiol (21g, 0.2mol) in water (340ml) maintained at 0 °C was added Boc-anhydride (52.3g, 0.24mol). The mixture was allowed to equilibrate to room temperature. The pH was adjusted to and maintained at 10.5 with 4N NaOH. NaOH (2 eq.) is added and the reaction mixture was kept at room temperature under an atmosphere of nitrogen overnight. The reaction mixture is cooled to 0 °C and the pH is adjusted to 2.5 with 4N HCl. The acidic mixture is extracted with ethylacetate (6x100ml). The ethylacetate extracts were combined, washed with half-saturated KHSO₄ (3x150ml) and saturated brine (1x150ml), dried over MgSO₄ and evaporated to dryness. The title compound was isolated as an oil (bp 110-112 °C at 0.5 mm Hg). The yield of the title compound was 31.3g (81%).

### C. N-Boc-N-methyl-2-amino-acetaldehyde (45C)

To a solution of N-Boc-N-methyl-1-amino-2,3-propandiol (20g, .097 mol) in water (100ml) was added KIO₄ (24.68g, .108 mol). The reaction mixture was stirred for 2.5 hr under an atmosphere of nitrogen and then filtered. The filtrate was extracted with chloroform (5x50ml). The resulting chloroform extracts are dried over MgSO₄, filtered, and evaporated to dryness. The title compound 13.26g (79%) was isolated as a clear oil (bp 76-80 °C at 0.5 mm Hg).

### D. N-[(N-Boc-N-methyl)-2-aminoethyl]glycine ethyl ester (45D)

Glycine ethyl ester hydrochloride (0.58 mol) was dissolved in absolute ethanol (100ml) and added dropwise to a solution of N-Boc-N-methyl-2-amino-acetaldehyde (10g, .058 mol) and NaOAc (9.32g, .114 mol) dissolved in absolute ethanol (130ml) at 0 °C. This solution was hydrogenated using palladium on carbon (1.65g) and hydrogen (1eq) overnight. The reaction mixture was filtered and water (80ml) was added. The pH was adjusted to 8 with 2N NaOH and extracted with dichloromethane (5x60ml). The organic phase was dried over MgSO₄ and evaporated to dryness. The residue was purified by kugelrohr distillation (100 °C, 0.5 mm Hg) to give 10.6g (74.3%) of the title compound as a clear oil.

### E. General Procedure for synthesis and deprotection of the Monomers (A, C, G, T)

The monomers containing the four natural bases attached via an acetyl linker were synthesized using N-Boc-N-methyl-1-aminoethylglycine ethyl ester (X). To a solution of N-Boc-N-methyl-1-aminoethylglycine ethyl ester (X, 1.82g, 7mmol) in dry DMF (30ml) was added DHBT-OH (1.26g, 7.7 mmol) and the carboxymethyl derivative of the base. Dichloromethane (30ml) was added and the solution was cooled to 0 °C. DCC (1.73g, 0.84 mmol, 1.2 eq) was added and the mixture was stirred for 1 hr. The reaction mixture was allowed to warm to room temperature and stirred for 3 hr and then filtered.

The protected A monomer, N-[(N-Boc-N-methyl)-2-aminoethyl]-N-[(9-adeninyl)acetyl]glycine ethyl ester, was synthesized using the above procedures. The reaction mixture was evaporated, redissolved in dichloromethane (300ml), washed with half-saturated aqueous NaHCO₃ (3x100 half-saturated aqueous KHSO₄, (2x100ml) and saturated brine (1x100ml). The organic phase was dried over MgSO₄ and evaporated to dryness. Absolute ethanol (60ml) and water (30ml) was added to the residue and the mixture was stirred overnight. The resulting precipitate was filtered and dried to afford the protected A monomer.

The protected A monomer is dissolved in THF and cooled to 0 °C. LiOH (25ml) was added and the reaction mixture was stirred for 30 min. The pH was adjusted to 1 with HCl (2N). The resulting precipitate was filtered and dried to afford N-[(N-Boc-N-methyl)-2-aminoethyl]-N-[(9-adeninyl)acetyl]-glycine.

The protected C monomer, N-[(N-Boc-N-methyl)-2-aminoethyl]-N-[(1-cytosinyl)acetyl]glycine ethyl ester, was synthesized using the above procedures. The reaction mixture was evaporated to dryness. Ether (50ml) was added to the residue and the mixture was stirred for 2 hr and filtered. This was repeated two times and then followed by addition of half-saturated aqueous NaHCO₃, filtered and dried. The resulting residue was dissolved in boiling dioxane (60ml) and precipitated with water (60ml), filtered and dried. The precipitate was purified by silica gel column chromatography using methanol/dichloromethane 5/95 to afford the protected C monomer.

The protected C monomer is dissolved in THF and cooled to 0 °C. LiOH (60ml) was added and the reaction mixture was stirred for 30 min. The pH was adjusted to 2 with HCl (2N). The mixture was extracted with dichloromethane and the organic phase dried over MgSO₄ and evaporated to afford .72g (27%) N-[(N-Boc-N-methyl)-2-aminoethyl]-N-[(1-cytosinyl)acetyl]glycine (mp 181-184 °C).

The protected G monomer, N-[(N-Boc-N-methyl)-2-aminoethyl]-N-[(9-guaninyl)acetyl]glycine ethyl ester, was synthesized using the above procedures. The reaction mixture was evaporated, redissolved in dichloromethane (80ml), washed with half-saturated aqueous KHSO₄, (3x40ml), dried over MgSO₄, and evaporated to dryness. The precipitate was recrystallized in ethylacetate and dried to give the protected G monomer.

The protected G monomer is dissolved in methanol (40ml) NaOH (40ml, 2N) was added. The mixture was stirred at room temperature for 1 hr and then cooled to 0 °C. The pH was adjusted to 2 with HCl (2N). The resulting precipitate was filtered, dried and recrystalized from absolute ethanol to afford 1.27g (43%) of N-[(N-Boc-N-methyl)-2-aminoethyl]-N-[(9-guaninyl)acetyl)glycine (mp 189-192 °C).

The protected T monomer, N-[(N-Boc-N-methyl)-2-aminoethyl]-N-[(1-thyminyl)acetyl]glycine ethyl ester, was synthesized using the above procedures. Dichloromethane (60ml) was added to the reaction mixture and the resulting mixture was washed with half-saturated NaHCO₃ (3x30ml), half-saturated aqueous KHSO₄ (2x30ml), and saturated brine (1x30ml). The organic phase was dried over MgSO₄ and evaporated. The residue was redissolved in dichloromethane, cooled to 0 °C and precipitated by slow addition of petroleum ether with vigorous stirring. Filtration of the mixture gave the protected T monomer as a white solid.

The protected T monomer is dissolved in methanol (45ml) and cooled to 0 °C. NaOH (45ml, 2N) was added and the mixture was stirred at room temperature for 2 hrs. The pH was adjusted to 2 with HCl (2N) and the organic phase was extracted with ethylacetate. The organic phase was dried over MgSO₄ and evaporated to dryness to afford 1.45g (52%) of N-[(N-Boc-N-methyl)-2-aminoethyl]-N-[(1-thyminyl)acetyl]-glycine (mp 114-118 °C).

### F. General Procedures for Oligomerization

The prepared monomers were used for oligomerization by solid phase peptide synthesis on MBHA-resin.

Using the above procedures the following oligomers were prepared: H-GT*AGAT*CACT*Lys-CONH2          (SEQ ID NO:33) H-Gly-G*T*A*G*A*T*C*A*C*T*-Lys-CONH2          (SEQ ID NO:34) H-TTTTTT*TTTT-Lys-CONH2          (SEQ ID NO:35) H-TTTT*TT*TTT-Lys-CONH2 (SEQ ID NO:36) H-TTTT*TT*TT*TT-Lys-CONH2 (SEQ ID NO:37) H-Gly-T*T*T*T*T*T*T*T*T*T*-Lys-CONH2          (SEQ ID NO:38) wherein a * denotes a monomer functionalized to have an N-methyl group on the 2-aminoethyl portion of the monomer.

### 2. Method B

### A. N-Boc sarcosylglycine methyl ester

To a suspension of Boc-sarcosine (9.0 g, 048 mol) in 70 ml of CH₂Cl₂ at -20°C was added isobutyl chlorofomate (7.1g, 0.052mol) and N-Methylmorpholine (4.8 g, 0.047mol). After 2 min glycine methyl ester, hydrochloride (7.19g, 0.057mol) and N-methylmorpholine (5.76g, 0.057 mol) in CH₂Cl₂ (60 ml) was added. The reaction was stirred at -10°C for 1 h and allowed to warm to room temperature. After 4h at room temperature, the reaction was filtered and washed with salt. NaHCO₃ (100ml), 1N NaHCO₃ 100 ml, twice with 1N KHSO₄ (100 ml), water (100 ml) and brine (70 ml). The organic phase was dried with Na₂SO₄ and evaporated. The yield of Boc sarcosyl-glycine methyl ester was 89%.

### B. N-[(N-Boc)(N-methyl)(1-thiocarbonyl)-2-aminoethyl]-glycine methyl ester

A suspension of the N-Boc-sarcosylglycine methyl ester (10.0 g, 0.041 mol) and Lawessons reagent (9.87 g, 0.024mol) in toluene (90 ml), was heated to 80°C for 1h and then evaporated to dryness. The product was purified by column chromatography (CH₂Cl₂/MeOH 95:5) on silica gel to give 63% of the title compound.

### C. N-[(N-Boc)(N-methyl)-2-aminoethyl]-glycine methyl ester

To a suspension of the N-[(N-Boc)(N-methyl)(1-thiocarbonyl)-2-aminoethyl]-glycine methyl ester (2.67 g, 0.0097 mol) and NiCl₂6H₂O (18.39 g, 0.077 mol) in THF:MeOH 1:1 (100ml) at -20°C, NaBH₄ (8.78g, 0.23 mol) was slowly added so that the temperature did not exceed 0°C. After addition of the NaBH₄, the reaction was allowed to go to room temperature. The reaction mixture was filtered several times through celite and evaporated to dryness. The product was isolated by column chromatrography on silica gel with CH₂CL₂/MeOH 90:10 eluent. The yield of the title compound was 60%.

### D. N-[(N-Boc)(N-methyl)-2-aminoethyl]-N-[(1-thyminyl)acetyl] glycine methyl ester

To a suspension of N-[(N-Boc)(N-methyl)-2-aminoethyl]-glycine methyl ester (0.9 g, 0.0037 mol) and DHBT-OH (0.65 g, 0.004 mol) in CH₂Cl₂ 10 ml was added N-1-carboxymethylthymine (0.74 g, 0.04 mol) dissolved in DMF (6ml). The solution was cooled in an ice bath and DCC (0.905, 0.049 mol) was added. The reaction mixture was stirred for 1 hour and the ice bath was removed. The reaction mixture was left for 16 hours at room temperature. The reaction mixture was filtered and washed twice with 1N KHSO₄ (15 ml) and three times with NaHCO₃ (15 ml), brine (10 ml) and water (10 ml) and dried with Na₂SO₄. The product was purified by column chromatography on silica gel with CH₂Cl₂/MeOH 95:5 eluent. The yield of the title compound was 55%.

### E. N-[(N-Boc)(N-methyl)-2-aminoethyl]-N-[(1-thyminyl)acetyl]glycine

N-[(N-Boc)(N-methyl)-2-aminoethyl]-N-[(1-thyminyl)acetyl] glycine methyl ester was dissolved in 1N NaOH in MeOH/H₂O 1:1 for 2 hours. The pH was adjusted to 2 with 2N HCl and extracted with ethyl acetate (50 ml). The organic phase was dried with Na₂SO₄ and evaporated to dryness. The yield of the title compound was 77%.

### F. Oligomerization of N-methyl monomers

MBHA resin was adjusted to a loading of 0.1 mmol/mg by applying N-Boc N'-2-chloro-Z-lysine and DIC in CH₂Cl₂/DMF 1:1 24 hours. Coupling of the monomers including the N-methyl derivative follows the normal protocol for coupling of PNA monomers to the solid support (see Example 26). Coupling of the monomer following the N-methylated monomer follows this protocol: 3 eq. of the monomer and 3 eq. of PyBorP was dissolved in CH₂Cl₂/DMF and added to the solid phase. Diisoproylethylamine (9 eq.) was added. The reaction mixture was left for 24 hours. The reaction mixture was washed twice with DMF for a few seconds and once with DMF for two minutes. Then it was washed four times with CH₂Cl₂. This was repeated four times and the standard protocol for PNA oligomerization was then followed again.

### EXAMPLE 46

### Alternative Synthetic Route for Production of Functionalized PNA Monomers

### A. (S)-3-Amino-2-oxetanone p-toluenesulfonate (46A)

Boc serine is treated with Ph₃P, DEAD, and TFA, followed by TsOH to give the title compound.

### B. [(2-N-Tosyl)-3-N-Boc]propionic acid (46B)

Compound **46A** is treated with Boc₂NH to give the title compound.

### C. Ethyl (2-N-phthaloyl)-(3-N-Boc)-2,3-diaminopropionate (46C)

Compound **46B** is treated with ethoxycarbonylphthalimide followed by ethanol, DCC and HOBT to give the title compound.

### D. Ethyl-(2-N-phthaloyl)-2,3-diaminopropionate (46D), ethyl-(3-N-Boc)-2,3-diaminopropionate (46E)

Compound **46C** is either deprotected at the C-3 Boc group to give the amino group by tretment with TFA/dichloromethane to give Compound **46D,** or deprotected at the C-2 amino group by treatment with hydrazine to give **46E**.

The compounds ethyl-(2-N-phthaloyl)-2,3-diaminopropionate and ethyl-(3-N-Boc)-2,3-diaminopropionate are used directly in the syntheses of functionalized PNA backbones as described in Examples **30**-**38**.

### EXAMPLE 47

### Incorporation of Biotin into an Aminoethyl Glycine-PNA Oligomer H-TTC(Biotin)-TT-Lys-NH₂ (SEQ ID NO:39)

The protected aminoethyl glycine-PNA oligomer is assembled on a Boc-Lys(ClZ) modified MBHA resin with a substitution of approximately 0.10 mmol/g. The synthesis is initiated on 100 mg (dry weight) of t-Boc-Lys(ClZ)-MBHA resin, preswollen in dichloromethane. The aminoethyl glycine-PNA-oligomer is synthesized as per the procedures of Example **26.** In step (3) an aeg-cytosine monomer from Examples **10** thru **17** which has been further derivatized following the procedures of Sproat B.S., *et al., Nucleic Acids Research,* **1989,** *17*, 3373-3386, to include an N-4 (5-trifluoroacetylaminopentyl) protected linking moiety. The procedure of Sproat is modified for use with a PNA monomer by simply using an aminoethyl glycine cytosine monomer havine the N-4 exocyclic amino group deprotected and the amino and the carboxyl group of the aminoethyl glycine protected. The aminoethyl glycine-PNA was cleaved from the resin as per the procedures of Example **28**. Yield: 5.8 mg (purity 90%), purified by RP-HPLC, (µBondapak C18). MS(FAB+) *m*/*z*:(found/calcd) 2702/2701

### EXAMPLE 48

### N-Terminal Conjugation of Biotin to a PNA

The N-terminal Boc-blocked PNA containing resin is measured out to typically provide 0.1-1.0 millimoles of PNA. This weight of resin is suspended in a 1:1 (v:v) dichloromethane:dimethylformamide solution (5mL/100mg of resin) and allowed to swell for a period of time if desired. The solvent is then removed by filtration and the resin resuspended in trifluoroacetic acid (3mL/100mg of resin) and shaken for 2 minutes. The trifluoroacetic acid is removed by filtration and this is repeated one time. The resulting resin is resuspended in pyridine (1mL/10µmoles of resin) and vacuum filtered to remove the pyridine. This is repeated. This washing step is repeated twice. The resin is suspended in 1:1 (v:v) pyridine:dimethylformamide and to this suspension is added the biotin (2-10 molar equivalents), TBTU (1.9-9.9 molar equivalents), and di-isopropylethlamine (5-20 molar equivalents) such that the final concentration of PNA monomer is 0.2M. The suspension is shaken for 15-60 minutes and the spent coupling solution is removed by filtration. The resin is then washed three times with 1:1 (v:v) dichloromethane:dimethylformamide solution (5mL/100mg of resin) giving the N-terminal biotin-attached PNA. This can be cleaved from the resin as described in Example **28**. Starting with BocGly-GCAT-CO-Merrifield resin one obtains Biotin-Gly-GCAT-COOH and starting with BocGly-GCAT-Lys-CO-Merrifield resin one gets Biotin-Gly-GOAT-COOH.

### EXAMPLE 49

### N-Terminal Conjugation of Pteroic Acid to a PNA

An N-Boc-O-Benzyl-Blu-blocked PNA containing resin is measured out to typically provide 0.1-1.0 millimoles of PNA. This weight of resin is suspended in a 1:1 (v:v) dichloromethane:dimethylformamide solution (5mL/100mg of resin) and allowed to swell for a period of time if desired. The solvent is then removed by filtration and the resin resuspended in trifluoroacetic acid (1mL/100mg of resin) and shaken for 2 minutes. The trifluoroacetic acid is removed by filtration and this is repeated one time. The resulting resin is resuspended in pyridine solution (1mL/1gm of resin) and vacuum filtered to remove the pyridine. This is repeated. This is followed by resuspension followed by filtration (designated "washing") using 1:1 (v:v) dichloromethane:dimethylformamide solution (5mL/1gm of resin). This washing step is repeated twice. The resin is suspended in 1:1 (v:v) pyridine:dimethylformamide and to this suspension is added the N-Boc pteroic acid (prepared from pteroic acid, Aldrich Chem Co., 2-10 molar equivalents), TBTU (1.9-9.9 molar equivalents), and diisopropylethylamine (5-20 molar equivalents) such that the final concentration of PNA monomer is 0.2m. The suspension is shaken for 15-60 minutes and the spent coupling solution is removed by filtration. The resin is then washed three times with 1:1 (v:v) dichloromethane:dimethylformamide solution (5mL/100mg of resin) giving the N-terminal folic acid-attached PNA. This can be cleaved from the resin as described in Example **28.** Starting with N-Box-O-Benzyl-Glu-GCAT-CO-Merrifield resin one obtains Boc-folate-GCAT-COOH and starting with N-Boc-O-Benzyl-Glu-GCAT-Lys-COMerrifield resin one gets N-Boc-folate-GOAT-COOH.

### EXAMPLE 50

### Coupling to a Free COOH of a PNA or PNA Derivative

A PNA or PNA derivative having a single free COOH such as CH₃CONH-GCAT-COOH is dissolved in tetrahydrofuran:water to provide a solution that is 0.1 M in PNA and to this is added 1-(3-dimethylaminopropyl)-3-ethylcarbodimide hydrochloride [EDCI] and Rhodamine 123 hydrate to give a solution which is 0.1-1.0 molar in both EDCI and Rhodamine. The solution is stirred for 0.1-2 hours, then evaporated to a solid and purified by preparative HPLC as described in Example **29**.

By this method one can prepare the following compounds. Rhodamine = Rhod

CH₃CONH-GCAT-CONHRhod (SEQ ID NO:40)

CH3CONHY-GCAT-Asp(Rhod)-CONH2 (SEQ ID NO:41)

Chol-GlyGCAT-Asp(Rhod-CONH2  (SEQ ID NO:42)

Chol-GCAT-Asp(Rhod)-CONH2 (SEQ ID NO:43)

RhodNHOC(CH2)4CONH-GCAT-Lys-CONH2 (SEQ ID NO:44)

RhodNHOC(CH2)4CONH-GCAT-CONH2 (SEQ ID NO:45)

H₂N-GCAT-CONHRhod (SEQ ID NO:46)

H2N-Gly-GCAT-CONRhod (SEQ ID NO:47)

H2N-GCAT-Lys-CONHRhod (SEQ ID NO:48)

H2N-Gly-GCAT-Lys-CONHRhod (SEQ ID NO:49)

Fluor-GCAT-CONHRhod (SEQ ID NO:50)

Fluor-GlyGCAT-CONHRhod  (SEQ ID NO:51)

Fluor-GCAT-Gly-CONHRhod (SEQ ID NO:52)

CH3CONH-GCAT-Lys(Fluor)-CONRhod  (SEQ ID NO:53)

Chol-GlyGCAT-Lys(Fluor)-CONHRhod  (SEQ ID NO:54)

Chol-GCAT-Lys(Fluor)-CONRhod  (SEQ ID NO:55)

RhodNHOC(CH2)4ACONH-GCAT-Lys(Fluor)-CONH2  (SEQ ID
NO:56)

Fluor-GlyGCAT-Lys(Fluor)-CONHRhod  (SEQ ID NO:57)

Fluor-GCAT-Lys(Fluor)-CONHRhod  (SEQ ID NO:58)

### EXAMPLE 51

### 6-S-Tritylthiohexylbromide (51), 6-S-tritylthiohexanoic acid (51A), 6-S-tritylthiohexylamine (51B), 6-S-tritylthiohexylmercaptan (51C)

To a solution of triphenylmethanethiol (Fluka; 69g, 250 mmol) in 500 mL 95% ethanol (EtOH) was added 11 grams of sodium hydroxide dissolved in 75 mL of water (275 mmol). After stirring for about 15 minutes under an atmosphere of argon, using an addition funnel, 1,6-dibromohexane (91.5 g, 375 mmol, 58 mL) dissolved in 100 mL of 95% EtOH was added dropwise over a period of 1 hour with vigorous stirring. After about 15 minutes of stirring, a brown white solid separates out from the reaction mixture. After stirring for an additional 4 hours, the reaction mixture was filtered. The filtrate was evaporated under high vacuum and the oily residue was combined with the filtered residue and dissolved in 500mL CH₂Cl₂, and filtered again. The filtrate was washed once with water (200 mL) and once with saturated NaCl solution. After drying the CH₂Cl₂ layer over MgSO₄, it was concentrated to 200 mL in volume. About 200 mL of hexane was added and the solution was left in the freezer. Three crops of cream white product was isolated to give 81g of 6-S-Tritylthiohexylbromide (51), mp 91-92 °C.

Portions of the product are independently treated with sodium cyanide followed by hydrolysis to give the corresponding acid, 6-S-tritylthiohexanoic acid (Compound **51A**), with lithium azide followed by triphenylphosphine reduction to give the corresponding amine, 6-S-tritylthiohexylamine (Compound **51B**), and with sodium hydrogen sulfide to give the corresponding thiol, 6-S-tritylthiohexylmercaptan (Compound **51C**).

### EXAMPLE 52

### N-[(N-Boc)(1-(6-S-tritylhexyloxymethyl))2-aminoethyl]-N-[(1-thyminly)acetyl]glycine ethyl ester (52A) and N-[(N-Boc)(2-(6-S-tritylhexyloxymethyl))2-aminoethyl]-N-[(1-thyminly)acetyl]glycine ethyl ester (52B)

The free carboxyl group of compound **35** or **36** is protected as the ethyl ester according to standard procedures *(see, e.g.,* Greene and Wuts, Protective Groups in Organic Synthesis, 2d edition, John Wiley & Sons, New York, 1991). The resulting ethyl aminoethylglycinate is alkylated with S-trityl-6-mercaptohexylbromide in the presence of DMF and sodium hydride to yield the title compounds.

### EXAMPLE 53

### N-[((N-Boc-) (1-{(6-thio)hexyloxymethyl})2-aminoethyl]-N-[(1-thyminly)acetyl]glycine ethyl ester (Compound 53A) and N-[((N-Boc-)(2-{(6-thio)hexyloxymethyl})2-aminoethyl]-N-[(1-thyminly)acetyl]glycine ethyl ester (Compound 53B)

Compound **52A** or **52B** (0.19 mmol) is dissolved in **4** mL chloroform. Silver Nitrate (8 mM) in EtOH (12 mL) is added and the reaction mixture is stirred for 45 minutes. Dithiothreitol (0.35 M) in chloroform (3 mL) is added and the reaction is stirred overnight. The white precipitate is filtered off and the solvent removed *in vacuo.* The residue is dissolved in dichloromethane, extracted once with saturated NaHCO₃ and saturated NaCl and dried over MgSO₄. The solvent is removed *in vacuo.* The product is purified on a silica gel column or by HPLC. The product is analyzed by ¹H, ¹³C, and ¹³C-APT NMR and mass spectroscopy.

### EXAMPLE 54

### Attachment of Thiol Linker at Position 2 and 8 of Purines

a. 2-Fluorohypoxanthene is treated with ethyl bromoacetate as per the procedures of Example **18** to give 9-carboxymethyl-2-fluorohypoxanthene ethyl ester which is further reacted with N-(N-Boc-2-aminoethyl) glycine ethyl ester as per the procedures of Examples **20** and **21** to give N-(N-Boc-2-aminoethyl)-N-[(2-fluorohypoxanthene-9-yl)-acetyl]glycine. N-(N-Boc-2-aminoethyl)-N-[(2-fluorohypoxanthene)acetyl]glycine is reacted with 6-S-tritylthiohexylamine following the procedures of Harris, *et al., J. Am. Chem. Soc.* **1991,** *113,* 4328. The resulting purine C-2 thiol linker, PNA monomer is incorporated into PNA oligomers.
b. 8-Bromoadenine is treated with ethyl bromoacetate as per the procedures of Example **18** to give 9-carboxymethyl-8-bromoadenine ethyl ester. 9-Carboxymethyl-8-bromoadenine ethyl ester is treated as per the procedures of Example **19** to put a Cbz protection group on the N-6 exocyclic amino group. The resulting N-6-Cbz-9-carboxymethyl adenine ethyl ester is further reacted with N-(N-Boc-2-aminoethyl) glycine ethyl ester as per the procedures of Examples **20** and **21** to give N-(N-Boc-2-aminoethyl)-N-[({N-6-Cbz-8-bromo}-9-adenyl)-acetyl]glycine ethyl ester. N-(N-Boc-2-aminoethyl)-N-[({N-6-Cbz-8-bromo}-9-adenyl)acetyl]glycine ethyl ester is further reacted with 6-S-tritylthiohexylamine following the procedures of Harris, *et al., J. Am. Chem. Soc.* **1991,** *113,* 4328. The resulting purine C-8 thiol linker, PNA monomer is incorporated into PNA oligomers.

### EXAMPLE 55

### Attachment Of Amino Linker At 6-Position of Purines

### b. N-(N-Boc-2-aminoethyl)-N-[((N-2-trifluoroacetyl)N-6-trifluoroacetylaminohexylamino-9-guaninyl)acetyl]glycine (55A)

Guanine is dissolved in pyridine and treated with trifluoroacetic anhydride. The resultant 2(trifluoroacetamido)--6-O-pyrilyl-guanine intermediate is further reacted with pentafluorophenol to give 6-pentafluorophenyloxy-2-trifluoroacetamido guanine. A similar reaction scheme has been previously described for 2'-deoxy guanosine by Gao, *et.al., J. Org. Chem*., **1992**, *57*, 6954.

The 2-(protected)functionalized 6-protected nucleobase is further reacted with 2-bromoethylacetate as per the procedures of Example **18** to give a protected acetyl tether at the 9 position. The compound is treated with N-6-trifluoroacetyl-hexane-1,6-diamine resulting in the replacement of the O-C₆F₅ with a bifunctional linking group (NH(CH₂)₆NHCOCF₃). This monoprotected diamine linker has been described by Agrawal, *et.al., Tetrahedron Lett.*, **1990,** *31*, 1543. After the protected linking group is attached the resulting compound is treated via the procedures of Example 22 to give the title compound. The final compound having a protected linking moiety is further incorporated into oligomers using standard methods. The protected linking moiety is deprotected and functionalized with a further functional group e.g. fluorescein or biotin.

### b. N-(N-Boc-2-aminoethyl)-N-[(N-6-trifluoroacetylaminohexylamino-9-adeninyl)acetyl]glycine (55B)

Hypoxanthine (6-hydroxypurine) is dissolved in pyridine and treated with pentafluorophenol to give 6-pentafluorophenyloxy-purine. The modified nucleobase is further treated with 2-bromoethylacetate as per the procedures of Example **18** to give the protected acetyl tether at the 9 position. The compound is treated with N-6-trifluoroacetyl-hexane-1,6-diamine resulting in the replacement of the O-C₆F₅ with a bifunctional linking group (NH(CH₂)₆NHCOCF₃). After the protected linking group is attached the resultant functionalized adenine nucleobase is treated via the procedures of Example 22 to give the title compound. The final compound having a protected linking moiety is further incorporated into oligomers using standard methods. The protected linking moiety is deprotected and functionalized with a further functional group e.g. fluorescein or biotin.

### EXAMPLE 56

### Coupling of Cholesterol to Thiol Containing PNA Monomer via Disulfide Bridge

Compound **53A** or **53B** is treated with 2,2'-dithiobis(5-nitropyridine) in methylene chloride overnight. The precipitated 5-nitro pyridine-2-thione is removed by filtration, and the filtrate is concentrated. The resultant product (**56C** or **56D,** respectively) is treated with thiocholesterol in CH₂Cl₂ and shaken overnight to give the compound (**56A** or **56B**, respectively) with cholesterol attached through a disulfide link. The resulting compounds are further used in PNA synthesis.

### EXAMPLE 57

### Conversion Of The Thiol Linker With A Trityl Protecting Group Into A Thiol Linker Protected by Disulfide Linkage

Compound **56C** or **56D** is treated with propylmercaptan in CH₂Cl₂ and stirred overnight. The resulting disulfide compound (**57A** and **57B**, respectively), which has a CH₂-O-(CH₂)₆-S-S-CH₂-CH₂-CH₃ linkage, is further derivatized and incorporated into PNA oliomers. The free thiol group is liberated, before conjugation, by the addition of dithiothreitol (DTT).

### EXAMPLE 58

### Conversion To A Base Labile Thiol linker

Compound **56C** or **56D** is treated with CBzCl (carbobenzyloxy chloride) in triethylamine. The resulting compound **58A** or **58B**, which contains a protected thiol group is used in the synthesis of PNA oligomers. The free thiol group may be regenerated from the monomer before or after the monomer is used for incorporation into a PNA oligomer by treatment with ammonia followed by DTT.

### EXAMPLE 59

### Conjugation Reactions Of PNA-O-hexylthiol Linker

To illustrate the conjugation potential of the PNA-*O*-thiol tether, PNA H₂N-GCA*T-COOH (SEQ ID NO:113) is synthesized as per the procedures of Example **26**. The astrisk (*) denotes the incorporation of a disulfide protected thiolhexyloxymethyl linker attached at the C-1 position of the 2-aminoethyl portion of the monomer in the oligomer, synthesized as per the procedures of Example **58**. The PNA is treated with 0.1M AgNO₃ in TEAA buffer followed by DTT treatment to generate a free thiol group. The thiol-PNA is then reacted with four classes of compounds each having a haloacetamide or a maleimide group and the desired functionality at the other end. The following compounds are employed: (1) a phospholipid maleimide, which can offer cell signalling and trafficking properties to PNAs; (2) 5-iodoacetamido-*O*-phenanthroline, which is a nucleic acid cleaving agent; this particular conjugation offers an added advantage of optimal placement for the cleaving agent as this reagent when complexed to cuprous ion reacts via a minor groove attack at the C-1' position; (3) pyrenemaleimide, which may stabilize a PNA-PNA, PNA-DNA or PNA-RNA duplex via intercalation; and (4) fluorescein maleimide, which is used as a general diagnostic tool, allowing the monitoring of PNA uptake. The conjugations are carried out in phosphate buffer (pH 8.0). Completion of reaction is monitored by HPLC analysis using a C-18 column and a linear 1% increase of CH₃CN concentration for every minute. The conjugates are purified by size exclusion and reverse phase HPLC and characterized by their UV-VIS spectra (where applicable). Fluorescein maleimide, pyrene maleimide and phospholipid maleimide are purchased from Molecular Probes (Eugene, Oregon). *O*-Phenanthroline-5-iodoacetamide is synthesized according to the published procedure of Sigman, *Biochemistry* **1990,** *29*, 9097.

### EXAMPLE 60

### N-[(N-Boc)-1-(5-phthaloylpentyloxymethyl)-2-aminoethyl]-N-[(1-thyminyl)acetyl]glycine Ethyl ester (57) and N-[(N-Boc)-2-(5-phthaloylpentyloxymethyl)-2-aminoethyl]-N-[(1-thyminyl)acetyl]glycine Ethyl ester (57A)

Compounds **35** and **36** are independently treated with ethanol/DCC to produce the ethyl ester which is then treated with 5-bromopentylphthalimide to yield the title compounds.

### EXAMPLE 61

### N-[(N-Boc)-1-(5-phthaloylpentyloxymethyl)-2-aminoethyl]-N-[(9-adeninyl)-acetyl]glycine Ethyl ester (61) and N-[(N-Boc)-2-(5-phthaloylpentyloxymethyl)-2-aminoethyl]-N-[(9-adeninyl)-acetyl]glycine Ethyl ester (61A)

Compounds **61** and **61A**, are prepared using the general procedures of Examples **1** thru **6**, **35** and **36**, and further treated according to the procedures of Example **60**, to yield the intermediate ethyl esters and the title compounds.

### EXAMPLE 62

### N-[(N-Boc)-1-(5-aminopentyloxymethyl)-2-aminoethyl]-N-[(9-adeninyl)-acetyl]glycine Ethyl ester (62) and N-[(N-Boc)2-(5-aminopentyloxymethyl)-2-aminoethyl]-N-[(9-adeninyl)-acetyl]glycine Ethyl ester (62A)

Compound **61** or **61A** is dissolved in 200 ml methanol in a 500 ml flask. Hydrazine is added to the stirring reaction mixture. The mixture is heated to 60-65° in an oil bath and refluxed 14 hours. Solvent is evaporated *in vacuo*. The residue is dissolved in dichloromethane (250 ml) and extracted twice with an equal volume NH₄OH. The organic layer is evaporated to yield the crude product. NMR is used to assay product purity. The products are used in subsequent reactions without further purification.

### EXAMPLE 63

### N-[(N-Boc)-1-(5-phthaloylpentyloxymethyl)-2-aminoethyl]-N-[(1-thyminyl)-acetyl]glycine methyl ester (63)

Substrate **42** is converted to the title compound as per the procedures of Example **60.**

### EXAMPLE 64

### N-[(N-Boc)-1-(5-aminopentyloxymethyl)-2-aminoethyl]-N-[(1-thyminyl)-acetyl]glycine methyl ester (64)

Substrate **63** is converted to the title compound as per the procedures of Example **62.**

### EXAMPLE 65

### Synthesis of PNA Oligomer Having Amine Containing Functionalities

The following PNA oligomers having aminoethylglycine backbones are synthesized according to the procedures of Examples **26** et seq.: H₂N-GCA* TGC AT-COOH (SEQ ID NO:60), H₂N-GCA T**GC AT-COOH (SEQ ID NO:61), H2N-GCA T***GC A***T-COOH (SEQ ID NO:62) H2N-CTG TCT CCA* TCC TCT TCA CT-COOH (SEQ ID NO:63) H₂N-CTG TCT CCA*** TCC TCT TCA*** CT-COOH (SEQ ID NO:64) H₂N-CCA A**GC CUC AGA-COOH (SEQ ID NO:65) H₂N-CCA GGC UCA GA*T-COOH (SEQ ID NO:66) wherein a*, **, or *** represents a PNA monomer functionalized to incorporate a pentyl-N-phthalimido functionality at a position having a hydroxy methyl group. A * indicates a pentyl-N-phthalimido functionality attached to the hydroxy methyl group at the C-1 carbon of the 2-aminoethyl portion of the monomer as synthesized in Examples **60** and **61**. A ** indicates a pentyl-N-phthalimido functionality attached to the hydroxy methyl group at the C-2 carbon of the 2-aminoethyl portion of the monomer as synthesized in Examples **60** and **61**. A *** indicates a pentyl-N-phthalimido functionality at serine-O of the serine portion of the monomer as synthesized in Example **63.** PNA Oligomers SEQ ID NO:63 and 64 are compounds complimentary to the E2 region of the bovine papilloma virus-1 (BPV-1). PNA oligomers SEQ ID NO:65 and 66 are compounds complementary to the HIV-1 TAR region.

### EXAMPLE 66

### Conjugation Of PNA Oligomers Through a Hydroxyl Group

### A. Functionalization with Biotin

### 1. Single Site Modification

About 10 O.D. units (A₂₆₀) of PNA oligomer SEQ ID NO:63 is treated as per the procedures of Example **62** to remove the phthaloyl protecting group. The resulting oligomer (see, Example **65**) is dried in a microfuge tube. The PNA oligomer is dissolved in 200 µl of 0.2 M NaHCO₃ buffer and D-biotin-N-hydroxysuccinimide ester (2.5 mg, 7.3 µmols) (Sigma, St. Louis, MO) is added followed by 40 µl DMF. The solution is allowed to stand overnight. The solution is then applied to a Sephadex G-25 column (0.7 x 15 cm) and the PNA oligomer fractions are combined. Analytical HPLC is used to determine the percentage conversion to the product. The product is purified by HPLC (Waters 600E with 991 detector, Hamilton PRP-1 column 0.7 x 15 cm; solvent A: 50 mM TEAA pH 7.0; B: 45 mM TEAA with 80% acetonitrile: 1.5 ml flow rate: Gradient: 5% B for first 5 minutes, linear (1%) increase in B every minute thereafter) and further desalted on Sephadex G-25 to give the PNA oligomer: CTG TCT CCA* TCC TCT TCA CT (SEQ ID NO:67) wherein A* represents a PNA monomer functionalized to incorporate a biotin functionality linked via a pentylamino linking group attached to the C-1 hydroxymethyl group of the PNA monomer.

### 2. Multiple Site Modification

About 10 O.D. units (A₂₆₀) of PNA oligomer SEQ ID NO:64 is treated as per the procedures of Example **62** to remove the phthaloyl protecting group. The resulting oligomer (see, Example **65**) is treated utilizing the method of Example **61(A) (1)** above with D-biotin-N-hydroxysuccinimide ester (5 mg) in 300 µl of 0.2 M NaHCO₃ buffer/ 50 µl DMF. Analytical HPLC is used to monitor progress of the reation. HPLC and Sephadex G-25 purification give the PNA oligomer: CTG TCT CCA* TCC TCT TCA* CT (SEQ ID NO:68) wherein A* represents a PNA monomer functionalized to incorporate a biotin functionality linked via a pentylamino linking group to the hydroxyl group of the serine portion of the PNA.

### B. Functionalization with Fluorescein

### 1. Single Site Modification

A 1M Na₂CO₃/1M NaHCO₃ buffer (pH 9.0) is prepared by adding 1M NaHCO₃ to 1M Na₂CO₃. A 200 µl portion of this buffer is added to 10 O.D. units of PNA oligomer SEQ ID NO:63 (which is first treated to remove the phthaloyl protecting group as above) (see, Example **65**) in a microfuge tube. A 10 mg portion of fluorescein-isothiocyanate in 500 µl DMF is added to give a 0.05 M solution. A 100 µl portion of the fluorescein solution is added to the solution of PNA oligomer in the microfuge tube. The tube is covered with aluminum foil and allowed to stand overnight. The reaction mixture is applied to a Sephadex G-25 column (0.7 x 20 cm) that has been equilibrated with 25% (v/v) ethyl alcohol in water. The column is eluted with the same solvent. Product migration can be seen as a yellow band well separated from dark yellow band of the excess fluorescein reagent. The fractions showing absorption at 260 nm and 485 nm are combined and purified by HPLC as per the purification procedure of Example **66(A)(1)**. The product is lyophilized and desalted on Sephadex to give the PNA oligomer: CTG TCT CCA* TCC TCT TCA CT (SEQ ID NO:69) wherein A* represents a PNA monomer functionalized to incorporate a fluorescein functionality linked via a pentylamino linking group to the hydroxymethyl group at the C-1 position of the 2-aminoethyl portion of the PNA.

### 2. Multiple Site Modification

About 10 O.D. units (A₂₆₀) of PNA oligomer SEQ ID NO:64 is treated as per the procedures of Example **62** to remove the phthaloyl protecting group. The resulting oligomer (see Example **60)** is dissolved in 300 µl of the 1M Na₂HCO₃/ 1M Na₂CO₂ buffer of Example **61(B)(1)** and 200 µl of the fluorescein-isothiocyanate stock solution of Example **61(B)(1)** is added. The resulting solution is treated as per Example **61(B)(1).** Analytical HPLC is used to indicate the percent of doubly labeled product. Work up of the reaction gives the PNA oligomer: CTG TCT CCA* TCC TCT TCA* CT (SEQ ID NO:70) wherein A* represents a PNA monomer functionalized to incorporate a fluorescein functionality linked via an pentylamino linking group to the hydroxyl group of the serine portion of the PNA.

### C. Functionalization with Cholic Acid

### 1. Cholic Acid N-Hydroxysuccinimide Ester (Compound 61(C)(1)(A))

Anhydrous DMF (150ml) was added to a mixture of cholic acid (4.09g, 15mmol) and N-hydroxysuccinimide (5.25g, 45 mmol). The mixture was stirred in the presence of nitrogen. EDAC (4ml, 25mmol) was then added and the mixture was stirred overnight. The solution was then evaporated to a gum and partitioned between 1:1 ethyl acetate and 4% NaHCO₃ solution (pH 7.9) (100ml each). The organic layer was washed with saturated NaCl solution, dried over anhydrous Na₂SO₄ and evaporated to yield the title compound as a pale yellow foam (4.6g, 91%). ¹³C NMR (DMSO-d₆) δ 12.27, 16.71, 22.58, 22.80, 25.42, 26.19, 27.20, 28.49, 30.41, 30.56, 34.36, 34.82, 34.82, 35.31, 39.09, 39.09, 41.38, 41.53, 45.84, 46.05, 66.25, 70.45, 71.03, 169.28 and 170.16.

### 2. Single Site Modification

About 10 O.D. units (A₂₆₀) of PNA oligomer SEQ ID NO:63 is treated as per the procedures of Example **62** to remove the phthaloyl protecting group. The resulting oligomer *(see,* Example 60) is treated with cholic acid-NHS ester (Compound **1** in Application Serial Number 782,374, 5 mg, 9.9 µmols) in 200 µl of 0.2 M NaHCO₃ buffer/40 µl DMF. The reaction mixture is heated for 16 hours at 45°C. Analytical HPLC is used to determine the percent of product formation. Work up of the reaction gives the PNA oligomer: CTG TCT CCA˙ TCC TCT TCA CT (SEQ ID NO:71) wherein A* represents a PNA monomer functionalized to incorporate a cholic acid functionality linked via a pentylamino linking group to the hydroxymethyl group at the C-1 position of the 2-aminoethyl portion of the PNA.

### 3. Multiple Site Modification

About 10 O.D. units (A₂₆₀) of PNA oligomer SEQ ID NO:64 is treated as per the procedures of Example **62** to remove the phthaloyl protecting group. The resulting oligomer *(see,* Example **65**) is treated with cholic acid-NHS ester (Compound 1 in Application Serial Number 782,374, 10 mg, 19.8 µmols) in 300 µl of 0.2 M NaHCO₃ buffer/ 50 µl DMF. The reaction mixture is heated for 16 hours at 45°C. The product is isolated according to the method of Example **66(A)(1)**. Analytical HPLC is used to determine the percentages of doubly and singly labeled products. Work up according to the procedure of Example **66(A)(1)** gives the PNA oligomer: CTG TCT CCA* TCC TCT TCA* CT (SEQ ID NO:72) wherein A* represents a PNA monomer functionalized to incorporate a cholic acid functionality linked via a pentylamino linking group to the hydroxyl group of the serine portion of the PNA.

### D. Functionalization with Digoxigenin

### 1. Single Site Modification

About 10 O.D. units (A₂₆₀) of PNA oligomer SEQ ID NO:63 is treated as per the procedures of Example **62** to remove the phthaloyl protecting group. The resulting oligomer (see, Example **65**) is treated with digoxigenin-3-O-methylcarbonyl-ε-aminocaproic N-hydroxy succinimide ester (Boehringer Mannheim Corporation, Indianapolis, IN) in 200 µl of 0.1 M borate pH 8.3 buffer/ 40 µl DMF. The reaction mixture is allowed to stand overnight. The product is isolated according to the method of Example **66(A)(1)**. Work up of the reaction gives the PNA oligomer: CTG TCT CCA* TCC TCT TCA CT (SEQ ID NO:73) wherein A* represents a PNA monomer functionalized to incorporate a digoxigenin functionality linked via an pentylamino linking group to the hydroxymethyl group at the C-1 position of the 2-aminoethyl portion of the PNA.

### 2. Multiple Site Modification

About 10 O.D. units (A₂₆₀) of PNA oligomer SEQ ID NO:64 is treated as per the procedures of Example **62** to remove the phthaloyl protecting group. The resulting oligomer (*see*, Example **30**) is treated with digoxigenin-3-O-methylcarbonyl-ε-aminocaproic N-hydroxy succinimide ester (Boehringer Mannheim Corporation, Indianapolis, IN) in 300 µl of 0.1 M borate pH 8.3 buffer/50 µl DMF. The reaction mixture was allowed to stand overnight. The product was isolated according to the method of Example **66(A)(1).** Work up as per Example **66(A) (1)** gives the PNA oligomer: CTG TCT CCA* TCC TCT TCA* CT (SEQ ID NO:74) wherein A* represents nucleotides functionalized to incorporate a cholic acid functionality linked via a pentylamino linking group to the hydroxyl group of the serine portion of the PNA.

### EXAMPLE 67

### Functionalization of PNA Monomers with Pyrene N-[(N-Boc)-1-{(5-{4-(1-pyrenyl)butanoylamino}pent-1-yl)oxymethyl}-2-aminoethyl]-N-[(uracil-1-yl)acetyl]glycine ethyl ester (67) and N-[(N-Boc)-2-{(5-{4-(1-pyrenyl)butanoylamino}pent-1-yl)oxymethyl}-2-aminoethyl]-N-[(uracil-1-yl)acetyl]glycine ethyl ester (67A)

### A. Preparation of N-[(N-Boc)-1-(5-aminopentyloxymethyl)-2-aminoethyl]-N-(uracil-1-yl)acetyl]glycine ethyl ester (67a) and N-[(N-Boc)-2-(5-aminopentyloxymethyl)-2-aminoethyl]-N-[(uracil-1-yl)acetyl]glycine ethyl ester (67b)

The title compounds are synthesized as per the procedures of Examples **1** thru **11, 35, 39, 60,** and **62.**

### B. Preparation of N-[(N-Boc)-1-{(5-{4-(1-pyrenyl)-butanoylamino}pent-1-yl)oxymethyl}-2-aminoethyl]-N-[(uracil-1-yl)acetyl]glycine ethyl ester (67)

Compound **67a** or **67b** (0.78 mmol), are dissolved in anhydrous DMF (15 mL). 1-Hydroxybenzotriazole (1.17 mmol) and 1-pyrene-butyric acid pentafluorophenyl ester (1.17 mmol) are added to the reaction mixture. The mixture is stirred under argon at room temperature for 2 hours, after which it is concentrated *in vacuo*. Residual DMF is coevaporated with toluene. The residue is dissolved in dichloromethane (50 mL) and washed with an equal volume saturated NaHCO₃. The aqueous layer is washed with dichloromethane and the combined organic extracts are washed with an equal volume saturated NaCl. The aqueous layer is washed with dichloromethane and the combined organic layers dried over MgSO₄ and concentrated. The residue is purified by silica gel column chromatography to yield the respective product.

### EXAMPLE 68

### A. Preparation of N-[[(N-Boc)-1-[(5-(5-fluorosceinylthioureido)-pent-1-yl)oxymethyl]-2-aminoethyl]-N-[(uracil-1-yl)acetyl]glycine ethyl ester (68)

Fluorescein isothiocyanate (Isomer I, available from Cal Biochem, La Jolla, CA) was treated with 12 equivalents of pivaloyl chloride in Et₃N/THF to give di-O-pivaloyl fluorescein isothiocyanate. This compound was purified using a silica gel column with 3:1 hexane:ethyl acetate. Compound **67a** is then condensed with dipivaloyl fluorescein isothiocyanate in CH₂Cl₂/pyrimidine. The protecting groups are removed by treatment with dilute acid. The title compound is then purified by elution from a silica column using ethyl acetate hexane as the eluent.

### B. Preparation of N-[[(N-Boc)-2-[(5-(5-fluorosceinylthioureido)-pent-1-yl)oxymethyl]-2-aminoethyl]-N-[(uracil-1-yl)acetyl]glycine ethyl ester (68A)

Substituting **67b** for **67a** into the above procedure will give the title compound.

### EXAMPLE 69

### A. Preparation of N-[(N-Boc)-1-[1-({N-(cholesterol-O-carboxy)-5-aminopentyl}oxymethyl]-2-aminoethyl]-N-[(uracil-1-yl)acetyl]]glycine ethyl ester (69)

Compound **67a** (6.0 mmol) is dissolved in anhydrous pyridine/dichloromethane 50/50 (v/v) (20 mL). Cholesteryl chloroformate (Fluka, 6.68 mmol) is dissolved in anhydrous dichloromehthane (20 ml) and added slowly under argon with a syringe to the stirring reaction mixture. The mixture is stirred under argon at room temperature for 2 h after which it is concentrated *in vacuo.* Residual DMF is coevaporated with toluene. The residue is dissolved in dichloromethane (50 mL) and washed with an equal volume of saturated NaHCO₃. The aqueous layer is washed with dichloromethane and the combined organic extracts washed with an equal volume of saturated NaCl. The aqueous layer is washed with dichloromethane and the combined organic layers dried over MgSO₄ and concentrated. The residue is chromatographed on a silica gel column with a gradient of 25% ethyl acetate in hexanes to 100% ethyl acetate. The desired product elutes with 100% ethyl acetate and is assayed for purity by TLC.

### B. Preparation of N-[(N-Boc)-2-[1-({N-(cholesterol-O-carboxy)-5-aminopentyl}oxymethyl)-2-aminoethyl]-N-[(uracil-1-yl)acetyl]]glycine ethyl ester (69a)

Substituting **67b** for **67a** into the above procedure will give the title compound.

### EXAMPLE 70

### A. Preparation of N-[(N-Boc)-1-[{N-(2,4-dinitrophenyl)-5-aminopentyl}oxymethyl]-2-aminoethyl]-N-[(uracil-1-yl]acetyl]glycine ethyl ester (70)

Compound **67a** (1.37 mmol) is dissolved in methanol (20 mL). 2,4-Dinitrofluorobenzene (DNFB, 0.25 grams, 1.37 mmol) is added and the mixture shaken on a mechanical shaker. The reaction is monitored by TLC. After 90 min, another 0.25 grams of DNFB is added and the reaction mixture shaken an additional 30 min, followed by addition of another 0.25 grams of DNFB. After shaking 2.5 hours, the mixture is concentrated *in vacuo* and chromatographed on a silica gel column, eluting with a gradient of 25% ethyl acetate in hexanes to 100% ethyl acetate. The desired product elutes with 100% ethyl acetate and is assaye for purity by TLC.

### B. Preparation of N-[(N-Boc)-2-[{N-(2,4-dinitrophenyl)-5-aminopentyl}oxymethyl]-2-aminoethyl]-N-[(uracil-1-yl)acetyl]glycine ethyl ester (70A)

Substituting **67b** for **67a** into the above procedure will give the title compound.

### EXAMPLE 71

### A. Preparation of N- [(N-Boc) -1- [(N-(N_{α}-Nᵢₘ-Di-FMOC-_{L} histidinyl)-5-aminopentyl)oxymethyl]-2-aminoethyl]-N-[(uracil-1-yl)acetyl]glycine ethyl ester (71)

Compound **67a** (1.51 mmol) is dissolved in dichloromethane (25 mL) and cooled to 0°C in an ice bath. Nα, Nim-Di-FMOC-_{L}-histidine pentafluorophenyl ester (3.1 mmol, purchased from Sigma) and 1-hydroxybenzotriazole (0.24 mmol, purchased from Fluka) are added to the stirred reaction mixture stirred under argon. After 15 minutes, the ice bath is removed and the mixture stirred under argon at room temperature for 72 h. The mixture is concentrated *in vacuo* and chromatographed on a silica gel column, eluting with a gradient of 50% ethyl acetate in hexanes to 70% ethyl acetate in hexanes. The desired product elutes with 70% ethyl acetate.

### B. Preparation of N-[(N-Boc)-2-[(N-(N_{α}-Nᵢₘ-Di-FMOC-_{L}-histidinyl)-5-aminopentyl)oxymethyl]-2-aminoethyl]-N-[(uracil-1-yl]acetyl]glycine ethyl ester (71A)

Substituting **67b** for **78a** into the above procedure will give the title compound.

### EXAMPLE 72

### A. Preparation of N-[[(N-Boc)-1-[(N-(Ω-methyl-polyethylene glycol-propionoyl)-5-aminopentyl]oxymethyl]]-2-aminoethyl]-N-[(uracil-1-yl)acetyl]glycine ethyl ester (72)

Compound 67a, (1.55 mmol) is dissolved in anhydrous DMF (15 mL). 1-Hydroxybenzotriazole (1.75 mmol) and polyethylene glycol-propionic acid-NHS-ester (1.75 mmol) are added to the reaction mixture. The mixture is stirred under argon at room temperature for 2 hours after which it is concentrated *in vacuo.* Residual DMF is coevaporated with toluene. The residue is dissolved in dichloromethane (50 mL) and then washed with an equal volume saturated NaHCO₃. The aqueous layer is washed with dichloromethane and the combined organic extracts washed with an equal volume saturated NaCl. The aqueous layer is washed with dichloromethane and the combined organic layers dried over MgSO₄ and concentrated. The residue is chromatographed on a silica gel column, eluting with a gradient of 50% ethyl acetate in hexanes to 100% ethyl acetate.

### B. Preparation of N-[[(N-Boc)-2-[(N-(Ω-methylpolyethylene glycol-propionoyl)-5-aminopentyl)oxymethyl]]-2-aminoethyl]-N-[(uracil-1-yl)acetyl]glycine ethyl ester (72a)

Substituting **67b** for **67a** into the above procedure will give the title compound.

### EXAMPLE 73

### A. Preparation of Macrocycle derivatized PNA monomer

Compound **67a,** (1.55 mmol) is treated according to the procedures of Example **67** with the macrocycle 4-{1,4,8,11-tetraza-[tri-(trifluoroacetyl)cyclotetradec-1-yl]}methyl benzoic acid-N-hydroxy succinimide ester (prepared according to Simon Jones, *et. al., Bioconjugate Chem.* **1991,** 2, 416) to yield the product.

Substituting **67b** for **67a** into the above procedures will give the 2 substituted compound.

### EXAMPLE 74

### Functionalization with Folic Acid

### A. Preparation of N-[(N-Boc)-1-[(5-(N-folate)-aminopentyl)oxymethyl]-2-aminoethyl]-N-[(uracil-1-yl)-acetyl]glycine ethyl ester (74a)

Compound **67a**, is treated according to the procedures of Example **67** with folic acid pentafluorophenyl ester (protected with an isobutyryl protecting group) to yield the product.

### B. Preparation of N-[(N-Boc)-2-[(5-(N-folate)-aminopentyl)oxymethyl]-2-aminoethyl]-N-[(uracil-1-yl)-acetyl]glycine ethyl ester (74b)

Substituting **67b** for **67a** into the above procedures will give 2 substituted compound.

### EXAMPLE 75

### A. N-[(N-Boc)-1-[(N-(2-methoxy-6-chloro-9-(6-aminohexanoyl]acridine]-5-aminopentyl]oxymethyl]-2-amino-ethyl]-N-[(uracil-1-yl)acetyl]glycine ethyl ester (75)

6,9-Dichloro-2-methoxyacridine (Adlrich, 36 mmol) and phenol (2.5 g) were placed together on a round-bottom flask with a stirring bar and to this 6-amino-hexanoic acid (9.3 g, 71 mmol) was added and the flask was heated to 100° (oil bath) for 2 hours. TLC (10% methanol in methylene chloride) showed complete disappearance of starting material. The reaction mixture was cooled and poured into 200 mL of methanol. The product isolates out as a yellow solid (about 10 g). This compound was then converted into its pentafluorophenol ester.

Compound **67a** (78 mmol) is dissolved in anhydrous DMF (15 mL). 1-Hydroxybenzotriazole (1.17 mmol) and 2-methoxy-6-chloro-9-(6-aminohexanoyl)acridine pentafluorophenyl ester (1.17 mmol) are added to the reaction mixture. The mixture is stirred under argon at room temperature for 2 h, after which it is concentrated *in vacuo*. Residual DMF is coevaporated with toluene. The residue is dissolved in dichloromethane (50 mL) and washed with an equal volume of saturated NaHCO₃. The aqueous layer is washed with dichloromethane and the combined organic extracts washed with an equal volume of saturated NaCl. The aqueous layer is then washed with dichloromethane and the combined organic layers dried over MgSO₄ and concentrated. The residue is purified by silica gel column chromatography.

### B. Preparation of N-[(N-Boc)-2-[(N-(2-methoxy-6-chloro-9-(6-aminohexanoyl)acridine)-5-aminopentyl)-oxymethyl]-2-aminoethyl]-N-[(uracil-1-yl)acetyl]glycine ethyl ester (75a)

Substituting **62b** for **62a** into the above procedures will give 2 substituted compound.

### EXAMPLE 76

### A. Preparation of N-[(N-Boc)-1-{(-5-(N,N-dimethylamino)pentyl)oxymethyl}-2-aminoethyl]-N-[(uracil-1-yl)acetyl]glycine ethyl ester (76)

Compound **67a** (0.29 mmol) is dissolved in 4 ml methanol. Sodium acetate pH 4.0 (2 ml), sodium cyanoborohydride (0.3 mmol) and 37% formaldehyde in water (300 µl) are added to the reaction mixture, which is stirred 2 hours, after which it is concentrated *in vacuo*. The residue is dissolved in dichloromethane (50 mL) and washed with an equal volume saturated NaHCO₃. The aqueous layer is washed with dichloromethane and the combined organic extracts washed with an equal volume saturated NaCl. The aqueous layer is washed with dichloromethane and the combined organic layers dried over MgSO₄ and concentrated. The residue is chromatographed on a silica gel column, eluting with a gradient of 50% ethyl acetate in hexanes to 100% ethyl acetate. The desired product elutes with 10% Methanol-90% ethyl acetate.

### A. Preparation of N-[(N-Boc)-2-{(-5-(N,N-dimethylamino)pentyl)oxymethyl}-2-aminoethyl]-N-[(uracil-1-yl)acetyl]glycine ethyl ester (76a)

Substituting **67b** for **67a** into the above procedures will give 2 substituted compound.

### EXAMPLE 77

### Functionalization of PNA Oligomer with Reporter Enzymes, Peptides and Proteins

### A. Use of Heterobifunctional Linker

### 1. Synthesis of PNA-Maleimide Conjugate

PNA oligomer SEQ ID NO:63 is treated as per the procedures of Example **62** to remove the phthaloyl protecting group. The resulting oligomer is lyophilized in a 5 ml pear-shaped flask. Sulfo-SMCC reagent, Pierce Chemical Co. (Rockford, Il) (46 µmols) is dissolved in phosphate buffer (800 µl, 0.1M, pH 7.0) and added to the PNA oligomer bearing flask. An additional 200 µl of buffer are used to wash the reagent and transfer it to the flask containing the PNA oligomer. The contents of the flask are stirred overnight and loaded on to a Sephadex G-25 column (1 x 40 cm) equipped with a fraction collector. The PNA oligomer-maleimide conjugate containing fractions are collected and tested by analytical HPLC for separation from other NHS type products.

### 2. Synthesis of PNA oligomer-Peptide Conjugate

An aliquot of the PNA oligomer-maleimide conjugate of Example **77(A)(1)** (300 nmols) is lyophilized in a microfuge tube. SV40 peptide (pro-asp-lys-lys-arg-lys-cys)(about 2.5 µmols) is taken up in phosphate buffer (800 µl, 0.1 M, pH 7.0) and added to the PNA oligomer-maleimide conjugate containing tube. The contents of the tube are stirred overnight under an argon atmosphere. The reaction mixture is passed through a Sephadex G-25 column and the PNA-peptide conjugate fractions are identified by HPLC. Isolation of the product from product-bearing fractions via HPLC and desalting on Sephadex G-25 will yield a PNA oligomer of the sequence: CTG TCT CCA' TCC TCT TCA CT  SEQ ID NO:75 wherein A* represents a PNA monomer functionalized to incorporate a SV40 peptide functionality linked via a pentyl-amino-sulfo-SMCC (sulfosuccinimidyl-4-(N-maleimidomethyl)cyclohexane-1-carboxylate) linking group to the hydroxymethyl group of the designated PNA monomer.

### B. Use of Homobifunctional Linker

### 1. Synthesis of PNA Oligomer-Disuccinimidyl Suberate Conjugate

PNA oligomer SEQ ID NO:63 is treated as per the procedures of Example **62** to remove the phthaloyl protecting group. The resulting oligomer is evaporated to dryness and dissolved in freshly prepared 0.1 M NaHCO₃/50 mM EDTA (100 µl, pH 8.25). The solution is then treated with a solution of DSS, Pierce Chemical Co. (Rockford, Il) (7 µmol) in 200 µl DMSO. The solution is stored at room temperature for 15 minutes and then immediately applied to a Sephadex G-25 column (1 x 40 cm) which has been previously packed and washed with water at 4°C. The PNA oligomer fractions are combined immediately in a 25 ml pear-shaped flask and are rapidly frozen in dry ice/isopropyl alcohol and lyophilized to a powder.

### 2. Synthesis of PNA Oligomer-Protein Conjugate

A solution of calf intestinal alkaline phosphatase (Boehringer Mannheim) (2.06 ml, 147 nmol) is spun at 4°C in a Centricon microconcentrator at 6000 rpm until the volume is less than 50 µl. It is then redissolved in 1 ml of cold Tris buffer (pH 8.5, 0.1M containing 0.1 NaCl and 0.05 M MgCl₂) and concentrated twice more. Finally the concentrate is dissolved in 400 µl of the same buffer. This solution is added to the activated PNA oligomer from Example **77(B)(1)** and the solution stored for 18 hrs at room temperature. The product is diluted to approximately 30 ml and applied to a Sephadex G-25 column (1 x 20 cm, chloride form) maintained at 4°C. The column is eluted with 50 nM Tris-Cl pH 8.5 until the UV absorbance of the fractions eluted, reach near zero values. The column is then eluted with a NaCl salt gradient 0.05 M to 0.75 M (150 ml each). The different peaks are assayed for both PNA oligomer presence and alkaline phosphatase activity and the product-bearing fractions are combined. Typically the first peak will be excess enzyme, the second peak the PNA oligomer-protein conjugate and the third peak unreacted PNA oligomer. Isolation of the product from the product-bearing fractions via HPLC and desalting on Sephadex G-25 will yield a PNA oligomer of the sequence: CTG TCT CCA* TCC TCT TCA CT SEQ ID NO:76 wherein A* represents a PNA monomer functionalized to incorporate an alkaline phosphatase functionality linked via a DSS linking group to the aminopentyl oxymethyl of the designated PNA monomer.

### EXAMPLE 78

### Retinoic Acid Conjugated PNA oligomers

### A. Retinoic Acid N-Hydroxysuccinimide Ester

Anhydrous DMF (150 ml) was added to a mixture of retinoic acid (15 mmol, Fluka) and N-hydroxysuccinimide (45 mmol). The mixture was stirred in the presence of argon. EDAC [ethyl-3-(3-dimethylamino)propyl carbodiimide] (4 ml, 25 nmol) was then added and this mixture was then stirred overnight. The solution was then evaporated to a yellow gum and dissolved in 200 ml ethylacetate and washed successively with 4% NaHCO₃ solution (200 ml) followed by saturated NaCl solution, dried over anhydrous MgSO₄ and evaporated to yield the desired compound as a yellow solid in nearly 90% yield.

### B. Retinoic Acid Functionalized PNA Monomers

Compound **67a, 67b, 62,** or **62A** is dissolved in 200 µl of 0.2 M NaHCO₃ buffer and retinoic acid N-hydroxysuccinimide ester (7.3 µmols) is added followed by 40 µl DMF. The solution is allowed to stand overnight. The solution is then applied to a Sephadex G-25 column (0.7 x 15 cm) and the PNA oligomer fractions are combined. Analytical HPLC is used to determine the percentage conversion to the product. The product is purified by HPLC (Waters 600E with 991 detector, Hamilton PRP-1 column 0.7 x 15 cm; solvent A: 50 mM TEAA pH 7.0; B: 45 mM TEAA with 80% acetonitrile: 1.5 ml flow rate: Gradient: 5% B for first 5 minutes, linear (1%) increase in B every minute thereafter) and further desalted on Sephadex G-25 to give the retinoic acid-PNA monomer conjugate.

### C. Retinoic Acid Functionalized PNA Oligomers

The retinoic acid-PNA monomer conjugates of Example **78B** may be incorporated into PNA oligomers by deprotection of the PNA monomer carboxyl group and insertion of the resulting monomer into the synthetic procedures of Example **26**.

### EXAMPLE 79

### Folic Acid Conjugated Oligonucleotide

A mixture of folic acid (30 mg, 68 µmols) and 1-hydroxybenzotriazole (30 mg, 222 µmols) is dissolved in 9 ml of dry DMF. To this solution, 50 µL of EDAC (312 µmols) is added. The resultant yellow viscous material is vortexed well and 500 µL from the solution is transferred into a 0.2M NaHCO₃ buffer solution containing PNA oligomer SEQ ID NO:63 which has previously been treated according to the procedure of Example **62** to convert the NPHTH group to the free amine. The solution is vortexed, covered with aluminum foil and allowed to react for 16 hrs. The mixture is then loaded onto a Sephadex G-25 column (1 x 40 cm). The PNA oligomer fraction is collected, concentrated and passed one more time through a Sephadex G-25 column. Isolation of the product from the product-bearing fractions via HPLC and desalting on Sephadex G-25 will yield a PNA oligomer of the sequence: CTG TCT CCA* TCC TCT TCA CT SEQ ID NO:77 wherein A* represents a PNA monomer functionalized to incorporate a folic acid functionality linked to the aminopentyloxymethyl of the designated PNA monomer.

### EXAMPLE 80

### Methyl Folate Conjugated PNA Oligomer

In a like manner to Example **79**, 5-methyl folate is conjugated to deprotected PNA oligomers SEQ ID NO:63 and SEQ ID NO:64 to give the conjugated methyl folate group tethered to the C-1 position of the 2-aminoethyl portion of the PNA oligomer and to the Serine O portion when SEQ ID NO:64 is used which also has multiple sites for conjugation.

### EXAMPLE 81

### Pyridoxal Conjugated PNA Oligomer

PNA oligomer SEQ ID NO:63 or PNA oligomer SEQ ID NO:64 is treated according to the procedure of Example **62** to convert the N-PHTH group to the free amine. The resulting PNA oligomer is dissolved in 100 microliters of water, and 100 ml of 1M NaOAc buffer (pH 5.0) is added followed by 5 mg of pyridoxal hydrochloride (24 µmols) and 50 µl of 60 mM NaCNBH₃ solution. The solution is vortexed, left overnight, and is then passed through a Sephadex G-25 column and further purified in an analytical HPLC column.

### EXAMPLE 82

### Tocopherol Conjugated PNA Oligomer

### A. Vitamin E (Tocopherol)-hemisuccinate-NHS ester

α-Tocopherolhemisuccinate (Sigma, 5 g, 9.4 mmols) was treated with 3 equivalents of N-hydroxysuccinimide and 2 equivalents of EDAC as described above in Example **78A**. The work-up is the same as for Example **51**, and yields the title compound as a light brown wax-like solid.

### B. Tocopherol Conjugated PNA Oligomer

α-Tocopherol-hemisuccinate-NHS ester is treated according to the procedure of Example **78B** and **78C** above to obtain tocopherol attached via a tether to the desired PNA oligomer.

### EXAMPLE 83

### Conversion of a PNA Oligomer Having an Alkyl Aminolinker to a PNA Oligomer Having a Thiolinker

### A. PNA Oligomer SEQ ID NO:78

PNA oligomer (SEQ ID NO:63) H₂N-CTG TCT CCA* TCC TCT TCA CT-COOH wherein * represents a pentyl N-phthaloyl oxymethyl group attached at the C-1 position of the 2-aminoethyl portion of the designated monomer in the oligomer, is treated with hydrazine/ethanol followed by 5 mg SATA (N-succinimidyl-S-acetylthioacetate) in 0.2M NaHCO₃ buffer. The reaction mixture is passed through a Sephadex G-25 column, and the PNA oligomer fraction is concentrated and treated with 200 mM hydroxylamine hydrochloride solution in water (1 ml). The resulting PNA oligomer (SEQ ID NO:78) H₂N-CTG TCT CCA* TCC TCT TCA CT-COOH has a CH₂-O-(CH₂)₅-N-C(O)-CH₂-SH group attached to the C-1 position of the 2-aminoethyl portion of the designated (*) monomer in the oligomer.

### B. PNA Oligomer SEQ ID NO:79

PNA oligomer (SEQ ID NO:64) H₂N-CTG TCT CCA*** TCC *TCT* TCA*** CT-COOH wherein *** represents a pentyl N-phthaloyl oxymethyl group attached at the serine O- portion of the designated monomer in the oligomer, is treated with hydrazine/ethanol followed by 5 mg SATA (N-succinimidyl-S-acetylthioacetate) in 0.2M NaHCO₃ buffer. The reaction mixture is passed through a Sephadex G-25 column, and the PNA oligomer fraction is concentrated and treated with 200 mM hydroxylamine hydrochloride solution in water (1 ml) to give PNA oligomer (SEQ ID NO:79) H₂N-CTG TCT CCA*** TCC TCT TCA*** CT-COOH having a CH₂-O-(CH₂)₅-N-C(O)-CH₂-SH group attached to the serine O- portion of the designated (***) monomer in the oligomer.

### EXAMPLE 84

### Conjugation of o-Phenanthroline to PNA Oligomers

### A. PNA Oligomer SEQ ID NO:80

PNA oligomer (SEQ ID NO:78) H₂N-CTG TCT CCA* TCC TCT TCA CT-COOH from Example **83(A)** is treated with 2 mg of 5-(iodoacetamide)-*o*-phenanthroline reagent followed by shaking overnight. The oligomer is purified by a size exclusion column and reverse phase HPLC to yield; 
CTG TCT CCA* TCC TCT TCA CT-COOH  PNA Oligomer SEQ ID
NO:80
 wherein * represents a PNA monomer functionalized with phenanthroline via a thiol linker of the structure -CH₂-O-(CH₂)₅-NH-C(=O)-CH₂-S-CH₂-C(=O)-NH- at the C-1 position of the 2-aminoethyl portion of the designated monomer in the oligomer.

### B. PNA Oligomer SEQ ID NO:81

PNA oligomer (SEQ ID NO:79) H₂N-CTG TCT CCA*** TCC TCT TCA*** CT-COOH from Example **83B** having a CH₂-O-(CH₂)₅-N-C(O)-CH₂-SH group attached to the serine O- portion of the designated (***) monomer in the oligomer is treated with 2 mg of 5-(iodoacetamide)-*o*-phenanthroline reagent followed by shaking overnight. The oligomer is purified by a size exclusion column and reverse phase HPLC to yield 
H2N-CTG TCT CCA*** TCC TCT TCA*** CT-COOH PNA oligomer SEQ
ID NO:81
 wherein *** represents a PNA monomer functionalized at the serine O- position with phenanthroline via a thiol linker of the structure -CH₂-O-(CH₂)₅-NH-C(=O)-CH₂-S-CH₂-C(=O)-NH-.

### EXAMPLE 85

### Conjugation of Pyrene to PNA Oligomers

### A. Single site modification

PNA oligomer (SEQ ID NO:63) H₂N-CTG TCT CCA* TCC TCT TCA CT-COOH, where * denotes a pentyl N-phthaloyl oxymethyl group at the C-1 position of the 2-aminoethyl portion of the designated monomer, (approximately 60 nmols) is treated with hydrazine/ethanol to give the free amine. The free amine is dried in a microfuge tube and is dissolved in 200 µl of 0.2 M NaHCO₃ buffer. Pyrene-1-butyric acid N-hydroxysuccinimide ester (*i.e*., succinimidyl-1-pyrene butyrate, 3 mg, 7.79 µmols, Molecular Probes, Eugene, Oregon) is added followed by 400 µl of DMF. The mixture is incubated at 37°C overnight, and the solution is applied to a Sephadex G-25 column (1 x 40 cm). The PNA oligomer fractions are combined, and the product is purified by HPLC to give PNA oligomer (SEQ ID NO:82) H₂N-CTG TCT CCA* TCC TCT TCA CT-COOH, where * denotes a pyrene attached via a CH₂-O-(CH₂)₅-NHC(=O)-(CH₂)₃- linking group at the C-1 position of the 2-aminoethyl portion of the designated monomer.

### B. Multiple site modifications

PNA oligomer (SEQ ID NO:64) H₂N-CTG TCT CCA*** TCC TCT TCA*** CT-COOH, where *** denotes a pentyl N-phthaloyl oxymethyl group at the serine -O portion of the designated monomer, is treated with hydrazine/ethanol to give the free amine. The free amini is further treated with two equivalents of pyrene-1-butyric acid N-hydroxysuccinimide (6 mg in 400 µl DMF) and worked up in the same fashion as Example **85(A)**. Sephadex G-25 purification followed by HPLC purification gives the doubly pyrene-conjugated PNA oligomer SEQ ID NO:83 H₂N-CTG TCT CCA*** TCC TCT TCA*** CT-COOH where *** denotes a pyrene attached via a CH₂-O-(CH₂)₅-NHC(=O)-(CH₂)₃-linking group at the serine -O portion of the designated monomers in the oligomer. The oligomer is further purified as per the procedures of **85(A).**

### EXAMPLE 86

### Conjugation of Acridine to PNA Oligomers

### A. Single site modification

PNA oligomer SEQ ID NO:63 (2 µmol) is treated with hydrazine/ethanol to give the free amine. The free amine is dried and dissolved in 1M NaHCO₃/Na₂CO₃ buffer, pH 9.0, 200 µl. 9-Acridinyl-isothiocyanate, (5 mg, 2.1 µmols, Molecular Probes, Eugene, Oregon) is dissolved in 200 µl DMF. This solution is added to the PNA oligomer, and the mixture is vortexed, covered with aluminum foil and left at 37°C overnight. The reaction mixture is purified by passing through a Sephadex G-25 column (1 x 40 cm), concentrated and further purified by HPLC to give PNA oligomer SEQ ID NO:84 H₂N-CTG TCT CCA* TCC TCT TCA CT-COOH where * denotes a 9-acridinyl group attached via a CH₂-O-(CH₂)₅-NHSCN-linking moiety to the C-1 position of the 2-aminoethyl portion of the indicated (*) monomer.

### B. Multiple site modifications

PNA oligomer SEQ ID NO:64 H₂N-CTG TCT CCA*** TCC TCT TCA*** CT-COOH is treated with hydrazine/ethanol to give the free amine. The free amine is treated with 400 µl of 1M Na₂CO₃/NaHCO₃ buffer (pH 9.0) and further treated with 10 mg of 9-acridinyl-isothiocyanate in 400 µl of DMF. The reaction mixture is vortexed, covered with aluminum foil and left at 37°C overnight. The reaction mixture is purified as for the single site reaction of Example **85(A)**. The doubly conjugated acridine-oligonucleotide elutes as the last peak in the HPLC following single-modification products to give PNA oligomer SEQ ID NO:85 H₂N-CTG TCT CCA*** TCC TCT TCA*** CT-COOH where *** denotes a 9-acridinyl group attached via a CH₂-O-(OH₂)₅-NHSON-linking moiety to the serine O- position on the designated monomers in the oligomer.

### EXAMPLE 87

### Conjugation of Porphyrin to PNA Oligomers

Methylpyroporphyrin XX1 ethyl ester (Aldrich) is condensed with aminocaproic acid using N-hydroxysuccinimide and EDAC. The resultant carboxylic acid is then activated again with N-hydroxy succinimide and EDAC and treated with PNA oligomer SEQ ID NO:63 according to the procedure of Example **85(A)** to give PNA oligomer SEQ ID NO:86 H₂N-CTG TCT CCA* TCC TCT TCA CT-COOH wherein * denotes a 2-porphyrin group tethered to the C-1 position of the 2-aminoethyl portion of the designated PNA oligomer.

### EXAMPLE 88

### Conjugation of Hybrid Intercalator-Ligand to PNA Oligomers

### A. Photonuclease/intercalator ligand

The photonuclease/intercalator ligand 6-[[[9-[[6-(4-nitrobenzamido)hexyl]amino]acridin-4-yl]carbonyl]amino]-hexanoyl-pentafluorophenyl ester is synthesized according to the procedure of Egholm *et al., J. Am. Chem. Soc.* **1992,** *114,* 1895.

### B. Single site modification

10 O.D. units of PNA oligomer SEQ ID NO: 63 is treated with hydrazine/ethanol to deblock the amino group. The resultant material is dissolved in 100 µl of 0.1 M borate buffer (pH 8.4) and treated with 330 µl of DMF solution (10 mg in 1 ml of DMF) of 6-[[[9-[[6-(4-nitrobenzamido)-hexyl]amino]acridin-4-yl]carbonyl]amino]hexanoylpentafluorophenyl ester. The solution is covered with aluminum foil and allowed to react overnight. The reaction mixture is purified by Sephadex G-25 and HPLC purification to give PNA oligomer SEQ ID NO:87 H₂N-CTG TCT CCA* TCC TCT TCA CT-COOH wherein * denotes a photonuclease/intercalator ligand attached with a tether to the C-1 position of the 2-aminoethyl portion of the designated monomer in the oligomer.

### C. Multiple site modification

10 O.D. units A₂₆₀ of PNA oligomer SEQ ID NO:64 is treated with hydrazine/ethanol to deblock the protected amino positions. The resultant material is dissolved in 200 µl of 0.1 M borate buffer (pH 8.4) and treated with 660 µl of the DMF solution of 6-[[[9-[[6-(4-nitrobenzamido)hexyl]-amino]acridin-4-yl]carbonyl]amino]hexanoylpentafluorophenyl ester (10 mg in 1 ml solution) and the solution is covered with aluminum foil and left aside overnight. The solution is purified by Sephadex G-25 and reverse phase HPLC to give PNA oligomer SEQ ID NO:88 H₂N-CTG TCT CCA*** TCC TCT TCA*** CT-COOH wherein *** denotes a photonuclease/intercalator ligand attached with a tether to the serine O- position of the designated monomers in the oligomer.

### EXAMPLE 89

### Conjugation of Bipyridine Complex to PNA Oligomers

### A. Bipyridine complex

Succinimidyl-4-carboxyl-4'-methyl-2,2'-bipyridine is synthesized according to the procedure of Telser, *et al., J.* *Am. Chem. Soc.* **1989,** *111,* 7221.

### B. Single site Modification

10 O.D. A₂₆₀ units of PNA oligomer SEQ ID NO:63 is treated with hydrazine/ethanol to deblock the protected amino group. The resultant material is reacted with a 200 fold molar excess of succinimidyl-4-carboxyl-4'-methyl-2,2'-bipyridine in 0.1 M borate buffer pH 8.5/DMF. The solution is purified by Sephadex G-25 and reverse phase HPLC to yield PNA oligomer SEQ ID NO:89 H₂N-CTG TCT CCA* TCC TCT TCA CT-COOHH₂N-CTG TCT wherein * denotes a bipyridinyl complex attached via a linker to the C-1 position of the 2-aminoethyl portion of the designated monomer in the oligomer.

### EXAMPLE 90

### Conjugation of Aryl Azide Photocrosslinkers to PNA Oligomers

### A. Conjugation of N-hydroxysuccinimidyl-4-azidobenzoate (HSAB)

PNA oligomer SEQ ID NO:63 is treated with hydrazine/ethanol to give the free-amine containing PNA oligomer: CTG TCT CCA* TCC TCT TCA CT-COOH SEQ ID NO:90 wherein * denotes a pentylamino oxymethyl group attached at the C-1 of the 2-aminoethyl portion of the designated monomer in the oligomer.

PNA oligomer SEQ ID NO:90 (approx. 50 nmols) is dried, dissolved in 500 ml of 0.2M NaHCO₃ buffer pH 8.1 and treated with 25 mg of N-hydroxysuccinimidyl-4-azidobenzoate (HSAB, 96 µmols, available both from Pierce & Sigma) dissolved in 500 µl of DMF. The mixture is stirred overnight at 37°C and passed twice over Sephadex G-25 column (1 x 40 cm). The PNA oligomer fraction is purified by reverse-phase HPLC (5% → 40% CH₃CN in 60 min) on a reverse phase column to give PNA oligomer SEQ ID NO:91 CTG TCT CCA* TCC TCT TCA CT-COOH wherein * denotes an HSAB group attached via a tether to the C-1 position of the 2-aminoethyl portion of the designated monomer in the oligomer.

### B. Conjugation of N-succinimidyl-6(4'-azido-2'-nitrophenyl-amino)hexanoate

PNA oligomer SEQ ID NO:90 is dissolved in 500 ml of NaHCO₃ buffer (0.2M, pH 8.1) and treated with 500 µL of DMF containing 500 mg of N-succinimidyl-6(4'-azido-2'-nitrophenyl-amino)hexanoate (SANPAH, 128 µmols, available both from Pierce and Sigma). The reaction vial is wrapped with aluminum foil and heated at 37°C overnight. The reaction mixture is passed twice over a Sephadex G-25 column (1 x 40 cm). The PNA oligomer fraction is purified by reverse-phase HPLC (5% → 40% CH₃CN in 60 min.) on a reverse phase column to give PNA oligomer SEQ ID NO:92 CTG TCT CCA* TCC TCT TCA CT-COOH wherein * denotes a 6(4'-azido-2'-nitrophenylamino)hexanoate group attached via a tether to the C-1 position of the 2-aminoethyl portion of the designated monomer in the oligomer.

### EXAMPLE 91

### Conjugation of Imidazole-4-acetic acid to PNA Oligomers

### A. Activated imidazole-4-acetic acid

Imidazole-4-acetic acid was reacted with 2,4-dinitrofluoro benzene. The resulting imidazole-N-(DNP)-4-acetic acid was converted to its N-hydroxy succinimide ester by treating with NHS/EDAC according to the procedure of Example **78**.

### B. PNA conjugation

10 O.D. A₂₆₀ units of PNA oligomer SEQ ID NO:90 is reacted with a 100 fold molar excess of imidazole-N-(DNP)-4-acetic acid NHS ester in 0.1M borate buffer pH 8.5/DMF (200 µL each). The mixture is stirred overnight and further treated with 200 µL of mercaptoethanol to cleave off the DNP protecting group. The resulting reaction mixture is purified by passing through a Sephadex G-25 column followed by reverse phase HPLC to give PNA oligomer SEQ ID NO 93 CTG TCT CCA* TCC TCT TCA CT-COOH wherein * denotes an imidazolyl group attached via a tether to the C-1 position of the 2-aminoethyl portion of the designated monomer in the oligomer.

### EXAMPLE 92

### Conjugation of Metal Chelating Agents to PNA Oligomers

### A. EDTA Complex

To form an EDTA Fe(II) complex for coupling to a PNA oligomer as a nucleic acid cleaving agent, tricylohexyl ester of EDTA is synthesized according to the procedure of Sluka, *et al., J. Am. Chew. Soc.* **1990,** *112*, 6369.

### B. PNA Oligomer Modification

The tricyclohexyl ester of EDTA (1.25 mg, 1.94 mmol) and hydroxybenzotriazole (HOBt, 1 mg, 6.6 mmol) are dissolved in DMF (50 µL) and EDAC 10 µL is added. To this solution, PNA oligomer SEQ ID NO:90 (10 OD units) in 100 µL 0.1M borate buffer is added and left overnight. The solution is passed through a Sephadex G-25 column and the PNA oligomer fraction is treated with cone. NH₃ (100 µL) for 1 hr. to cleave off the acid protecting groups. Finally purification is effected by size exclusion chromatography and HPLC.

### C. DTPA Site Modification

PNA oligomer SEQ ID NO:90 is treated with diethylene triamine pentaacetic anhydride (DTPA) in 0.1M NaHCO₃/DMF to offer single-site modification. The conjugate is complexed with Gadolinium ion (Gd III) to form a contrasting agent, which is useful as, *inter alia,* an uptake measuring agent. The resulting PNA oliogmer SEQ ID NO:94 CTG TCT CCA* TCC TCT TCA CT-COOH wherein * denotes the complexed Gadolinium ion attached via a tether to the C-1 position of the 2-aminoethyl portion of the designated monomer in the oligomer.

### EXAMPLE 93

### Conjugation of Cholesterol To PNA Oligomers

### A. Method 1 - via aminolinker

Cholesterol-hemisuccinate is first converted to its N-hydroxy succinimide ester, which is then conjugated to PNA oligomer SEQ ID NO:90 according to the procedure of Example **85(A)** to give PNA oligomer SEQ ID NO:95 CTG TCT CCA* TCC TCT TCA CT-COOH wherein * denotes cholesterol attached via a tether to the C-1 position of the 2-aminoethyl portion of the designated monomer in the oligomer or PNA oligomer SEQ ID NO:64 which is first treated with hydrazine/ethanol to free the amine and then further treated as per the procedures of Example **85(A)** to give PNA oligomer SEQ ID NO:96 H₂N-CTG TCT CCA*** TCC TCT TCA*** CT-COOH wherein *** denotes a cholesterol attached via a tether to the serine O-position of the designated monomers in the oligomer.

### B. Method 2 - Conjugation of Cholesterol via a Disulfide Bridge

### 1. S-(2-thio-5-nitropyridyl)-thio cholesterol

Thiocholesterol (1.4 g, 3.5 mmol) is added to a stirred solution of 2,2'-dithiobis(5-nitropyridine) (1.4 g 4 mmol) in chloroform (20 mL) containing glacial acetic acid (400 µL) under an argon atmosphere. The reaction is allowed to continue overnight at room temperature, after which the precipitated 5-nitropyridine-2-thione is removed and the solution evaporated and purified on a silica column to give S-(2-thio-5-nitropyridyl)-thio cholesterol.

### 2. Conjugation to PNA Oligomer

PNA oligomer SEQ ID NO:59 H₂N-GCA*T-COOH, wherein * denotes a disulfide protected thiohexyloxymethyl linking group attached to the C-1 position of the 2-aminoethyl portion of the monomer in the oligomer, is reacted with an excess of S-(2-thio-5-nitropyridyl)-thiocholesterol to attach the cholesterol moiety to the thiol group of the PNA oligomer via a disulfide bridge to give PNA oligomer SEQ ID NO:97 H₂N-GCA*T-COOH wherein * denotes a cholesterol group attached via a tether to the C-1 position of the 2-aminoethyl portion of the monomer in the oligomer.

### Example 94

### Coupling of Conjugate Groups to Primary Amino Groups of a PNA or a PNA Derivative

PNAs and PNA derivatives having primary amino groups can have a conjugate group attached thereto including terminal amine groups and those "masked" during PNA oligomer synthesis and later regenerated.

Amino hexanoic acid (AHA) groups are added to PNA oligomers during solid phase synthesis as N-*t*-butyloxycarbonyl-ε-amino-hexanoic acid following the standard protocols.

### I. General Synthesis of PNA Conjugates via an Amino Group

### A. Coupling to Primary Amino Groups of H-PNA-NH₂, H-PNA-Lys-NH₂ and AHA-PNA-NH₂ (AHA = -NH-(CH₂)₅-C=(O)O-)

The three 2-aminoethylglycine-based PNA structures below were synthesized such that each has at least one free amino group: 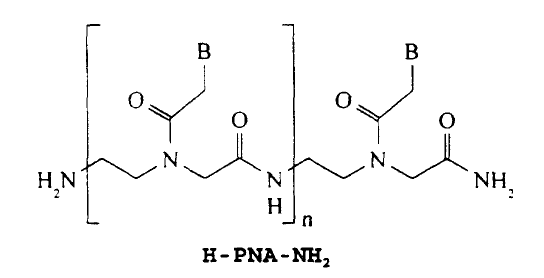 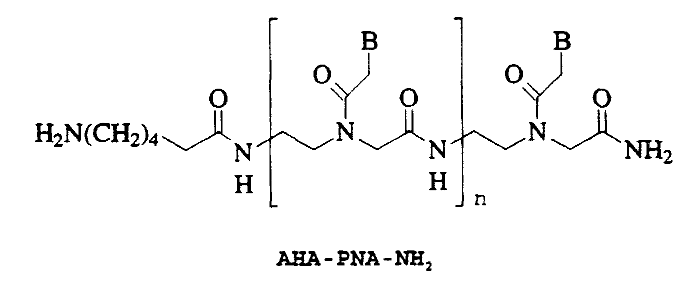 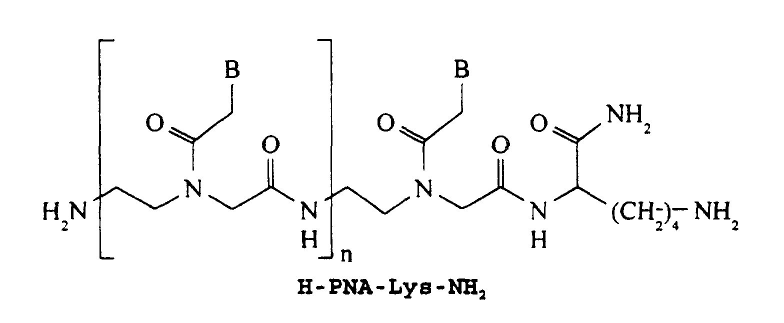 where B is a nucleobase or a derivative thereof.

### B. Coupling of D-biotin-N-hydroxysuccinimide Ester (BAE)

H-PNA-NH₂, H-PNA-Lys-NH₂, or AHA-PNA-NH₂ (0.2µL, 2.5 µg/µL in DMF) is added to a reaction flask containing 0.2M Na₂CO₃ (2.5 µL), 0.2M, NaHCO₃ (2.5 µL), and CH₃CN (5 µL). BAE (0.2µL, 20 µg/µL in DMF) is added to the reaction flask in three portions, while shaking the flask. The reaction is allowed to proceed at room temperature for 15 minutes after each addition. Fifteen minutes after the last addition the reaction mixture is separated by HPLC (reverse phase C-18 column) using the following two-buffer gradient system:

| Time (min) | % Buffer A | % Buffer B |
|---|---|---|
| 0 | 100 | 0 |
| 10 | 100-r | r |
| 40 | 100-q | q |
| 50 | 100 | 0 |
| Buffer A = 0.1% TFA in H₂O. | | |
| Buffer B = 0.1% TFA; 60% CH₃CN; 40% H₂O | | |

### C. Coupling of Acridine-N-hydroxysuccinimide Ester (AAE)

H-PNA-NH₂, H-PNA-Lys-NH₂, or AHA-PNA-NH₂ (27.5 µg) is added to a reaction flask containing 0.2M Na₂CO₃ (2.5 µL), 0.2M, NaHCO₃ (2.5 µL), and CH₃CN (5 µL). AAE (0.7µL, 20 µg/µL in DMF) is added to the reaction flask in three portions, while shaking the flask. The reaction is allowed to proceed at room temperature for 15 minutes after each addition. Fifteen minutes after the last addition the reaction mixture is separated by HPLC according to the procedure of Example **44(B).**

### D. Coupling of Azidobenzoyl Chloride (ABC)

H-PNA-NH₂, H-PNA-Lys-NH₂, or AHA-PNA-NH₂ (1.3 µL, 2.5 µg/µL in DMF) is added to a reaction flask containing 0.2M Na₂CO₃ (10 µL), and CH₃CN (10 µL). Azidobenzoyl chloride (30µg) is added to the reaction flask, which is allowed to remain at room temperature for fifteen minutes. Orthophosphoric acid (0.5 µl, 85% solution) is added, and the solution is extracted with chloroform five times. The aqueous phase is then separated on the HPLC according to the procedure of Example **94(B)**.

### E. Coupling of Alkanoyl Chloride

H-PNA-NH₂, H-PNA-Lys-NH₂, or AHA-PNA-NH₂ (1.3 µL, 2.5 µg/µL DMF) is added to a reaction flask containing 0.2M Na₂CO₃ (2.5 µL), NaHCO₃ (2.5 µL), and CH₃CN (5 µL). One microliter of the alkanoyl chloride is added to the reaction flask with shaking, and the reaction is allowed to proceed at 37°C for 15 minutes. The aqueous solution is extracted with hexane three times, and the resulting aqueous phase is separated by HPLC according to the procedure of Example **94(B).**

### F. Coupling of Cholesteryl Chloroformate (CC)

H-PNA-NH₂, H-PNA-Lys-NH₂, or AHA-PNA-NH₂ (1.3 µL, 2.5 µg/µL in DMF) is added to a reaction flask containing 0.2M Na₂CO₃ (2.5 µL), and CHCl₃ (2.5 µL). Cholesteryl chloroformate (30 µg) is added to the reaction flask, which is shaken at room temperature for five days and nights. The aqueous phase is then separated on the HPLC according to the procedure of Example **94(B).**

### G. Coupling of Dansyl Chloride (DC)

Dansyl chloride (0.16 µL of a 1 µg/µL in CH₃CN) is added to a reaction flask containing 0.2M Na₂CO₃ (0.5 µL) and 0.2M NaHCO₃ (0.5 µL). H-PNA-NH₂, H-PNA-Lys-NH₂, or AHA-PNA-NH₂ (3.34 µL, 2.5 µg/µL DMF) is then added, and the reaction is allowed to proceed in the dark at room temperature for 1.5 hours. The reaction mixture is then separated by HPLC according to the procedure of Example **94(B).**

### H. Coupling of Folic Acid (FA)

H-PNA-NH₂, H-PNA-Lys-NH₂, or AHA-PNA-NH₂ (1.3 µL, 2.5 µg/µL DMF) is placed in a reaction flask containing folic acid (0.5 µL of 1µg/µL in DMSO), EDC (0.16 µL, 10 µg/µL in DMSO) and DMSO (3.34 µL). The reaction is allowed to proceed overnight in the dark at room temperature, and then folic acid (0.5 µL, 1µg/µL in DMSO) and EDC (0.16 µL, 10 µg/µL in DMSO) are added to the flask. The reaction is allowed to proceed another six hours in the dark at room temperature, followed by another addition of folic acid (0.5 µL, 1µg/µL in DMSO) and EDC (0.16 µL, 10 µg/µL in DMSO). The reaction is allowed to continue overnight in the dark at room temperature. Water (16.26 µL) is added, and the reaction mixture is lyophilized, taken up in water, and separated by HPLC according to the procedure of Example **94(B).**

### I. Coupling of Maleimido Active Ester (N-Maleimidopropionic Acid N-hydroxysuccinide Ester) (MAE)

H-PNA-NH₂, H-PNA-Lys-NH₂, or AHA-PNA-NH₂ (1.3 µL, 2.5 µg/µL in DMF) is added to a reaction flask containing 0.2M Na₂CO₃ (1.25 µL), 0.2M NaHCO₃ (1.25 µL), and CH₃CN (6.9 µL). MAE (0.2 µL, 20 µg/µL in DMF) is added to the reaction flask in three portions, while shaking the flask. The reaction is allowed to proceed at room temperature for 15 minutes after each addition. Fifteen minutes after the last addition the reaction mixture is separated by HPLC according to the procedure of Example **94(B)** except that TFA is omitted from the gradient, and the product is collected.

### J. Coupling of Quinone active ester (anthraquinone-2 carboxylic acid-ODhbt ester) (QAE)

H-PNA-NH₂, H-PNA-Lys-NH₂, or AHA-PNA-NH₂ (1.3 µL, 2.5 µg/µL in DMF) is added to a reaction flask containing 0.2M Na₂CO₃ (1.3 µL), 0.2M NaHCO₃ (1.3 µL), and CH₃CN (5.2 µL). QAE (0.9 µL, 5 µg/µL in DMF) is added to the reaction flask in three portions, while shaking the flask. The reaction was allowed to proceed at room temperature for 15 minutes after each addition. Fifteen minutes after the last addition the reaction mixture is separated by HPLC according to the procedure of Example **94(B)**.

### K. Coupling of Fluorescein

A PNA or PNA derivative having a single free NH₂ such as H₂N-GCAT-COOH is dissolved in tetrahydrofuran:water to provide a solution that is 0.1 M in PNA and to this is added fluorescein isothiocyanate to give a solution which is 0.1-1.0 M in fluorescein isothiocyanate. The solution is stirred for 0.1-2 hours, then evaporated to a solid and purified by preparative HPLC.

### L. Coupling of EDTA

EDTA conjugates are prepared from EDTA-tribenzyl ester by standard solid phase synthesis. Purification is by HPLC.

### M. Coupling of Nitrillo Triacetic Acid (NTA)

NTA conjugates are prepared as dibenzylesters from glycine-tert-butylester using standard solid phase synthesis. Purification is by HPLC.

### N. Coupling of 5-Amino-1,10-phenanthrolineglutaramide (Phen₁)

### 1. 5-Amino-1,10-phenanthroline-glutarimide

5-Amino-1,10-phenanthroline (350mg, 1.8mmol) was refluxed with glutaric anhydride (2.0g, 17.5mmol) in pyridine (3 mL) and triethylamine (3 mL) for 2 hours. The reaction mixture was reduced to a viscous oil by rotary evaporation. Column purification on silica with 10% methanol in methylene chloride (product produced bright red spots with Fe(II) on TLC) gave the crude product (320mg 1.1mmol). Further purification of the crude product was by recrystalization once from 30 mL boiling water to give the title compound (250mg, 0.85mmol) as centimeter long transparent needles.

### 2. 5-Amino-1,10-phenanthroline-glutaramide

The purified title compound from **94(I)N(1)** above (250mg, 0.85mmol) was refluxed in water (3 mL) and triethylamine (3 mL) for 10 minutes. The material was evaporated on a rotary evaporator and further coevaporated with 3, 10 mL portions of water. This afforded the title compound in a quantative yield with a trace of triethylamine as a contaminant. The material was recrystalized from water (30 mL) to give after drying the purified title compound (200mg, 0.61mmol) (72%) as a white macrocrysaline monohydrate.

### 3. Coupling to PNA

The triethylammonium salt of 5-Amino-1,10-phenanthroline-glutaramide (60mg, 0.14mmol) from Example **94(I)N(2)** above was dissolved in DMF (1 mL) and pyridine (0.5 mL). HBTU (45 mg, 0.12mmol) was added followed by diisopropylethylamine (0.05 mL, 0.3mmol). This solution is then added to an N-terminal end of a growing PNA or can be used to functionalize gel.

### O. Coupling of Piperazine-N-acetic acid-N'-acetic acid-(5'-phenanthryl)-amide (Phen₂)

### 1. Piperazine-N-acetic acid-tert-butylester-N'-acetic acid-(5'-phenanthryl)-amide

Iodoacetamido-phenanthroline (150mg, 0.41mmol) and piperazineacetic acid-tert-butylester (90mg, 0.48mmol) were mixed in DMF (1 mL) and triethylamine (0.5 mL). The reagents were stirred for 1 hour at room temperature and then the solvents were removed by rotary evaporator. The product was purified by silica gel column chromatography with pyridine (15%) in DMF as the eluent (product produced bright red spots with Fe(II) on TLC) to give the title compound in a 70% yield (120mg, 0.29mmol).

### 2. Piperazine-N-acetic acid-N'-acetic acid-(5'-phenanthryl)-amide

Piperazine-N-acetic acid-*tert*-butylester-N'-acetic acid-(5'-phenanthryl)-amide (120mg, 0.29mmol) was dissolved in dichloromethane (3 mL) and trifluoroacetic acid (3 mL). The mixture was stirred at room temperature for 3 hours and then the solvent was removed in *vacuo* at 0.1 mmHg. This gave the title compound (with traces of starting material) in a quantitative yield as a trifluoroacetate.

### 3. Coupling to PNA

Piperazine-N-acetic acid-N'-acetic acid-(5'-phenanthryl)-amide (150mg, 0.25mmol) as a trifluoroacetate was dissolved in DMF (2mL) and diisopropylethylamine (1mL). To the resulting solution (1.5mL) was added HBTU (38mg, 0.1mmol) and this solution is then added to an N-terminal end of a growing PNA or can be used to functionalize gel.

### P. Coupling of Fluorₚₕₑₜ and Fluorₘₑₜ

### 1. Fluorescein-(2-phenyl)ethylester-O-acetic acid-tert-butylester

Fluorescein-(2-phenyl)ethylester (3g, 6.9mmol) was dissolved in DMF (10mL) and triethylamine (2.5mL). The mixture was heated to 70 °C, and bromoacetic acid tert-butylester (2g, 10mmol) was added. The reaction mixture was maintained at 70 °C for 2 hours and then cooled to room temperature. Dichloromethane (40ml) was added and the resulting solution was extracted with 5% NaHCO₃ (2 X 10 mL). The organic phase was dried over Na₂CO₃, filtered and evaporated in *vacuo.* The residue was purified twice using silica gel column chromatography with methanol (3-15%) in dichloromethane as the eluent. The title was obtained in a 53% yield (2.0g, 3.6mmol).

### 2. Fluorescein-(2-phenyl)ethylester-O-acetic acid

Fluorescein-(2-phenyl)ethylester-O-acetic acid-*tert*-butylester (2.0g, 3.6mmol) was dissolved in dichloromethane (10ml) and trifluoroacetic acid (10 mL). The mixture was stirred overnight and then evaporated to a viscous oil. Following 2 column purifications with methanol (10%) in dichloromethane as the eluent the title compound precipitated from the last eluate, and was dissolved in warm ethanol (96%, 150 mL). The ethanol was evaporated to leave approximately 30 mL and upon cooling the title compound precipitated. The title compound was isolated as a microcrystaline red powder in a 78% yield (1.4g, 2.8mmol).

### 3. Fluorescein-monomer-ethylester

Fluorescein-(2-phenyl)ethylester-O-acetic acid (1.08g, 2.0mmol) and Compound 8, N-Boc-aminoethyl glycine ethyl ester (500mg, 2.0mmol) was dissolved in DMF (15 mL) and dichloromethane (5mL), HDBTU (324mg) and DCC (480mg) were added. The mixture was heated to 50 °C for 1 hour and then allowed to stand at room temperature overnight. DCU was filtered off and ether (50 mL) was added followed by washing with 5% NaHCO₃ (20 mL) and 5% NaHSO₄ (20 mL). The organic phase was dried and evaporated to dryness. Purification was by silica gel column chromatography using methanol (0-15%) in dichloromethane as eluent. The title conmpound was isolated in a 67% yield (0.98g, 13.5mmol).

### 4. Fluorescein-monomers Fluorₚₕₑₜ and Fluorₘₑₜ

Fluorescein-(2-phenyl)ethylester (300mg, 41mmol) was dissolved in THF (20mL) and LiOH (1 M, 5 mL) was added. The mixture was stirred for 35 minutes at room temperature, poured onto ice/dilute NaHSO₄ and was extracted with ethyl acetate. The organic phase was dried and evaporated to dryness. Purification was by silica gel column chromatography using DMF as eluent. The title conmpound Fluorₚₕₑₜ was isolated as a dark red oil in a 30% yield (100mg, 1.4mmol). The monomers are incorporated into PNA oligomers using standard protocols. (The transesterification product Fluorₘₑₜ was also observed in one synthesis and used to form a PNA conjugate.)

### Q. PNA-Staphylococcal Nuclease Conjugate

Maleimido-PNA derivative (3µg) from **94(I)** above was dissolved in 10mM NaPO₄ buffer (100 µL, pH 7.0) and was incubated with SH-straphylococcal nuclease mutant Lys116-Cys (10 µg) (Corey, *et.al*., *Science*, **1987**, 238, 1401), for 2 hours at room temperature. The PNA-nuclease conjugate was separated from excess maleimido-PNA by chromatography on a Sephadex G-50 in 10 mM NaPO₄ pH 7.0.

### II. Coupling of Conjugates to AHA-T₂CT₂CT₄-NH₂

### A. Acridine-AHA-T₂CT₂CT₄-NH₂

PNA AHA-T₂CT₂CT₄-NH₂ (SEQ ID NO:98) was synthesized using standard methods and techniques as per Example **26**. 3300µg of PNA (SEQ ID NO:98) was dissolved in 0.2M Na₂CO₃ (300µL), 0.2M NaHCO₃ (300µL) and CH₃CN (600µL). Acridine-N-hydroxy-succinimide-ester (AAE) (100µL, 20µg/µL DMF) was added in 5 portions while shaking the reaction vessel. The mixture was left for 15 minutes at room temperature after each portion of AAE. The reaction was monitored by HPLC using a reversed phase spherisorp S50DS1 C-18 column (250x4.6 mm) and a gradient of varying concentrations of Buffer A (0.1% TFA in H2O) and Buffer B (0.1% TFA, 60% CH₃CN, 40% H₂O). The retention time of the starting material PNA AHA-T₂CT₂CT₄-NH₂ (SEQ ID NO:98) was approxamately 15 minutes and that of the final PNA, acridine-AHA-T₂CT₂CT₄-NH₂ was 23 minutes.

After completion of the reaction, the PNA was precipitated with 12 ml of isobutanol, lyophilized and then redissolved in 1 ml H₂O. The acridine-PNA was purified by HPLC using a reversed phase C-18 column and the same conditions as above.

In a subsequent binding assay of the acridine-PNA to dsDNA the Kd shows that there is a 10-100 fold increase in the binding compared to that of unmodified PNA and that it binds to dsDNA at 150 mM salt at µmolar (6.5) concentrates.

### B. Anthraquinone-AHA-T₂CT₂CT₄-NH₂

115µg of PNA AHA-T₂CT₂CT₄-NH₂ (SEQ ID NO:98) was dissolved in 0.2M Na₂CO₃ (111µL), 0.2M NaHCO₃ (111µL) and CH₃CN (444µL). Anthraquinone-DHBT-ester (QAE) (79.5µL, 5µg/µL CH₃Cl) was added in 3 portions while shaking the reaction vessel. The mixture was left for 10 minutes at room temperature after each portion of QAE was added. The reaction was monitored by HPLC using a reversed phase spherisorp S50DS1 C-18 column (250x4.6 mm) and a gradient of varrying concentrations of Buffer A (0.1% TFA in H2O) and Buffer B (0.1% TFA, 60% CH₃CN, 40% H₂O). The retention time of the starting material PNA AHA-T₂CT₂CT₄-NH₂ (SEQ ID NO:98) was approxamately 21 minutes and that of the final PNA, acridine-AHA-T₂CT₂CT₄-NH₂ was 30 minutes.

After completion of the reaction, the H₂O phase with the PNA was lyophilized and then redissolved in 1 ml H₂O. The Quinone-PNA was purified by HPLC using a reversed phase C-18 column and the same conditions as above.

### C. Biotin-AHA-T₂CT₂CT₄-NH₂

78µL, 2.5µg/µL of PNA AHA-T₂CT₂CT₄-NH₂ (SEQ ID NO:98) was dissolved in 0.2M Na₂CO₃ (150µL), 0.2M NaHCO₃ (150µL) and CH₃CN (300µL). Biotin-N-hydroxy-succinimide-ester (BAE) (120µL, 2µg/µL DMF) was added in 4 portions while shaking the reaction vessel. The mixture was left for 15 minutes at room temperature after each portion of BAE was added. The reaction was monitored by HPLC using a reversed phase spherisorp S50DS1 C-18 column (250x4.6 mm) and a gradient of varrying concentrations of Buffer A (0.1% TFA in H2O) and Buffer B (0.1% TFA, 60% CH₃CN, 40% H₂O). The retention time of the starting material PNA AHA-T₂CT₂CT₄-NH₂ (SEQ ID NO:98) was approxamately 15 minutes and that of the final PNA, acridine-AHA-T₂CT₂CT₄-NH₂ was 41 minutes.

After completion of the reaction, the H₂O phase with the PNA was lyophilized and then redissolved in 0.45 ml H₂O. The biotin-PNA was purified by HPLC using a reversed phase C-18 column and the same conditions as above.

### D. Folic Acid-AHA-T₂CT₂CT₄-NH₂

400µL 2.5µg/µL of PNA AHA-T₂CT₂CT₄-NH₂ (SEQ ID NO:98) was mixed with 154µL 1.0µg/µL DMSO folic acid, 50µL 10µg/µL of DMSO EDC, and 1228µL DMSO. The mixture was left overnight in the dark at room temperature. The next day 150µL 1.0µg/µL DMSO folic acid plus 50µL 10µg/µL DMSO EDC was added and the mixture was left for 6 hours in the dark at room temperature. Then 150µL 1.0µg/µL DMSO folic acid plus 50µL 10µg/H₂O DMSO EDC was added and the mixture was left overnight in the dark at room temperature. The following day H₂O (5mL) was added and the H₂O-phase was lyophilized and then redissolved in 400µL 1:1 mixture of H₂O and DMSO. The reaction was monitored by HPLC using a reversed phase spherisorp S50DS1 C-18 column (250x4.6 mm) and a gradient of varrying concentrations of Buffer A (0.1% TFA in H2O) and Buffer B (0.1% TFA, 60% CH₃CN, 40% H₂O). The retention time of the starting material PNA AHA-T₂CT₂CT₄-NH (SEQ ID NO:98) was approxamately 24 minutes and that of the final PNA, folic acid-AHA-T₂CT₂CT₄-NH was 34 minutes.

The folic acid PNA was purified by HPLC using a reversed phase C-18 column and the same conditions as above.

### E. Octanoyl-AHA-T₂CT₂CT₄-NH₂

370µL, 2.0µg/µL of PNA AHA-T₂CT₂CT₄-NH₂ (SEQ ID NO:98) was dissolved in 0.2M Na₂CO₃ (340µL), 0.2M NaHCO₃ (340µL) and CH₃CN (680µL). 80µL CH₃(CH₂)₆COCl was added in 4 portions while shaking the reaction vessel. The mixture was left for 15 minutes at room temperature after each portion of CH₃(CH₂)₆OOCl was added. The reaction was monitored by HPLC using a reversed phase spherisorp S50DS1 C-18 column (250x4.6 mm) and a gradient of varrying concentrations of Buffer A (0.1% TFA in H2O) and Buffer B (0.1% TFA, 60% CH₃CN, 40% H₂O). The retention time of the starting material PNA AHA-T₂CT₂CT₄-NH (SEQ ID NO:98) was approxamately 21 minutes and that of the final PNA, acridine-AHA-T₂CT₂CT₄-NH was 30 minutes.

After completion of the reaction, the H₂O phase with the PNA was lyophilized and then redissolved in 0.45 ml H₂O. The octanoyl-PNA was purified by HPLC using a reversed phase C-18 column and the same conditions as above.

### F. Psoralen-AHA-T₂CT₂CT₄-NH₂

4600µg of PNA AHA-T₂CT₂CT₄-NH (SEQ ID NO:98) was dissolved in H₂O (920µL), 0.2M NaHCO₃ (230µL), and 1917µL DMF. 552µL 10µg/µL DMF psoralene-N-hydroxy-succinimide-ester (PAE) was added in 4 portions while shaking the reaction vessel. The mixture was left for 15 minutes at room temperature after each portion of PAE was added. The reaction was monitored by HPLC using a reversed phase spherisorp S50DS1 C-18 column (250x4.6 mm) and a gradient of varrying concentrations of Buffer A (0.1% TFA in H2O) and Buffer B (0.1% TFA, 60% CH₃CN, 40% H₂O). The retention time of the starting material PNA AHA-T₂CT₂CT₄-NH (SEQ ID NO:98) was approxamately 20 minutes and that of the final PNA, psoralen-AHA-T₂CT₂CT₄-NH was 30 minutes.

After completion of the reaction, the H₂O phase with the PNA was lyophilized and then redissolved in 1 mL H₂O. The psoralen-PNA was purified by HPLC using a reversed phase C-18 column and the same conditions as above (SEQ ID NO:99)        Acridine-AHA-T2CT2CT4-NH2 (SEQ ID NO:100)    AnthraQuinone-AHA-TTC TTC TTT T-NH2 (SEQ ID NO:101)     Biotin-AHA-TTC TTC TTT T-NH2 (SEQ ID NO:102)    Folic acid-AHA-TTC TTC TTT T-NH2 (SEQ ID NO:103)     Octanoyl-AHA-TTC TTC TTT T-NH2 (SEQ ID NO:104)    Psoralen-AHA-T2CT2CT4-NH2 (SEQ ID NO:105)    H-TTT TTT TTT T-Lys-Biotin (SEQ ID NO:106)    Biotin-H-TTT TTT TTT T-Lys-Biotin (SEQ ID NO:107)     H-TTT TTT TTT T-Lys-Hexyl-Biotin (SEQ ID NO:108)     Biotin-AHA-Tyr-TTC TTC TTT T-Lys-NH2 (SEQ ID NO:109)    Biotin-AHA-CTC TTT TTT T-NH2 (SEQ ID NO:110)     Biotin-TTT CTT CTC ACT TCT T-NH2 (SEQ ID NO:111)     Biotin-Hexyl-TTT CTT CTC ACT TCT T-NH2 (SEQ ID NO:112)     H-TTT TTT TTT T-Lys-Maleimide (SEQ ID NO:113)     Maleimide-H-TTT TTT TTT T-Lys-Maleimide (SEQ ID NO:114)     H-Tyr-TTT TTT TTT T-Lys-Maleimide (SEQ ID NO:115)    Maleimide-AHA-TTC TTC TTT T-NH2 (SEQ ID NO:116)    Maleimide-AHA-Tyr-TTC TTC TTT T-NH2 (SEQ ID NO:117)    Maleimide-TTT TT-NH₂ (SEQ ID NO:118)    Quinone-AHA-TTC TTC TTT T-NH2 (SEQ ID NO:119)    Quinone-AHA-Tyr-TTC TTC TTT T-NH2 (SEQ ID NO:120)    Acridine-AHA-TTC TTC TTT T-NH2 (SEQ ID NO:121)    Acridine-AHA-TTT TGG TGT GGG TCT-NH2 (SEQ ID NO:122)    N3-Benzoyl-AHA-TTC TTC TTT T-NH2 (SEQ ID NO:123)    N₃-Benzoyl-TTT CTT CTC ACT TCT T-NH₂ (SEQ ID NO:124)    H-GTA GAT CAC T-Lys-Benzoyl-N3 (SEQ ID NO:125)    H-AGT CAT CTA C-Lys-Benzoyl-N3 (SEQ ID NO:126)     Dodecanoyl-AHA-TTC TTC TTT T-NH2 (SEQ ID NO:127)    Dodecanoyl-AHA-CTC TTT TTT T-NH2 (SEQ ID NO:128)    Myristoyl-AHA-CTC TTT TTT T-NH₂ (SEQ ID NO:129)     Cholesteryl-formyl-AHA-TTC TTC TTT T-NH2 (SEQ ID NO:130)     Cholic Acid-FLUOphet-CTC TTT TTT T-NH2 (SEQ ID NO:131) Dansyl-TTT TT-NH2 (SEQ ID NO:132)     PHEN1-AHA-TGT ACG TCA CAA CTA-NH2 (SEQ ID NO:133)    EDTA-AHA-TGT ACG TCA CAA CTA-NH2 (SEQ ID NO:134)     EDTA-AHA-TTC TTC TTT T-NH2 (SEQ ID NO:135)     EDTA-AHA-Lys₂-TTC TTC TTT T-Lys₂NH₂ (SEQ ID NO:136)    PHEN2-AHA-TTC TTC TTT T-NH2 (SEQ ID NO:137)    PHEN2-AHA-Lys-Lys-TTC TTC TTT T-NH2 (SEQ ID NO:138)    NTA-AHA-TTC TTC TTT T-NH2 (SEQ ID NO:139)    NTA-AHA-Lys₂-TTC TTC TTT T-Lys₂-NH₂ (SEQ ID NO:140)    FLUOₘₑₜ-AHA-CTC TTT TTT T-NH₂ where Lys is lysine, Tyr is tyrosine, AHA is amino hexanoic acid, NTA is nitrillo triacetic acid, PHEN₁ is a substituted phenathroline as reported by Jenkins, Y. and Barton, J.K., *JACS* **1992**, *114*, 8736-38. PHEN₂ is a similar compound that incorporates a pipirazine linker. FLUOₚₕₑₜ is a partially deprotected flourescein monomer while FLUOₘₑₜ is a transeterified flourescein product.

### Example 95

### N-Terminal Polyamine End Labeled PNA Oligomers

Polyamines are attached to the N-terminal end of PNA oligomer having the sequence H₂N-GCA TGC AT-C(O)OCH₃ SEQ ID NO:141 synthesized as per standard methods and techniques, wherein the polyamine is one of the following:

**TABLE III**

| | |
|---|---|
| - 1,6 Diaminohexane | PNA Oligomer SEQ ID NO:142 |
| - Diethylenetriamine | PNA Oligomer SEQ ID NO:143 |
| - Triethylenetetramine | PNA Oligomer SEQ ID NO:144 |
| - Spermine | PNA Oligomer SEQ ID NO:145 |
| - Pentaethylenehexamine | PNA Oligomer SEQ ID NO:146 |

### A. Preparation of the PNA Oligomer

PNA oligomer SEQ ID NO:141 having the terminal carboxyl group protected as a methyl ester and having a free amino terminal group is prepared in accordance with Examples **26-28,** and standard protection protocols.

### B. Preparation of Polyamine Functionalized PNA Oligomer

The PNA oligomer from Example **95(A)** above is dissolved in freshly prepared NaHCO₃ buffer (0.2 M, pH 8.1) and treated with a solution of disuccinimidyl suberate (DSS) (approximately 5 mgs) dissolved in 150 ul of methyl sulfoxide (DMSO). The reaction mixture is left to react for 20 minutes at room temperature. The mixture is then passed over a Sephadex G-25 column (0.7 x 45 cm) to separate the activated PNA oligomer-DSS from the excess DSS. The PNA oligomer-DSS is then frozen immediately and lyophilized to dryness. A solution of polyamine in 0.33 M NaOAc (approximately 6 mg polyamine in 300 ul 0.33 M NaOAc, pH 5.2, final solution pH 6-8.0) is added to the dried PNA oligomer-DSS, and this mixture is allowed to react overnight at room temperature. The resulting polyamine-PNA oligomer conjugate is characterized by reverse phase HPLC and a 20% denaturing gel. Solvent A is 50 mM TEAA, solvent B is CH₃CN. The HPLC gradient is from 0-10 mins, 95% solvent A, 5% solvent B; linear increase to 40% solvent B in the next 50 minutes using a Water's Delta-Pak C-18 reverse phase column.

HPLC analysis shows progressively faster migration times for the larger amines.

### C. Preparation of Biotin Functionalized PNA Oligomer Polyamine Conjugate

To further characterize the PNA-oligomer polyamine conjugate, biotin is attached to the free amines made available by the polyamines attached in the above Examples. About 10 O.D. units (A₂₆₀) of PNA oligomers SEQ ID NO:142 and SEQ ID NO:114, (approximately 58 nmoles) are dried in a microfuge tube.

The PNA oligomer polyamine conjugate is rehydrated in 400 ul of 0.2 M NaHCO₃ (pH 8.1) buffer and D-biotin-N-hydroxysuccinimide ester (approximately 5.0 mgs biotin for the 1,6 Diaminohexane conjugate, 8.0 mgs for the diethylenetriamine) (Sigma) is added followed by 200 ul of DMF. The solution is left to react overnight at room temperature. The solution is then passed over a NAP-25 column and analyzed by reverse phase HPLC. Solvent A is 50 mM TEAA and solvent B is CH₃CN. The HPLC gradient wis 0-10 mins, 95% A, 5% B; linear increase to 40% B in the next 50 minutes using a Water's Delta-Pak C-18, reverse phase column.

### EXAMPLE 96

### Internal Polyamine Labeled PNA Oligomer: linkage to formyl functionalized monomer

### 1. N-[(N-Boc-1-formyl)-2-aminoethyl]-N-[(1-thyminyl)-acetyl]glycine ethyl ester (97A)

Compound **35** is converted to the ethyl ester by standard techniques. The resulting compound is then treated according to the procedure of Example **37** to yield the title compound.

### 2. N-[(N-Boc){1-(6-aminohexyl)aminomethyl}-2-aminoethyl]-N-[(1-thyminyl)-acetyl]glycine ethyl ester (97B)

Compound **97A** is dissolved in 100 microliters of water, and 100 ml of 1M NaOAc buffer (pH 5.0) is added followed by 1,6 diaminohexane hydrochloride (24 µmols) and 50 µl of 60 mM NaCNBH₃ solution. The solution is vortexed, left overnight and purified in an analytical HPLC column. The product is then deprotected at the carboxyl group to yield the title compound **(97B).**

### 3. Incorporation of N-[(N-Boc){1-(6-aminohexyl)aminomethyl}-2-aminoethyl]-N-[(1-thyminyl)-acetyl]glycine ethyl ester (97B) into PNA oligomers.

The free amino group of compound **97B** is Boc-protected using standard techniques. The protected monomer is then incorporated into PNA oligomers according to the procedure of Example **26**.

### EXAMPLE 97

### C-8 Purine and C-5 Pyrimidine conjugation

The C-8 position of purines and the C-5 position of pyrimidines are modified as per the procedures outlined in Ruth, J.L., Oligonucleotides with reporter groups attached to the base, in *Oligonucleotides and Analogues, A Practical Approach,* Eckstein, F., IRL Press, New York.

### A. N-(N-Boc-2-aminoethyl)-N-[(N-6-Cbz)(8-[N-(6-trifluoroacetylaminohexyl)-3- (E)acrylamido]-9-adeninyl)acetyl]glycine (Compound 97A).

Compound **97A** is synthesized using the procedures of the above reference. A purine substituted protected PNA monomer is used in place of a nucleoside. N-(N-Boc-2-aminoethyl)-N-[(6-Cbz-9-adeninyl)acetyl]glycine ethyl ester **(21)** is used as the starting material instead of adenosine. After the protected linking group is attached the resulting compound is treated via the procedures of Example 22 to give the title compound. The final compound having a protected linking moiety is further incorporated into oligomers using standard methods. The protected linking moiety is deprotected and functionalized with a further functional group e.g. fluorescein or biotin.

### B. N-(N-Boc-2-aminoethyl)-N-[(N-4-Cbz)(5-[N-(6-trifluoroacetylaminohexyl)-3-(E)acrylamido]-1-cytosinyl)-acetyl]glycine (Compound 97B).

Compound **97B** is synthesized using the procedures of the above reference except that Compound **16** is used as the starting material instead of cytidine. After the protected linking group is attached the resulting compound is treated via the procedures of Example **17**. The final compound having a protected linking moiety is further incorporated into oligomers using standard methods. The protected linking moiety is deprotected and functionalized with a further functional group e.g. fluorescein or biotin.

### EXAMPLE 98

### 5-Propynyl Pyrimidines (Cytosine and Uracil)

### A. N-(N-Boc-2-aminoethyl)-N-[(N-4-Cbz) (5-propynyl)-1-cytosinyl)acetyl]glycine (Compound 98A)

Compound **98A** is synthesized using the procedures of Wagner, W.W., *et.al., Science,* **1993,** *260,* 1510-1513, except that Compound **16** is used as the starting material instead of cytidine. After the 5-propynyl group is attached to the protected N-1-acetylcytosine the resulting compound is treated via the procedures of Example **17**. The final compound having a 5-propynyl group is further incorporated into oligomers using standard methods.

### B. N-(N-Boc-2-aminoethyl)-N-[(5-propynyl)-1-urasinyl)acetyl]glycine (Compound 98B)

Compound **98B** is synthesized analogously to Compound **98A** except that the protection step for the exocyclic amino group is omitted and uracil is substituted for cytosine in the applicapable examples e.g. Examples **13**-**17**. After the 5-propynyl group is attached to N-1-acetyluracil the resulting compound is treated via the procedures of Example **17**. The final compound having a 5-propynyl group is further incorporated into oligomers using standard methods.

### EXAMPLE 99

### N-Fmoc-3-Aminopropionic Acid

Sodium bicarbonate (2.52 g, 30 mmol) and 3-aminopropionic acid (1.00 g, 11.2 mmol) were dissolved in 50 ml water and 50 ml dioxane was added. A solution of fluorenylmethyl chloroformate (3.10 g, 12.0 mmol) in 50 ml dioxane was added dropwise with stirring. After 6 hours the solution was diluted with water (100 ml) and saturated bicarbonate solution (50 ml), extracted once with diethyl ether, and the aqueous layer acidified to pH 2 with concentrated HCl. The cloudy solution was extracted with ethyl acetate (2 X 100 ml), washed with brine and dried with MgSO₄. After evaporation a mixture of the title product and the peptide dimer was obtained. The pure product was obtained by flash chromatography. ¹H NMR: (CDCl₃, 200 MHz) δ 7.95-7.26 (8H, m, ArH), 7.40-7.15 (3H, m, C**H**C**H**₂O),3.20 (2H, t, J=8 Hz, C**H**₂N) , 2.40 (2H, t, J=8 Hz, HOOCC**H**₂).

### EXAMPLE 100

### N-(N-Boc-2-aminoethyl)-N-(N-Fmoc-3-aminopropionoyl)glycine (100)

This compound is prepared as per the procedures described in Examples **10** and **11** substituting N-Fmoc-3-aminopropionic Acid **(99)** in place of N-1-carboxymethyl thymine. The final compound is further incorporated into oligomers using standard methods.

### EXAMPLE 101

### N-Imidazolyl-2-Acetic acid

Imidazole (3.7 g, 54 mmol) was added to a suspension of sodium hydride (2.6 g of a 60% dispersion in oil, 60 mmol) in 50 ml dry THF. Bromoacetic acid (3.4 g, 24 mmol) was then added and the mixture stirred overnight. Water (1 ml) was then added and the solvent removed under reduced pressure. The residue was taken up in water (50 ml, pH >10), extracted with ether and the organic layer discarded. The aqueous layer was acidified to pH 1 with concentrated HCl and extracted again with ether. The aqueous layer was evaporated to dryness. The oily residue was dissolved in absolute ethanol (EtOH) to precipitate NaCl, and recrystallized from acetone/methanol to give 1.22 g (7.5 mmol, 30%) pure product as the hydrochloride. ¹H NMR: (DMSO-d6, 200 MHz) δ 9.20 (s, H2), 7.76 (d, J = 1.5 Hz), 7.69 (d, J = 1.5 Hz), 5.20 (s, CH₂).0

### EXAMPLE 102

### N-(N-Boc-2-aminoethyl)-N-(N-Imidazolyl-2-Acetyl)glycine (102)

This compound is prepared as per the procedures described in Examples **10** and **11** substituting N-imidazolyl-2-acetic acid **(101)** in place of N-1-carboxymethyl thymine. The final compound is further incorporated into oligomers using standard methods.

### EXAMPLE 103

### Benzyl 3,6,9,12-Tetraoxatridecanoate (103)

Triethyleneglycol monomethyl ether (10 mmol) and benzyl bromoacetate (11 mmol) are added to a suspension of anhydrous K₂CO₃ (15 mmol) in 50 ml anhydrous DMF. The suspension is stirred at room temperature overnight. Water is added and the emulsion is extracted with ethyl acetate (3 X 200 ml), washed with water, brine, and dried with MgSO₄. The solvent is evaporated and the residual oil purified by flash chromatography to give the title compound.

### EXAMPLE 104

### 3,6,9,12-Tetraoxatridecanoic Acid (104)

Benzyl-3,6,9,12-Tetraoxatridecanoate (**103**, 5 mmol) is dissolved in methanol (50 ml) and 10% palladium on carbon is added (100 mg catalyst/mmol). The suspension is shaken under 30 psi H₂ until the starting material is consumed. The suspension is filtered through a short pad of Celite, washed thoroughly with methanol, and the solvent evaporated. The product is used directly without purification.

### Example 105

### N-(N-Boc-2-aminoethyl)-N-(3,6,9,12-Tetraoxatridecanoyl)glycine (105)

This compound is prepared as per the procedures described in Examples **10** and **11** substituting 3,6,9,12-tetraoxatridecanoic Acid **(104)** in place of N-1-carboxymethyl thymine. The final compound is further incorporated into oligomers using standard methods.

### EXAMPLE 106

### N-α-(FMOC)-glutamic acid -γ-benzyl ester (106)

To a solution of γ-benzyl glutamate (10 mmol) in 50 ml dioxane and 50 ml water is added triethylamine (25 mmol), followed by a solution of fluorenylmethyl chloroformate (11 mmol) in 50 ml dioxane. The mixture is vigorously stirred until the starting material is consumed. The solution is acidified to pH 2 with concentrated HCl, extracted with ethyl acetate (2 X 250 ml), washed with brine, dried with MgSO₄ and evaporated. The product is used without purification.

### EXAMPLE 107

### N-α-(FMOC)-γ-benzyl-L-glutamic acid fluorenylmethyl ester (107)

N-α-(FMOC)-glutamic acid γ-benzyl ester (**106**, 5 mmol), fluorenylmethanol (5.5 mmol) and dimethylaminopyridine (0.5 mmol) are dissolved in 50 ml CH₂Cl₂. Dimethylaminopropyl ethyl carbodiimide (EDC, 6.0 mmol) is added, and the solution stirred at room temperature. After complete consumption of the starting material the solution is diluted with CH₂Cl₂, washed with 1% HCl, water and brine, dried with MgSO₄ and evaporated. The residue is purified by flash chromatography using ethyl acetate and hexane as eluant.

### EXAMPLE 108

### N-α-(FMOC)-L-glutamic acid α-fluorenylmethyl ester (108)

N-α-(FMOC)-γ-benzyl-L-glutamic acid fluorenylmethyl ester **(107,** 5 mmol) is dissolved in methanol (50 ml) and 10 % Palladium on carbon is added (100 mg catalyst/mmol). The suspension is shaken under 30 psi H₂ until the starting material is consumed. The suspension is filtered through a short pad of Celite, washed thoroughly with methanol, and the solvent evaporated. The product is used directly without purification.

### Example 109

### N-(N-Boc-2-aminoethyl)-N-[N-α-(FMOC) -L-glutamoyl-α-fluorenylmethyl ester]glycine (109)

This compound is prepared as per the procedures described in Examples **10** and **11** substituting N-α-(FMOC)-L-glutamic acid α-fluorenylmethyl ester **(108)** in place of N-1-carboxymethyl thymine. The final compound is further incorporated into the N-terminus of PNA oligomers using standard methods.

### EXAMPLE 110

### N- (N-Boc-2-aminoethyl)-N-(1-methyl-2-pyrroleacetyl)glycine (110)

This compound is prepared as per the procedures described in Examples **10** and **11** substituting 1-methyl-2-pyrrolecarboxylic acid (Aldrich) in place of N-1-carboxymethyl thymine. The final compound is further incorporated into oligomers using standard methods.

### EXAMPLE 111A

### Cleavage of Plasmid-DNA by NTA-Lys₂-T₂CT₂CT₄-Lys₂-NH₂

A mixture of 2.0µL of 5.9µM PNA NTA-Lys₂-T₂CT₂CT₄-Lys₂-NH₂ and 0.2µL of 5.9µM Fe²⁺ was incubated for 10 minutes at room temperature. Buffer (100 mM PO₄, pH 6.5 or 100 mM PO₄, pH 6.5, 10 mM EDTA), 9.5 µL H₂O and 0.5 µL ³²P-labeled target DNA, pA8G2 was added and the incubation was continued for 1 hour at 37 °C. 1µL of 40 mM DTT was added and the incubation was continued for 1 hour at 37 °C. Samples were analyzed on an 8% polyacrylamide sequencing gel according to the following scheme:

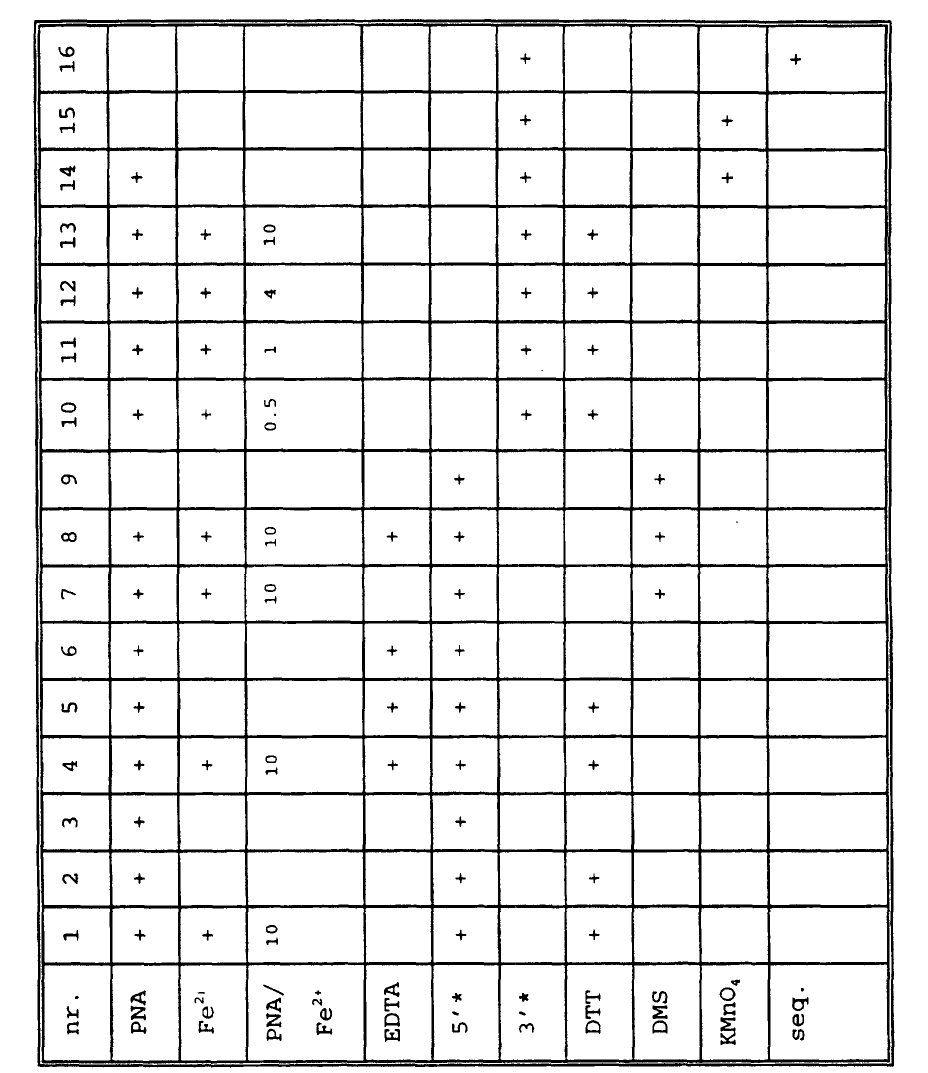

5'* denotes that the EcoR1/Pvu 11 fragment of pA8G2 was labeled at the 5'-end and 3'* denotes that this fragment was labeled at the 3'end. DMS denotes probing with dimethylsulphate and KMnO₄.

When the experimental results were analyzed by gel electrophoresis (see Figure 6) it was shown that PNA NTA Fe2⁺ was an effective oligonucleotide cleavage agent. A comparison of lane 1 to lane 4 where EDTA was employed to competitively bind available Fe2⁺ shows that the Fe2⁺-NTA complex was necessary and effective at cleaving the DNA strand. A comparison of lane 13 to lane 14 shows that the cleaving pattern has been extended beyond the PNA binding site as mapped by KMnO₄ cleavage of the strand displaced by the PNA, which is consistent with the Fe2⁺-NTA complex being oriented at that end.

### EXAMPLE 111B

### Cleavage of ³²P-Endlabeled DNA restriction fragment by NTA-PNA and NTA-PNA-Lys-AHA-Lys-AHA-Lys-PNA (bis PNA)

Example 111A was repeated using NTA-Lys₂-T₂CT₂CT₄-Lys₂-NH₂ and also with bis-PNA, NTA-Lys-TTT-TCT-TCT-T-Lys-AHA-Lys-AHA-Lys-TTC-TTC-TTT-T-Lys-NH₂. A ³²P-endlabeled, double stranded DNA restriction fragment containing a target AAAAGAAGAA region was used. The cleavage products were analyzed by high resolution polyacrylamide gel electrophoresis.

The results show that the NTA-PNA conjugate selectively cleaved the double stranded DNA target. Permanganate and DMS-protection probing (see, Cherny, D.Y., *et.al., Proc. Natl. Acad. Sci.,* **1993,** *90*, 1667), was performed to measure the binding of the PNA to the DNA target and these results (Figure 7, lanes 7, 8, and 14) show that the PNA binds to the target by PNA-DNA triplex strand displacement.

The cleavage of the restriction fragment was found to be proximal to the PNA binding site on the 3' and 5'-end for the NTA-Lys₂-T₂CT₂CT₄-Lys₂-NH₂ and only at the 5'-end for the bis-PNA, NTA-Lys-TTT-TCT-TCT-T-Lys-AHA-Lys-AHA-Lys-TTC-TTC-TTT-T-Lys-NH₂. These results are consistent with the triplex formation occurring with one strand binding by Watson and Crick and the other strand binding via Hoogsteen binding motifs.

### EXAMPLE 112

### Binding of PNA conjugates to double stranded DNA targets

A mixture of 200 cps ³²P-labeled *Eco*RI-*Pvu*II fragment (the large fragment labeled at the 3'-end of the *Eco*RI site) of the indicated plasmid, 0.5 µg carrier calf thymus DNA, and 300 ng PNA having an appropriate conjugate attached thereto in 100 µl buffer (10 mM Na-phosphate, 1mM EDTA pH 7) are incubated at 37°C for 60 min. 5 µl 20mM KMnO₄ is added and incubated at 20°C for 15 seconds. The reaction is stopped by addition of 3 µl 0.5 M 2-mercaptoethanol, 1M NaOAc and the DNA is precipitated by addition of 250 µl 2% potassium acetate in ethanol. The DNA is treated with hot (50 °C) 1M piperidine and is analyzed by electrophoresis in 10% polyacrylamide sequencing gels and the radiolabeled DNA bands visualized by autoradiography.

The target plasmids are prepared by cloning of the appropriate oligonucleotides into pUC19. Target A10: oligonucleotides GATCCA10G & GATCCT10G cloned into the BamHI site (plasmid designated pT10). Target A5GA4: oligonucleotides TCGACT4CT5G & TCGACA5GA4G cloned into the SalI site (plasmid pT9C). Target A2GA2GA4: oligonucleotides GA2GA2GA4TGCA & GT4CT2CT2CTGCA into the PstI site (plasmid pT8C2). The positions of the targets in the gel are indicated by bars to the left. A/G is an A+G sequence ladder of target P10.

### EXAMPLE 113

### A. Determination Of Cellular Uptake And Activity Of Thiol Linker Containing PNA Oligomers

This is determined by the inhibition of ICAM-1 (Intra Cellular Adhesion Molecule-1) utilizing the method of Chiang, *et al., J. Biol. Chem.* **1991,** *266 18162.*

**ICAM-1 Assay:** ICAM-1 expression on the cell surface is determined by ELISA using cells grown to confluence in 96-well plates. Cells are washed three times with Dulbecco's phosphate-buffered saline and fixed for 20 minutes in 2% formaldehyde diluted in Dulbecco's PBS. The cells are washed three times with Dulbecco's PBS, blocked for 1 hour at 37°C with 2% bovine serum albumin in Dulbecco's PBS, and incubated with ICAM-1 monoclonal antibody 84H10 (0.5 µg/ml) for 1 hour at 37°C. Antibody bound to the cells is determined by incubation with a 1:1000 dilution of biotinylated goat anti-mouse IgG followed by incubation with a 1:1000 dilution of β-galactosidase-conjugated streptavidin. The plates are developed with 100 µl of 3.3 mM chlorophenolred-β-D-galactopyranoside in 50 mM sodium phosphate, 1.5 mM MgCl₂, pH 7.0. Product formed is detected by absorbance at 575 nm. The data are expressed as percent control activity, which is calculated as described by Chiang, *et al.,* in the above reference.

**PNA Oligomer Treatment of Cells:** Cells are washed three times with Opti-MEM prewarmed to 37°C. Opti-MEM containing either 10 µg/ml DOTMA solution (HUVEC) or 20 mg/ml DOTMA solution (A549 cells) is added to each well of the plate (100 µl). PNA oligomers were sterilized by centrifugation through 0.2 µM Centrex cellulose acetate filters. PNA oligomers are added as 20 X stock solution to the wells and incubated for 4 hours at 37°C and then stimulated with the appropriate cytokine for 14-16 hours as indicated. ICAM-1 expression is determined as described above.

The following PNA oligomers having aminoethylglycine backbones are synthesized according to the procedures of the foregoing Examples: H₂N-TGG GA*G CCA TAG CGA GCC-COOH (SEQ ID NO:147) ICAM PNA; and H₂N-TCT GAG TAG CAG AGG AGC TA*A G (SEQ ID NO:148); a sequence in the 5'-cap region of ICAM. The astrisk (*) denotes the incorporation of a monomeric unit containing a protected thiol functionality according to Example **58**. PNA oligomer having SEQ ID NO:117 serves to evaluate the tritylthioether group in uptake experiments and to determine its ability to inhibit ICAM protein expression. PNA oligomer SEQ ID NO:118 is conjugated to *O*-phenanthroline and targeted against the 5'-cap-messenger RNA of the ICAM system to cleave the target RNA. The PNA oligomer is used in the above assay to assess its effect on ICAM expression.

### B. RNA Cleavage Assay Using Oligonucleotide Containing Thiol Linker

PNA oligomer SEQ ID NO:118 is thiol deprotected and conjugated to *O*-phenanthroline reagent. The conjugate is targeted against 5'-capped RNA of the ICAM system. The hybrid is incubated at 37°C over a 48 hour period in the presence of excess Cu(II) salt under buffered conditions. Analysis of the reaction by gel electrophoresis (as described by Baker, *J. Am. Chem. Soc.* **1993,** *115,* 3378) demonstrates that the oligonucleotide-O-phenanthroline-Cu complex cleaves the target RNA strand.

### EXAMPLE 114

### Inhibition of restriction enzyme cleavage by PNA-conjugate

A 2 µg portion of plasmid pT10 is mixed with the indicated amount of PNA conjugate in 20 µl TE buffer (10 mM Tris-HCl, 1 mM EDTA, pH 7.4) and incubated at 37°C for 120 min. 2 µl 10 × buffer (10 mM Tris-HCl, pH 7.5, 10 mM, MgCl₂, 50 mM NaCl, 1 mM DTT). *Pv*uII (2 units) and BamHI (2 units) are added and the incubation is continued for 60 min. The DNA is analyzed by gel electrophoresis in 5% polyacrylamide and the DNA is visualized by ethidium bromide staining.

### EXAMPLE 115

### Kinetics of PNA conjugate - dsDNA strand displacement complex formation

A mixture of 200 cps ³²P-labeled *Eco*RI-*Pvu*II fragment of pT10 (the large fragment labeled at the 3'-end of the *Eco*RI site), 0.5 µg carrier calf thymus DNA, and 300 ng of PNA conjugate in 100 µl buffer (200 mM NaCl, 50 mM Na-acetate, pH 4.5, 1 mM ZnSO₄) are incubated at 37°C. Periodically, 50 U of S₁ nuclease is added to each of 7 samples and incubation is continued for 5 min at 20°C. The DNA is then precipitated by addition of 250 µl 2% K-acetate in ethanol and analyzed by electrophoresis in a 10% polyacrylamide sequencing gel. The amount of strand displacement complex is calculated from the intensity of the S₁-cleavage at the target sequence, as measured by densitometric scanning of autoradiographs.

### EXAMPLE 116

### Stability of PNA-dsDNA complexes

A mixture of 200 cps ³²P-pT10 fragment, 0.5 µg calf thymus DNA and 300 ng of the desired PNA conjugate is incubated in 100 µl 200 mM NaCl, 50 mM Na-acetate, pH 4.5, 1 mM ZnSO₄ for 60 min at 37°C. A 2 µg portion of oligonucleotide GATCCA₁₀G is added and each sample is heated for 10 min at the temperature indicated, cooled in ice for 10 min and warmed to 20°C. A 50 U portion of S₁ nuclease is added and the samples treated and analyzed and the results quantified.

### EXAMPLE 117

### Inhibition of Transcription by PNA

A mixture of 100 ng plasmid DNA (cleaved with restriction enzyme *Pv*uII (see below) and 100 ng of PNA conjugate in 15 µl 10 mM Tris-HCl, 1 mM EDTA, pH 7.4 is incubated at 37°C for 60 min. Subsequently, 4 µl 5 x concentrated buffer (0.2 M Tris-HCl (pH 8.0), 40 mM MgCl₂, 10 mM spermidine, 125 mM NaCl) are mixed with 1 µl NTP-mix (10 mM ATP, 10 mM CTP, 10 mM GTP, 1 mM UTP, 0.1 µCi/µl ³²P-UTP, 5 mM DTT, 2 µg/ml tRNA, 1 µg/ml heparin) and 3 units RNA polymerase. Incubation is continued for 10 min at 37°C. The RNA is then precipitated by addition of 60 µl 2% potassium acetate in 96% ethanol at -20°C and analyzed by electrophoresis in 8% polyacrylamide sequencing gels. RNA transcripts are visualized by autoradiography. The following plasmids are used: pT8C2-KS/pA8G2-KS: oligonucleotides GA₂GA₂GA₄GTGAC & GT₄CT₂CT₂CTGCA cloned into the *PstI* site of pBluescript-KS⁺; pT10-KS/pA10-KS (both orientations of the insert were obtained). pT10UV5: oligonucleotides GATCCA₁₀G & GATCCT₁₀G cloned into the *Bam*HI site of a pUC18 derivative in which the lac UV5 *E.coli* promoter had been cloned into the *Eco*RI site (Jeppesen, *et al., Nucleic Acids Res.,* **1988**, *16*, 9545).

### EXAMPLE 118

### Biological stability of PNA

A mixture of PNA conjugate (10 µg) and a control, "normal" peptide (10 µg) in 40 µl 50 mM Tris-HCl, pH 7.4 is treated with varying amounts of peptidase from porcine intestinal mucosa or protease from Streptomyces caespitosus for 10 min at 37°C. The amount of PNA and peptide is determined by HPLC analysis (reversed phase C-18 column: 0-60% acetonitrile, 0.1% trifluoroacetic acid or other appropriate solvent system).

### EXAMPLE 119

### Inhibition of Gene Expression

A preferred assay to test the ability of peptide nucleic acids to inhibit expression of the E2 mRNA of papillomavirus is based on the well-documented transactivation properties of E2. Spalholtz, *et al.*, *J. Virol.,* **1987**, 61, 2128-2137. A reporter plasmid (E2RECAT) is constructed to contain the E2 responsive element, which functions as an E2 dependent enhancer. E2RECAT also contains the SV40 early promoter, an early polyadenylation signal, and the chloramphenicol acetyl transferase gene (CAT). Within the context of this plasmid, CAT expression is dependent upon expression of E2. The dependence of CAT expression on the presence of E2 is tested by transfection of this plasmid into C127 cells transformed by BPV-1, uninfected C127 cells and C127 cells cotransfected with E2RECAT and an E2 expression vector.

### A. Inhibition of BPV-1 E2 Expression

BPV-1 transformed C127 cells are plated in 12 well plates. Twenty four hours prior to transfection with E2RE1, cells are pretreated by addition of complementary PNA conjugates to the growth medium at final concentrations of 5, 15 and 30 mM. The next day cells are transfected with 10 µg of E2RE1CAT by calcium phosphate precipitation. Ten micrograms of E2RE1CAT and 10 µg of carrier DNA (PUC 19) are mixed with 62 µl of 2 M CaCl₂ in a final volume of 250 µl of H₂O, followed by addition of 250 µl of 2X HBSP (1.5 mM Na₂PO₂. 10 mM KCl, 280 mM NaCl, 12 mM glucose and 50 mM HEPES, pH 7.0) and incubated at room temperature for 30 minutes. One hundred microliters of this solution is added to each test well and allowed to incubate for 4 hours at 37°C. After incubation, cells are glycerol shocked for 1 minute at room temperature with 15% glycerol in 0.75 mM Na₂PO₂, 5 mM KCl, 140 mM NaCl 6 mM glucose and 25 mM HEPES, pH 7.0. After shocking, cells are washed 2 times with serum free DMEM and refed with DMEM containing 10% fetal bovine serum and oligonucleotide at the original concentration. Forty eight hours after transfection cells are harvested and assayed for CAT activity.

For determination of CAT activity, cells are washed 2 times with phosphate buffered saline and collected by scraping. Cells are resuspended in 100 µl of 250 mM Tris-HCl, pH 8.0 and disrupted by freeze-thawing 3 times. Twenty four microliters of cell extract is used for each assay. For each assay the following are mixed together in an 1.5 ml Eppendorf tube and incubated at 37°C for one hour: 25 µl of cell extract, 5 µl of 4 mM acetyl coenzyme A, 18 µl H₂O and 1 µl ¹⁴C-chloramphenicol, 40-60 mCi/mM. After incubation, chloramphenicol (acetylated and nonacetylated forms) is extracted with ethyl acetate and evaporated to dryness. Samples are resuspended in 25 µl of ethyl acetate, spotted onto a TLC plate and chromatographed in chloroform:methanol (19:1). Chromatographs are analyzed by autoradiography. Spots corresponding to acetylated and nonacetylated ¹⁴C-chloramphenicol are excised from the TLC plate and counted by liquid scintillation for quantitation of CAT activity. Peptide nucleic acids that depress CAT activity in a dose dependent fashion are considered positives.

### B. Inhibition of HPV E2 Expression

The assay for inhibition of human papillomavirus (HPV) E2 by peptide nucleic acids is essentially the same as that for BPV-1 E2. For HPV assays appropriate HPVs are cotransfected into either CV-1 or A431 cells with PSV2NEO using the calcium phosphate method described above. Cells which take up DNA are selected for by culturing in media containing the antibiotic G418. G418-resistant cells are then analyzed for HPV DNA and RNA. Cells expressing E2 are used as target cells for complementary studies. For each PNA, cells are pretreated as above, transfected with E2RE1CAT, and analyzed for CAT activity as above. Peptide nucleic acid conjugates are considered to have a positive effect if they can depress CAT activity in a dose dependent fashion.

### Example 120

### Iodination Procedure

A 5 µg portion of a PNA having a conjugate attached thereto is dissolved in 40 µl 100 mM Na-phosphate, pH 7.0, and 1 mCi Na¹²⁵I and 2 µl chloramine-T (50 mM in CH₃CN) are added. The solution is left at 20°C for 10 min and then passed through a 0.5 + 5 cm Sephadex G10 column. The first 2 fractions (100 µl each) containing radioactivity are collected and purified by HPLC: reversed phase C-18 using a 0-60% CH₃CN gradient in 0.1% CF₃COOH in H₂O, or other suitable solvent system. The ¹²⁵I-PNA elutes right after the PNA peak. The solvent is removed under reduced pressure.

### EXAMPLE 121

### Detection of mutant β-amyloid precursor protein gene expression (βAPP)

Point mutations in the gene encoding β-amyloid have been implicated in familial Alzheimer's disease (FAD). PNA oligomers are labeled after synthesis with fluorescein or other fluorescent tag as illustrated above. Alternatively,oligomers can be labeled with other reporter molecules before or after oligomer synthesis. Labeled PNA oligomers are contacted with tissue or cell samples suspected of abnormal βAPP expression under conditions in which specific hybridization can occur, and the sample is washed to remove unbound PNA oligomers. Label remaining in the sample indicates bound oligonucleotide and is quantitated using a fluorimeter, fluorescence microscope or other routine means.

Tissue or cell samples suspected of expressing a point mutation in the βAPP gene are incubated with a fluorescein-labeled PNA oligomer which is targeted to the mutant codon 717, codon 670 or codon 671 of βAPP mRNA. An identical sample of cells or tissues is incubated with a second labeled PNA oligomer which is targeted to the same region of normal βAPP mRNA, under conditions in which specific hybridization can occur, and the sample is washed to remove unbound PNA oligomer. Label remaining in the sample indicates bound PNA oligomer and can be quantitated using a fluorimeter or other routine means. The presence of mutant βAPP is indicated if the first sample binds labeled PNA oligomer and the second sample does not bind fluorescent label.

Double labeling can also be used with PNA oligomers and methods of the invention to specifically detect expression of mutant βAPP. A single tissue sample is incubated with a rhodamine-labeled PNA oligomer which is targeted to codon 717, codon 670 or codon 671 of mutant βAPP mRNA and a fluorescein-labeled PNA oligomer which is targeted to the translation initiation site of mutant βAPP mRNA, under conditions in which specific hybridization can occur. The sample is washed to remove unbound PNA oligomer and labels are detected by and fluorimetry with appropriate filters. The presence of mutant βAPP is indicated if the sample does not bind rhodamine-labeled PNA oligomer but does retain the fluorescein label.

### EXAMPLE 122

### Detection of mutant H-ras gene expression

Point mutations in the H-ras gene have been implicated in numerous aberrations of the Ras pathway. PNA oligomers are labeled after synthesis with fluorescein or other fluorescent tag as illustrated above. Alternatively, oligomers can be labeled with other reporter molecules before or after oligomer synthesis. Labeled PNA oligomers are contacted with tissue or cell samples suspected of abnormal ras expression under conditions in which specific hybridization can occur, and the sample is washed to remove unbound PNA oligomer. Label remaining in the sample indicates bound PNA oligomer and is quantitated using a fluorimeter, fluorescence microscope or other routine means.

Tissue or cell samples suspected of expressing a point mutation in the H-ras gene are incubated with a fluorescein-labeled PNA oligomer which is targeted to the mutant codon 12, codon 13 or codon 61 of H-ras mRNA. An identical sample of cells or tissues is incubated with a second labeled PNA oligomer which is targeted to the same region of normal H-ras mRNA, under conditions in which specific hybridization can occur, and the sample is washed to remove unbound PNA oligomer. Label remaining in the sample indicates bound PNA oligomer and can be quantitated using a fluorimeter or other routine means. The presence of mutant H-ras is indicated if the first sample binds labeled PNA oligomer and the second sample does not bind fluorescent label.

Double labeling can also be used with PNA oligomers and methods of the invention to specifically detect expression of mutant ras. A single tissue sample is incubated with a rhodamine-labeled PNA oligomer which is targeted to codon 12, codon 13 or codon 61 of mutant H-ras mRNA and a fluorescein-labeled PNA oligomer which is targeted to the translation initiation site of ras mRNA, under conditions in which specific hybridization can occur. The sample is washed to remove unbound PNA oligomer and labels are detected by and fluorimetry with appropriate filters. The presence of mutant ras is indicated if the sample does not bind rhodamine-labeled PNA oligomer but does retain the fluorescein label.

## Claims

1. A peptide nucleic acid conjugate comprising:
a peptide nucleic acid;
said peptide nucleic acid having a backbone;
said backbone having an amino end, a carboxyl end, and a plurality of amino groups;
said amino groups each having a tethered nucleobase; and a conjugate bound to said peptide nucleic acid either directly or through a linking moiety, wherein said conjugate is a terpene, an aromatic lipophilic molecule, a phospholipid, a cell receptor binding molecule, a crosslinking agent, a water soluble vitamin, a lipid soluble vitamin, an RNA/DNA cleaving complex, a porphyrin, or a polymeric compound selected from polymeric amines, polymeric glycols and polyethers, provided that the conjugate is not acridine.

2. A peptide nucleic acid conjugate of claim 1, wherein said conjugate is bound through said linking moiety to at least one of said backbone, said tether, or said nucleobase.

3. A peptide nucleic acid conjugate of claim 1, wherein said conjugate is bound to said backbone, said nucleobase or said tether.

4. A peptide nucleic acid conjugate of claim 3, wherein said conjugate is bound to at least one of said amino end or said carboxyl end of said backbone.

5. A peptide nucleic acid conjugate of the formula: 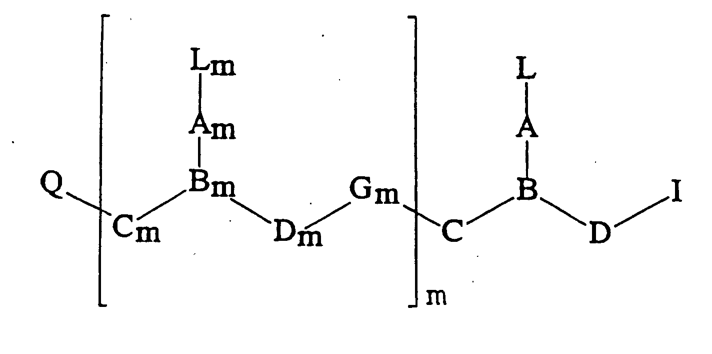 wherein:
m is an ineteger from 1 to about 50;
L and Lₘ independently are R¹²(R¹³)ₐ; wherein:
R¹² is hydrogen, hydroxy, (C₁-C₄)alkanoyl, a naturally occurring nucleobase, a non-naturally occurring nucleobase, an aromatic moiety, a DNA intercalator, a nucleobase-binding group, a heterocyclic moiety, a reporter ligand, or a conjugate;
provided that at least one of R¹² is a naturally occurring nucleobase, a non-naturally occurring nucleobase, a DNA intercalator, or a nucleobase-binding group;
R¹³ is a conjugate; and
a is 0 or 1;
C and Cₘ independently are (CR⁶R⁷)_{y}; wherein:
R⁶ and R⁷ independently are hydrogen, a side chain of a naturally occurring alpha amino acid, (C₂-C₆) alkyl, aryl, aralkyl, heteroaryl, hydroxy, (C₁-C₆) alkoxy, (C₁-C₆) alkylthio, a conjugate, NR³R⁴, SR⁵ or R⁶ and R⁷ taken together complete an alicyclic or heterocyclic system;
wherein R⁵ is hydrogen, a conjugate, (C₁-C₆)alkyl, hydroxy-, alkoxy-, or alkylthio-substituted (C₁-C₆)alkyl; and R³ and R⁴ independently are hydrogen, a conjugate, (C₁-C₄)alkyl, hydroxy- or alkoxy- or alkylthio-substituted (C₁-C₄)alkyl, hydroxy, alkoxy, alkylthio or amino;
D and Dₘ independently are (CR⁶R⁷)_{z};
each of y and z is zero or an integer from 1 to 10, wherein the sum y + z is greater than 2 but not more than 10;
Gₘ is independently -NR³CO-, -NR³CS-, -NR³SO-, or -NR³SO₂- in either orientation;
each pair of A-Aₘ and B-Bₘ are selected such that:
(a) A or Aₘ is a group of formula (IIa), (IIb) or (IIc) and B or Bₘ is N or R³N⁺; or
(b) A or Aₘ is a group of formula (IId) and B or Bₘ is CH; 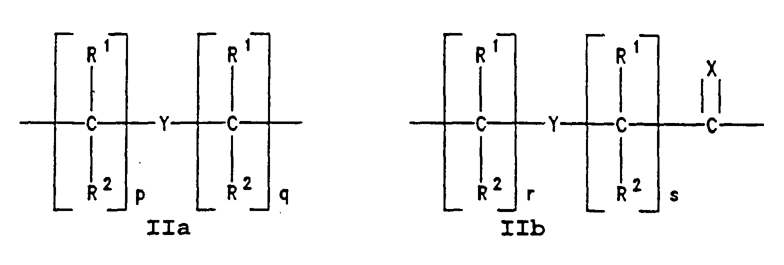 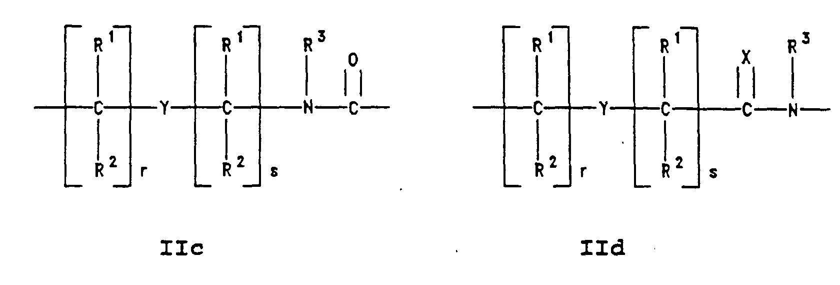 wherein:
X is O, S, Se, NR³, CH₂ or C(CH₃)₂;
Y is a single bond, O, S or NR⁴;
each of p and q is zero or an integer from 1 to 5, the sum p+q being not more than 10;
each of r and s is zero or an integer from 1 to 5, the sum r+s being not more than 10;
R¹ and R² independently are hydrogen, (C₁-C₄) alkyl, hydroxy-substituted (C₁-C₄)alkyl, alkoxy-substituted (C₁-C₄)alkyl, alkylthio-substituted (C₁-C₄)alkyl, hydroxy, alkoxy, alkylthio, amino, halogen or a conjugate;
I is -NR⁸R⁹ or -NR¹⁰C(O)R¹¹; wherein:
R⁸, R⁹, R¹⁰ and R¹¹ independently are hydrogen, alkyl, an amino protecting group, a reporter ligand, an intercalator, a chelator, a peptide, a protein, a carbohydrate, a lipid, a steroid, a nucleoside, a nucleotide, a nucleotide diphosphate, a nucleotide triphosphate, an oligonucleotide, an oligonucleoside, a soluble polymer, a non-soluble polymer or a conjugate;
Q is -CO₂H, -CO₂R⁸, -CO₂R⁹, -CONR⁸R⁹, -SO₃H, -SO₂NR¹⁰R¹¹ or an activated derivative of -CO₂H or -SO₃H; and
wherein at least one of R¹, R², R³, R⁴, R⁵, R⁶, R⁷, R⁶, R⁹, R¹⁰, R¹¹, R¹² and R¹³ is a conjugate wherein said conjugate is a terpene, an aromatic lipophilic molecule, a phospholipid, a cell receptor binding molecule, a crosslinking agent, a water soluble vitamin, a lipid soluble vitamin, an RNA/DNA cleaving complex, a porphyrin, or a polymeric compound selected from polymeric amines, polymeric glycols and polyethers; and
wherein said conjugate optionally includes a linking moiety, provided that the conjugate is not acridine.

6. A peptide nucleic acid conjugate of claim 5, wherein at least one group R¹² or one group R¹³ is a conjugate.

7. A peptide nucleic acid conjugate of claim 5 or claim 6, wherein at least one of R¹, R², R³, R⁴, R⁵, R⁶, R⁷, R⁸, R⁹, R¹⁰ or R¹¹ is a conjugate.

8. A peptide nucleic acid conjugate of claim 7, wherein at least one of said A-Aₘ groups include at least one of R¹, R², and R³.

9. A peptide nucleic acid conjugate of claim 7, wherein at least one of said B-Bₘ groups or said G-Gₘ groups include at least one group R³.

10. A peptide nucleic acid conjugate of claim 7, wherein at least one of said groups Q or I include at least one of groups R⁸, R⁹, R¹⁰ and R¹¹.

11. A peptide nucleic acid conjugate of claim 7, wherein at least one of said groups D-Dₘ, or C-Cₘ include at least one of R³, R⁴, R⁵, R⁶ and R⁷.

12. A peptide nucleic acid conjugate of any one of claim 5 to 11, wherein:
(i) m is from 1 to about 200, or
(ii) m is from 1 to about 50, or
(iii) m is from 1 to about 20.

13. A compound having one of the following formulas: 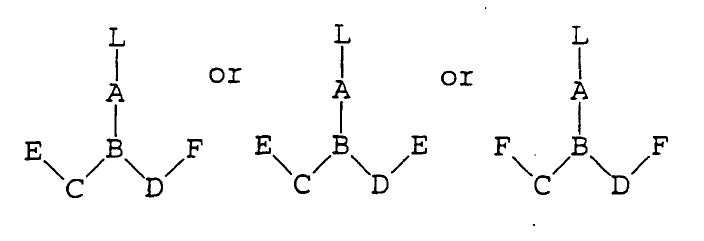 wherein:
L is R¹²(R¹³)ₐ; wherein:
R¹² is hydrogen, hydroxy, (C₁-C₄)alkanoyl, a naturally occurring nucleobase, a non-naturally occurring nucleobase, an aromatic moiety, a DNA intercalator, a nucleobase-binding group, a heterocyclic moiety, a reporter ligand, or a conjugate and at least one of R¹² is a naturally occurring nucleobase, a non-naturally occurring nucleobase, a DNA intercalator, or a nucleobase-binding group;
R¹³ is a conjugate; and
a is 0 or 1;
A and B are selected such that:
(a) A is a group of formula (IIa), (IIb) or (IIc) and B is N or R³N⁺; or
(b) A is a group of formula (IId) and B is CH; 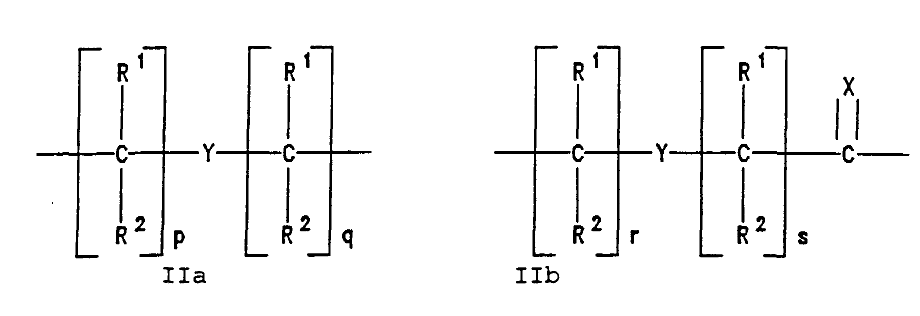 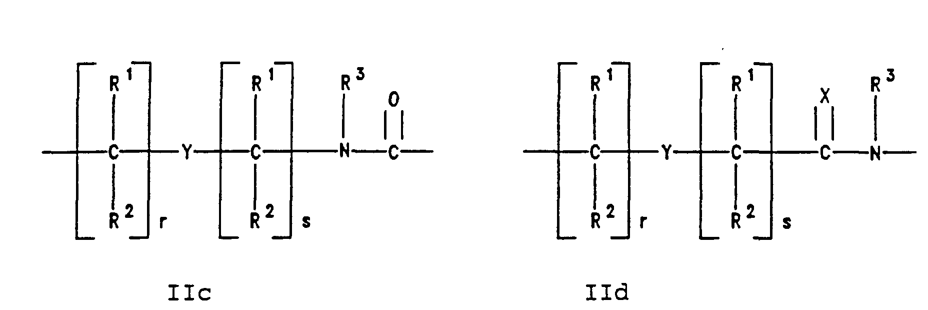 where:
X is O, S, Se, NR³, CH₂ or C(CH₃)₂;
Y is a single bond, O, S or NR⁴;
p and q independently are zero or an integer from 1 to 5, the sum p+q being not more than 10;
r and s independently are zero or an integer from 1 to 5, the sum r+s being not more than 10;
R¹ and R² independently are hydrogen, (C₁-C₄)alkyl, hydroxy-substituted (C₁-C₄)alkyl, alkoxy-substituted (C₁-C₄)alkyl, alkylthio-substituted (C₁-C₄)alkyl, hydroxy, alkoxy, alkylthio, amino, halogen or a conjugate;
C is (CR⁶R⁷)_{y};
D is (CR⁶R⁷)_{z}; wherein:
R⁶ and R⁷ independently are hydrogen, a side chain of a naturally occurring alpha amino acid, (C₂-C₆) alkyl, aryl, aralkyl, heteroaryl, hydroxy, (C₁-C₆) alkoxy, (C₁-C₆) alkylthio, a conjugate, NR³R⁴ and SR⁵ or R⁶ and R⁷ taken together complete an alicyclic or heterocyclic system;
R³ and R⁴ independently are hydrogen, a conjugate, (C₁-C₄)alkyl, hydroxy- or alkoxy- or alkylthio-substituted (C₁-C₄) alkyl, hydroxy, alkoxy, alkylthio or amino; and
R⁵ is hydrogen, a conjugate, (C₁-C₆) alkyl, hydroxy, alkoxy-, or alkylthio- substituted (C₁-C₆)alkyl;
each of y and z is zero or an integer from 1 to 10, the sum y + z being greater than 2 but not more than 10;
E independently is COOH, CSOH, SOOH, SO₂OH or an activated or protected derivative thereof;
F independently is NHR³ or NPgR³, where Pg is an amino protecting group; and
at least one of R¹, R², R³, R⁴, R⁵, R⁶, R⁷, R¹², and R¹³ is a conjugate wherein said conjugate is a terpene, an aromatic lipophilic molecule, a phospholipid, a cell receptor binding molecule, a crosslinking agent, a water soluble vitamin, a lipid soluble vitamin, an RNA/DNA cleaving complex, a porphyrin, or a polymeric compound selected from polymeric amines, polymeric glycols and polyethers; and wherein said conjugate optionally inlcludes a linking moiety, provided that the conjugate is not acridine.

14. A peptide nucleic acid conjugate of claim 13, wherein at least one group R³ is a conjugate.

15. A peptide nucleic acid conjugate of claim 13 or claim 14, wherein at least one of said groups A or said groups B include a conjugate.

16. A peptide nucleic acid conjugate of any one of claims 13 to 15, wherein at least one of group R¹ or group R² is a conjugate.

17. A peptide nucleic acid conjugate of any one of claims 13 to 16, wherein at least one of said groups C or said groups D include a conjugate.

18. A peptide nucleic acid conjugate comprising a plurality of PNA monomers wherein at least one of said PNA monomers has the formula: 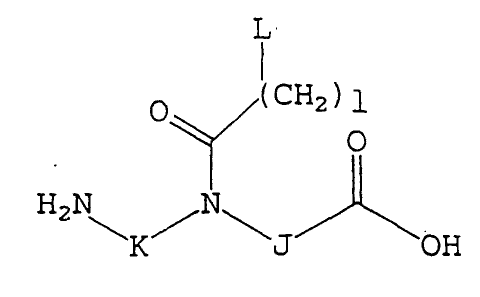 or formula: 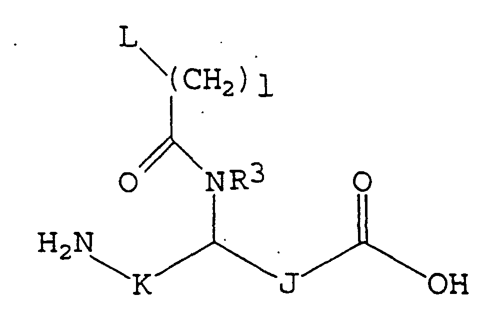 or formula: 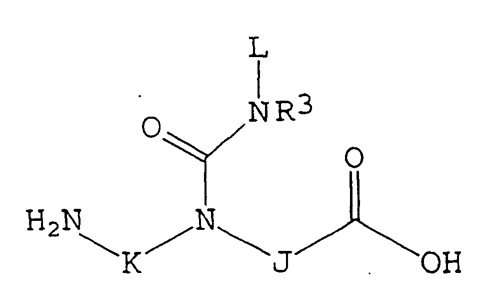 wherein:
L is R¹²(R¹³)ₐ; wherein:
R¹² is hydrogen, hydroxy, (C₁-C₄)alkanoyl, a naturally occurring nucleobase, a non-naturally occurring nucleobase, an aromatic moiety, a DNA intercalator, a nucleobase-binding group, a heterocyclic moiety, a reporter ligand, or a conjugate and at least one of R¹² is a naturally occurring nucleobase, a non-naturally occurring nucleobase, a DNA intercalator, or a nucleobase-binding group;
R¹³ is a conjugate; and
a is 0 or 1;
K is (CR⁶R⁷)_{z};
J is (CR⁶R⁷)_{y}; wherein:
R⁶ and R⁷ are independently hydrogen, a side chain of a naturally occurring alpha amino acid, (C₂-C₆) alkyl, aryl, aralkyl, heteroaryl, hydroxy, (C₁-C₆) alkoxy, (C₁-C₆) alkylthio, a conjugate, NR³R⁴ and SR⁵ or R⁶ and R⁷ taken together complete an alicyclic or heterocyclic system;
R³ and R⁴ independently are hydrogen, a conjugate, (C₁-C₄)alkyl, hydroxy- or alkoxy- or alkylthio-substituted (C₁-C₄)alkyl, hydroxy, alkoxy, alkylthio or amino;
R⁵ is hydrogen, a conjugate, (C₁-C₆)alkyl, hydroxy, alkoxy-, or alkylthio- substituted (C₁-C₆)alkyl;
each of y and z is zero or an integer from 1 to 10, the sum y + z being greater than 2 but not more than 10;
l is an integer from 1 to 5; and
at least one of R¹, R², R³, R⁴, R⁵, R⁶, R⁷, R¹², and R¹³ is a conjugate wherein said conjugate is a terpene, an aromatic lipophilic molecule, a phospholipid, a cell receptor binding molecule, a crosslinking agent, a water soluble vitamin, a lipid soluble vitamin, an RNA/DNA cleaving complex, a porphyrin, or a polymeric compound selected from polymeric amines, polymeric glycols and polyethers; and wherein said conjugate optionally includes a linking moiety, provided that the conjugate is not acridine.

19. A peptide nucleic acid conjugate of claim 18, wherein at least one of said group K or said group J includes a conjugate.

20. A peptide nucleic acid conjugate of any one of claims 13 to 19, wherein R¹² or R¹³ is a conjugate.

21. A peptide nucleic acid conjugate of any one of claims 13 to 20, wherein at least one of R³, R⁴, R⁵, R⁶, and R⁷ is a conjugate.

## Patentansprüche

1. Peptid-Nukleinsäure-Konjugat, umfassend:
eine Peptidnukleinsäure; wobei die Peptid-Nukleinsäure ein Rückgrat aufweist;
wobei das Rückgrat ein Aminoende, ein Carboxylende und eine Vielzahl von Aminogruppen aufweist;
wobei die Aminogruppen jeweils eine daran gebundene Nukleobase aufweisen; und ein Konjugat, das an die Peptid-Nukleinsäure entweder direkt oder über eine verknüpfende Einheit gebunden ist, wobei das Konjugat ein Terpen, ein aromatisches lipophiles Molekül, ein Phospholipid, ein Zellrezeptor-bindendes Molekül, ein Vernetzungsmittel, ein wasserlösliches Vitamin, ein Lipid-lösliches Vitamin, ein RNA/DNA-spaltender Komplex, ein Porphyrin, oder eine polymere Verbindung, ausgewählt aus polymeren Aminen, polymeren Glykolen und Polyethern, ist, mit der Maßgabe, daß das Konjugat nicht Acridin ist.

2. Peptid-Nukleinsäure-Konjugat nach Anspruch 1, wobei das Konjugat über die verknüpfende Einheit an mindestens eines von dem Rückgrat, der Anbindung oder der Nukleobase gebunden ist.

3. Peptid-Nukleinsäure-Konjugat nach Anspruch 1, wobei das Konjugat an das Rückgrat, die Nukleobase oder die Anbindung gebunden ist.

4. Peptid-Nukleinsäure-Konjugat nach Anspruch 3, wobei das Konjugat an mindestens eines von dem Aminoende oder dem Carboxylende des Rückgrats verbunden ist.

5. Peptid-Nukleinsäure-Konjugat der Formel: 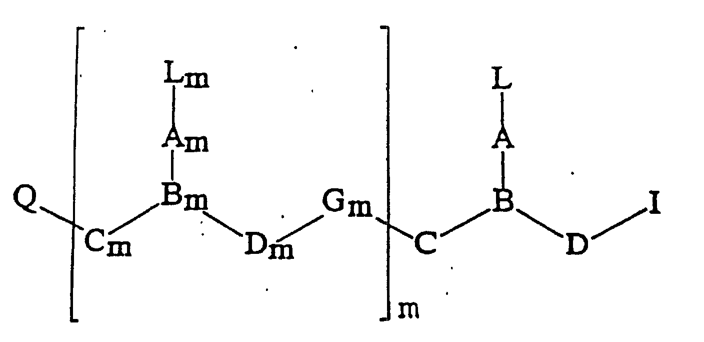
m eine ganze Zahl von 1 bis etwa 50 ist;
L und Lm unabhängig voneinander R¹² (R¹³)ₐ sind, worin:
R¹² Wasserstoff, Hydroxy, (C₁-C₄)Alkanoyl, eine natürlich vorkommende Nukleobase, eine nicht-natürlich vorkommende Nukleobase, eine aromatische Einheit, ein DNA-Interkalator, eine Nukleobase-bindende Gruppe, eine heterocyclische Einheit, ein Reporterligand oder ein Konjugat ist, mit der Maßgabe, daß mindestens eines von R¹² eine natürlich vorkommende Nukleobase, eine nicht-natürlich vorkommende Nukleobase, ein DANN-Interkalator oder eine Nukleobase-bindende Gruppe ist;
R¹³ ein Konjugat ist; und
a 0 oder 1 ist;
C und Cₘ unabhängig voneinander (CR⁶R⁷)_{y} sind, worin:
R⁶ und R⁷ unabhängig voneinander Wasserstoff, eine Seitenkette einer natürlich vorkommenden Alphaaminosäure, (C₂-C₆)-Alkyl, Aryl, Aralkyl, Heteroaryl, Hydroxy, (C₁-C₆)Alkoxy, (C₁-C₆)Alkylthio, ein Konjugat, NR³R⁴, SR⁵ sind oder R⁶ und R⁷ zusammengenommen ein alicyclisches oder heterocyclisches System vervollständigen;
worin R⁵ Wasserstoff, ein Konjugat, (C₁-C₆)Alkyl, Hydroxy-, Alkoxy- oder Alkylthio-substituiertes (C₁-C₆)Alkyl ist; und
R³ und R⁴ unabhängig voneinander Wasserstoff, ein Konjugat, (C₁-C₄)Alkyl, Hydroxy- oder Alkoxy- oder Alkylthio- substituiertes (C₁-C₄)Alkyl, Hydroxy, Alkoxy, Alkylthio oder Amino sind;
D und Dₘ unabhängig voneinander (CR⁶R⁷)_{z} sind;
y und z jeweils 0 oder eine ganze Zahl von 1 bis 10 sind, wobei die Summe y + z größer als 2, aber nicht mehr als 10 beträgt;
Gₘ unabhängig voneinander -NR³CO-, -NR³CS-, -NR³SO- oder -NR³SO₂in beiden Orientierungen ist;
jedes Paar von A-Aₘ und B-Bₘ derart ausgewählt sind, daß:
(a) A oder Aₘ eine Gruppe der Formel (IIa), (IIb) oder (IIc) ist und B oder Bₘ N oder R³N⁺ ist; oder
(b) A oder Aₘ eine Gruppe der Formel (IId) ist und B oder Bₘ CH ist; 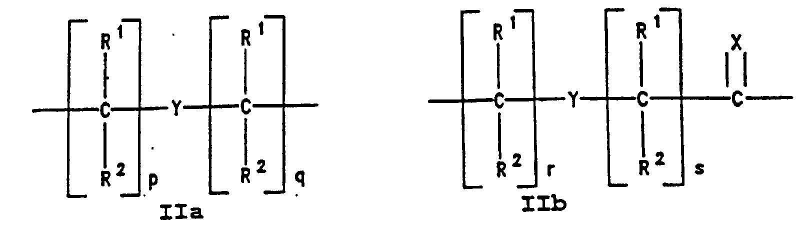 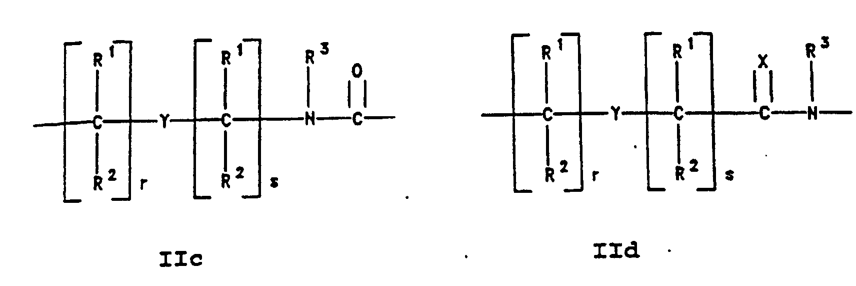 worin
X O, S, Se, NR³, CH₂ oder C(CH₃)₂ ist;
Y eine Einfachbindung, O, S oder NR⁴ ist;
p und q jeweils 0 oder eine ganze Zahl von 1 bis 5 sind, wobei die Summe p+q nicht mehr als 10 beträgt;
r und s jeweils 0 oder eine ganze Zahl von 1 bis 5 sind, wobei die Summe r+s nicht mehr als 10 beträgt,
R¹ und R² unabhängig voneinander Wasserstoff, (C₁-C₄)Alkyl, Hydroxysubstituiertes (C₁-C₄)Alkyl, Alkoxy-substituiertes (C₁-C₄)Alkyl, Alkylthio-substituiertes (C₁-C₄)Alkyl, Hydroxy, Alkoxy, Alkylthio, Amino, Halogen oder ein Konjugat sind;
I-NR⁸R⁹ oder -NR¹⁰C(O)R¹¹ ist, worin
R⁸, R⁹, R¹⁰ und R¹¹ unabhängig voneinander Wasserstoff, Alkyl, eine Aminoschutzgruppe, ein Reporterligand, ein Interkalator, ein Chelator, ein Peptid, ein Protein, ein Kohlenhydrat, ein Lipid, ein Steroid, ein Nukleosid, ein Nukleotid, ein Nukleotiddiphosphat, ein Nukleotidtriphosphat, ein Oligonukleotid, ein Oligonukleosid, ein lösliches Polymer, ein nicht-lösliches Polymer oder ein Konjugat sind;
Q -CO₂H, -CO₂R⁸, -CO₂R⁹, -CONR⁸R⁹, -SO₃H, -SO₂NR¹⁰R¹¹ oder ein aktiviertes Derivat von -CO₂H oder -SO₃H ist; und
worin mindestens eines von R¹, R², R³, R⁴, R⁵, R⁶, R⁷, R⁸, R⁹, R¹⁰, R¹¹, R¹² und R¹³ ein Konjugat ist, wobei das Konjugat ein Terpen, ein aromatisches lipophiles Molekül, ein Phospholipid, ein Zellrezeptor-bindendes Molekül, ein Vernetzungsmittel, ein wasserlösliches Vitamin, ein Lipid-lösliches Vitamin, ein RNA/DNA spaltender Komplex, ein Porphyrin oder eine polymere Verbindung, ausgewählt aus polymeren Aminen, polymeren Glykolen und Polyethern, ist; und
wobei das Konjugat gegebenenfalls eine verknüpfende Einheit einschließt, mit der Maßgabe, daß das Konjugat nicht Acridin ist.

6. Peptid-Nukleinsäure-Konjugat nach Anspruch 5, wobei mindestens eine Gruppe R¹² oder eine Gruppe R¹³ ein Konjugat ist.

7. Peptid-Nukleinsäure-Konjugat nach Anspruch 5 oder 6, wobei mindestens eines von R¹, R², R³, R⁴, R⁵, R⁶, R⁷, R⁸, R⁹, R¹⁰ oder R¹¹ ein Konjugat ist.

8. Peptid-Nukleinsäure-Konjugat nach Anspruch 7, wobei mindestens eine der A-Aₘ Gruppen mindestens eines von R¹, R² und R³ einschließt.

9. Peptid-Nukleinsäure-Konjugat nach Anspruch 7, wobei mindestens eine der B-Bₘ Gruppen oder der G-Gₘ Gruppen mindestens eine Gruppe R³ einschließt.

10. Peptid-Nukleinsäure-Konjugat nach Anspruch 7, wobei mindestens eine der Gruppen Q oder I mindestens eine der Gruppen R⁸, R⁹, R¹⁰ und R¹¹ einschließt.

11. Peptid-Nukleinsäure-Konjugat nach Anspruch 7, wobei mindestens eine der Gruppen D-Dₘ oder C-Cₘ mindestens eines von R³, R⁴, R⁵, R⁶ und R⁷ einschließt.

12. Peptid-Nukleinsäure-Konjugat nach einem der Ansprüche 5 bis 11, worin:
(i) m von 1 bis etwa 200 ist, oder
(ii) m von 1 bis etwa 50 ist, oder
(iii) m von 1 bis etwa 20 ist.

13. Verbindung mit einer der folgenden Formeln: 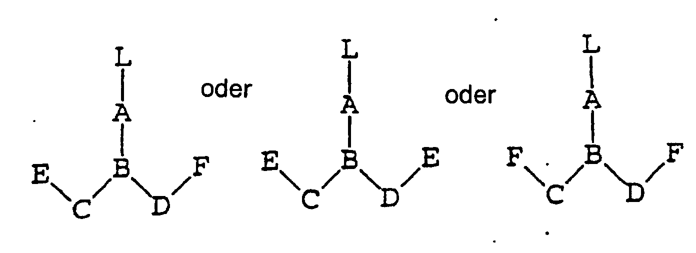 worin:
L R¹²(R¹³)ₐ ist; worin:
R¹² Wasserstoff, Hydroxy, (C₁-C₄)Alkanoyl, eine natürlich vorkommende Nukleobase, eine nicht natürlich vorkommende Nukleobase, eine aromatische Einheit, ein DNA Interkalator, eine Nukleobase-bindende Gruppe, eine heterocyclische Einheit, ein Reporterligand oder ein Konjugat ist und mindestens eines von R¹² eine natürlich vorkommende Nukleobase, eine nicht-natürliche vorkommende Nukleobase, ein DNA Interkalator oder eine Nukleobase-bindende Gruppe ist;
R¹³ ein Konjugat ist; und
a 0 oder 1 ist;
A und B derart ausgewählt sind, daß:
(a) A eine Gruppe der Formel (Ila), (Ilb} oder (Ilc) ist und B N oder R³N⁺ ist; oder
(b) A ein Gruppe der Formel (Ild) und B CH ist; 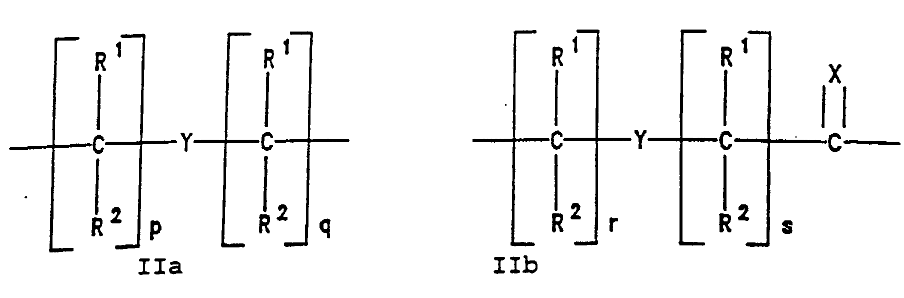 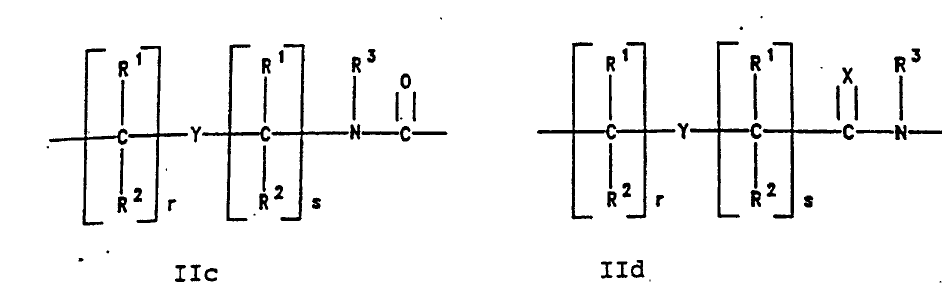 worin:
X O, S, Se, NR³, CH₂ oder C(CH₃)₂ ist;
Y eine Einfachbindung, O, S oder NR⁴ ist;
p und q unabhängig voneinander 0 oder eine ganze Zahl von 1 bis 5 sind, wobei die Summe p + q nicht mehr als 10 beträgt;
r und s unabhängig voneinander Null oder eine ganze Zahl von 1 bis 5 sind, wobei die Summe r+s nicht mehr als 10 beträgt;
R¹ und R² unabhängig voneinander Wasserstoff, (C₁-C₄)Alkyl, Hydroxysubstituiertes (C₁-C₄)Alkyl, Alkoxy-substituiertes (C₁-C₄)Alkyl, Alkylthio-substituiertes (C₁-C₄)Alkyl, Hydroxy, Alkoxy, Alkylthio, Amino, Halogen oder ein Konjugat sind;
C (CR⁶R⁷)_{y} ist;
D (CR⁶R⁷)_{z} ist, worin:
R⁶ und R⁷ unabhängig voneinander Wasserstoff, eine Seitenkette einer natürlich vorkommenden Alphaaminosäure, (C₂-C₆)Alkyl, Aryl, Aralkyl, Heteroaryl, Hydroxy, (C₁-C₆)Alkoxy, (C₁-C₆)Alkylthio, ein Konjugat, NR³R⁴ und SR⁵ sind oder R⁶ und R⁷ zusammengenommen ein alicyclisches oder heterocyclisches System vervollständigen;
R³ und R⁴ unabhängig voneinander Wasserstoff, ein Konjugat, (C₁-C₄)Alkyl, Hydroxy- oder Alkoxy- oder Alkylthio-substituiertes (C₁-C₄)Alkyl, Hydroxy, Alkoxy, Alkylthio oder Amino sind, und
R⁵ Wasserstoff, ein Konjugat, (C₁-C₆)Alkyl, Hydroxy-, Alkoxy- oder Alkylthio-substituiertes (C₁-C₆)Alkyl ist,
y und z jeweils 0 oder eine ganze Zahl von 1 bis 10 sind, wobei die Summe y + z größer als 2, aber nicht mehr als 10 beträgt;
E unabhängig voneinander COOH, CSOH, SOOH, SO₂OH oder ein aktiviertes oder geschütztes Derivat davon ist;
F unabhängig voneinander NHR³ oder NPgR³ ist, wobei Pg eine Aminoschutzgruppe ist; und
mindestens eines von R¹, R², R³, R⁴, R⁵, R⁶, R⁷, R¹² und R¹³ ein Konjugat ist, wobei das Konjugat ein Terpen, ein aromatisches lipophiles Molekül, ein Phospholipid, ein Zellrezeptor-bindendes Molekül, ein Vernetzungsmittel, ein wasserlösliches Vitamin, ein Lipid-lösliches Vitamin, ein RNA/DNA spaltender Komplex, ein Porphyrin oder eine polymere Verbindung, ausgewählt aus polymeren Aminen, polymeren Glykolen und Polyethern, ist, wobei das Konjugat gegebenenfalls eine verknüpfende Einheit einschließt, mit der Maßgabe, daß das Konjugat nicht Acridin ist.

14. Peptid-Nukleinsäure-Konjugat nach Anspruch 13, wobei mindestens eine Gruppe R³ ein Konjugat ist.

15. Peptid-Nukleinsäure-Konjugat nach Anspruch 13 oder 14, wobei mindestens eine der Gruppen A oder der Gruppen B ein Konjugat einschließt.

16. Peptid-Nukleinsäure-Konjugat nach einem der Ansprüche 13 bis 15, wobei mindestens eine der Gruppe R¹ oder Gruppe R² ein Konjugat ist.

17. Peptid-Nukleinsäure-Konjugat nach einem der Ansprüche 13 bis 16, wobei mindestens eine der Gruppen C oder der Gruppen D ein Konjugat einschüeßt.

18. Peptid-Nukleinsäure-Konjugat, umfassend eine Vielzahl von PNA-Monomeren, wobei mindestens eines der PNA-Monomere die Formel aufweist: 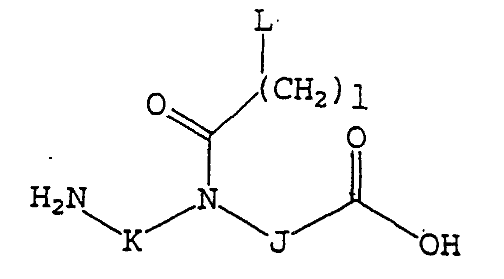 oder Formel: 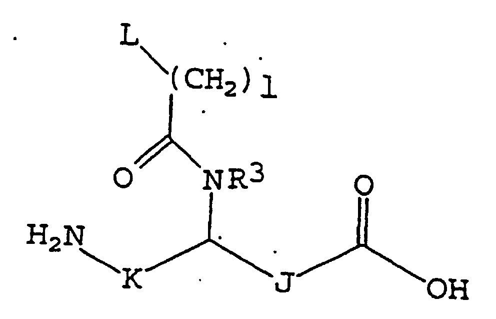 oder Formel: 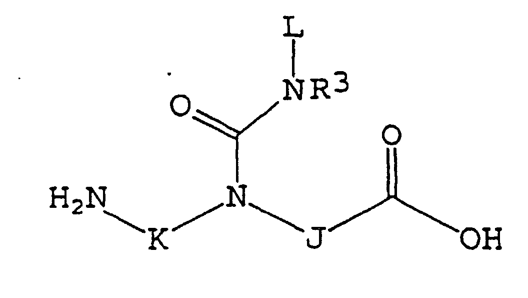 worin:
L R¹²(R¹³)ₐ ist; worin:
R¹² Wasserstoff, Hydroxy, (C₁-C₄)Alkanoyl, eine natürlich vorkommende Nukleobase, eine nicht-natürlich vorkommende Nukleobase, eine aromatische Einheit, ein DNA-Interkalator, eine Nukleobase-bindende Gruppe, ein heterocyclische Einheit, ein Reporterligand oder ein Konjugat ist und mindestens eines von R¹² eine natürlich vorkommende Nukleobase, eine nicht-natürlich vorkommende Nukleobase, ein DNA-Interkalator oder eine Nukleobase-bindende Gruppe ist;
R¹³ ein Konjugat ist; und
a 0 oder 1 ist;
K(CR⁶R⁷)_{z} ist;
J (CR⁶R⁷)_{y} ist, worin
R⁶ und R⁷ unabhängig voneinander Wasserstoff, eine Seitenkette einer natürlich vorkommenden Alphaaminosäure, (C₂-C₆)Alkyl, Aryl, Aralkyl, Heteroaryl, Hydroxy, (C₁-C₆)Alkoxy, (C₁-C₆)Alkylthio, ein Konjugat, NR³R⁴ und SR⁵ sind oder R⁶ und R⁷ zusammengenommen ein alicyclisches oder heterocyclisches System vervollständigen;
R³ und R⁴ unabhängig voneinander Wasserstoff, ein Konjugat, (C₁-C₄)Alkyl, Hydroxy- oder Alkoxy- oder Alkylthio-substituiertes (C₁-C₄)Alkyl, Hydroxy, Alkoxy, Alkylthio oder Amino sind;
R⁵ Wasserstoff, ein Konjugat, (C₁-C₆)Alkyl, Hydroxy-, Alkoxy- oder Alkylthio-substituiertes (C₁-C₆)Alkyl ist; y und z jeweils 0 oder eine ganze Zahl von 1 bis 10 sind, wobei die Summe y + z größer als 2, aber nicht mehr als 10 beträgt;
I eine ganze Zahl von 1 bis 5 ist; und
mindestens eines von R¹, R², R³, R⁴, R⁵, R⁶, R⁷, R¹² und R¹³ ein Konjugat ist, wobei das Konjugat ein Terpen, ein aromatisches lipophiles Molekül, ein Phospholipid, ein Zellrezeptor-bindendes Molekül, ein Vernetzungsmittel, ein wasserlösliches Vitamin, ein Lipid-lösliches Vitamin, ein RNA/DNA spaltender Komplex, ein Porphyrin oder eine polymere Verbindung, ausgewählt aus polymeren Aminen, polymeren Glykolen und Polyethern, ist und wobei das Konjugat gegebenenfalls eine verknüpfende Einheit einschließt, mit der Maßgabe, daß das Konjugat nicht Acridin ist.

19. Peptid-Nukleinsäure-Konjugat nach Anspruch 18, wobei mindestens eine der Gruppe K oder der Gruppe J ein Konjugat einschließt.

20. Peptid-Nukleinsäure-Konjugat nach einem der Ansprüche 13 bis 19, wobei R¹² oder R¹³ ein Konjugat ist.

21. Peptid-Nukleinsäure-Konjugat nach einem der Ansprüche 13 bis 20, wobei mindestens eines von R³, R⁴, R⁵, R⁶ und R⁷ ein Konjugat ist.

## Revendications

1. Conjugué d'acide nucléique peptidique comprenant:
un acide nucléique peptidique;
ledit acide nucléique peptidique possédant un squelette;
ledit squelette ayant une extrémité amino, une extrémité carboxyle et une pluralité de groupes amino;
lesdits groupes amino possédant chacun une nucléobase fixée par une attache; et un conjugué lié audit acide nucléique peptidique soit directement soit par l'intermédiaire d'un groupement de liaison, ledit conjugué étant un terpène, une molécule aromatique lipophile, un phospholipide, une molécule de liaison aux récepteurs cellulaires, un agent réticulant, une vitamine hydrosoluble, une vitamine liposoluble, un complexe de clivage d'ARN/ADN, une porphyrine, ou un composé polymère choisi parmi les amines polymères, les glycols polymères et les polyéthers, pourvu que le conjugué ne soit pas une acridine.

2. Conjugué d'acide nucléique peptidique selon la revendication 1, où ledit conjugué est lié par l'intermédiaire dudit groupement de liaison à au moins l'un parmi ledit squelette, ladite attache, ou ladite nucléobase.

3. Conjugué d'acide nucléique peptidique selon la revendication 1, où ledit conjugué est lié audit squelette, à ladite nucléobase ou à ladite attache.

4. Conjugué d'acide nucléique peptidique selon la revendication 3, où ledit conjugué est lié à au moins l'une parmi ladite extrémité amino ou ladite extrémité carboxyle dudit squelette.

5. Conjugué d'acide nucléique peptidique de formule: 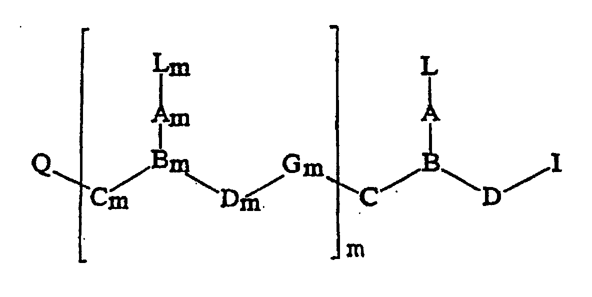 dans laquelle:
m est un nombre entier de 1 à environ 50;
L et Lₘ sont indépendamment R¹²(R¹³)ₐ; où:
R¹² est un atome d'hydrogène, un groupe hydroxy, un groupe alcanoyle en C₁-C₄, une nucléobase d'origine naturelle, une nucléobase d'origine non naturelle, un groupement aromatique, un agent intercalant d'ADN, un groupe de liaison de nucléobase, un groupement hétérocyclique, un ligand indicateur, ou un conjugué;
pourvu qu'au moins l'un des R¹² soit une nucléobase d'origine naturelle, une nucléobase d'origine non naturelle, un agent intercalant d'ADN, ou un groupe de liaison de nucléobase;
R¹³ est un conjugué; et
a vaut 0 ou 1;
C et Cₘ sont indépendamment (CR⁶R⁷)_{y}; où:
R⁶ et R⁷ sont indépendamment un atome d'hydrogène, une chaîne latérale d'un acide alpha-aminé naturel, ou un groupe alkyle en C₂-C₆, aryle, aralkyle, hétéroaryle, hydroxy, alcoxy en C₁-C₆, alkylthio en C₁-C₆, un conjugué, NR³R⁴, SR⁵, ou R⁶ et R⁷, pris conjointement, complètent un système alicyclique ou hétérocyclique;
où R⁵ est un atome d'hydrogène, un conjugué, un groupe alkyle en C₁-C₆, ou un groupe alkyle en C₁-C₆ substitué par des groupes hydroxy, alcoxy ou alkylthio; et
R³ et R⁴ sont indépendamment un atome d'hydrogéne, un conjugué, un groupe alkyle en C₁-C₄, ou un groupe alkyle en C₁-C₄ substitué par des groupes hydroxy, alcoxy ou alkylthio, ou un groupe hydroxy, alcoxy, alkylthio ou amino;
D et Dₘ sont indépendamment (CR⁶R⁷)_{z};
y et z valent chacun zéro ou un nombre entier de 1 à 20, la somme de y + z étant supérieure à 2 mais pas plus de 10;
Gₘ est indépendamment -NR³CO-, -NR³CS-, -NR³SO- ou -NR³SO₂- dans l'une ou l'autre orientation;
chaque paire de A-Aₘ et B-Bₘ est choisie de façon que:
(a) A ou Aₘ est un groupe de formule (IIa), (IIb) ou (IIc) et B ou Bₘ est N ou R³N⁺; ou
(b) A ou Aₘ est un groupe de formule (IId) et B ou Bₘ est CH; 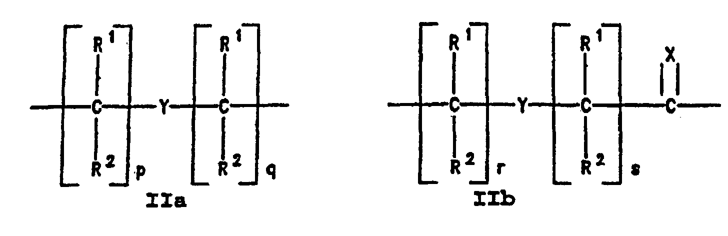 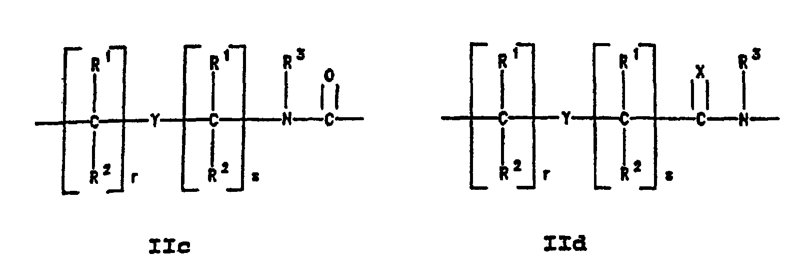 où:
X est O, S, Se, NR³, CH₂ ou C(CH₃)₂;
Y est une liaison simple, O, S ou NR⁴;
p et q valent chacun zéro ou un nombre entier de 1 à 5, la somme de p + q n'étant pas supérieure à 10;
r et s valent chacun zéro ou un nombre entier de 1 à 5, la somme de r + s n'étant pas supérieure à 10;
R¹ et R² représentent indépendamment un atome d'hydrogène, un groupe alkyle en C₁-C₄, un groupe alkyle en C₁-C₄ hydroxylé, un groupe alkyle en C₁-C₄ alcoxylé, un groupe alkyle en C₁-C₄ substitué par un alkylthio, ou un groupe hydroxy, alcoxy, alkylthio, amine, un atome d'halogène ou un conjugué;
I est -NR⁸R⁹ ou -NR¹⁰C(O)R¹¹; où:
R⁸, R⁹, R¹⁰ et R¹¹ sont indépendamment un atome d'hydrogène, un groupe alkyle, un groupe protecteur de groupe amino, un ligand indicateur, un agent intercalant, un chélateur, un peptide, une protéine, un glucide, un lipide, un stéroïde, un nucléoside, un nucléotide, un nucléotidediphosphate, un nucléotide-triphosphate, un oligonucléotide, un oligonucléoside, un polymère soluble, un polymère non soluble ou un conjugué;
Q est -CO₂H, -CO₂R⁸, -CO₂R⁹, -CONR⁸R⁹, -SO₃H, -SO₂NR¹⁰R¹¹ ou un dérivé activé de -CO₂H ou de -SO₃H; et
où l'un au moins parmi R¹, R², R³, R⁴, R⁵, R⁶, R⁷, R⁸, R⁹, R¹⁰, R¹¹, R¹² et R¹³ est un conjugué, où ledit conjugué est un terpène, une molécule aromatique lipophile, un phospholipide, une molécule de liaison aux récepteurs cellulaires, un agent réticulant, une vitamine hydrosoluble, une vitamine liposoluble, un complexe de clivage d'ARN/ADN, une porphyrine, ou un composé polymère choisi parmi les amines polymères, les glycols polymères et les polyéthers; et
où ledit conjugué comporte éventuellement un groupement de liaison, pourvu que le conjugué ne soit pas une acridine.

6. Conjugué d'acide nucléique peptidique selon la revendication 5, dans lequel au moins un groupe R¹² ou un groupe R¹³ est un conjugué.

7. Conjugué d'acide nucléique peptidique selon la revendication 5 ou la revendication 6, dans lequel l'un au moins parmi R¹, R², R³, R⁴, R⁵, R⁶, R⁷, R⁸, R⁹, R¹⁰ ou R¹¹ est un conjugué.

8. Conjugué d'acide nucléique peptidique selon la revendication 7, dans lequel au moins l'un desdits groupes A-Aₘ inclut au moins l'un parmi R¹, R² et R³.

9. Conjugué d'acide nucléique peptidique selon la revendication 7, dans lequel au moins l'un desdits groupes B-Bₘ ou desdits groupes G-Gₘ inclut au moins un groupe R³.

10. Conjugué d'acide nucléique peptidique selon la revendication 7, dans lequel au moins l'un desdits groupes Q ou I inclut au moins l'un des groupes R⁸, R⁹, R¹⁰ et R¹¹.

11. Conjugué d'acide nucléique peptidique selon la revendication 7, dans lequel au moins l'un desdits groupes D-Dₘ, ou C-Cₘ inclut au moins l'un des groupes R³, R⁴, R⁵, R⁶ et R⁷.

12. Conjugué d'acide nucléique peptidique selon l'une quelconque des revendications 5 à 11, dans lequel:
(i) m vaut de 1 à environ 200, ou
(ii) m vaut de 1 à environ 50, ou
(iii) m vaut de 1 à environ 20.

13. Composé répondant à l'une des formules suivantes: 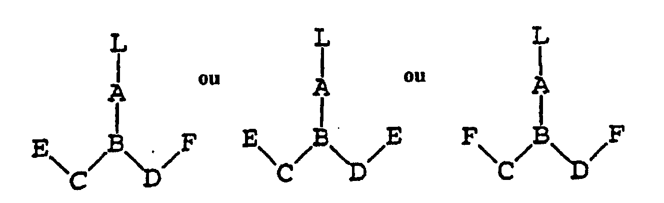 dans lesquelles:
L est R¹²(R¹³)ₐ; où:
R¹² est un atome d'hydrogène, un groupe hydroxy, un groupe alcanoyle en C₁-C₄, une nucléobase d'origine naturelle, une nucléobase d'origine non naturelle, un groupement aromatique, un agent intercalant d'ADN, un groupe de liaison de nucléobase, un groupement hétérocyclique, un ligand indicateur, ou un conjugué; et au moins l'un des R¹² est une nucléobase d'origine naturelle, une nucléobase d'origine non naturelle, un agent intercalant d'ADN ou un groupe de liaison de nucléobase;
R¹³ est un conjugué ; et
a vaut 0 ou 1;
A et B sont choisis de telle façon que:
(a) A est un groupe de formule (IIa), (IIb) ou (IIc) et B est N ou R³N⁺; ou
(b) A est un groupe de formule (IId) et B est CH; 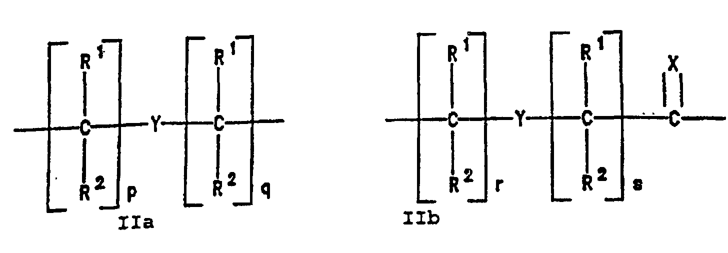 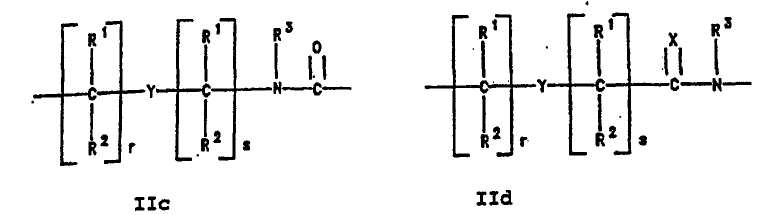 où:
X est O, S, Se, NR³, CH₂ ou C(CH₃)₂;
Y est une liaison simple, O, S ou NR⁴;
p et q valent chacun indépendamment zéro ou un nombre entier de 1 à 5, la somme de p + q n'étant pas supérieure à 10;
r et s valent chacun indépendamment zéro ou un nombre entier de 1 à 5, la somme de r + s n'étant pas supérieure à 10;
R¹ et R² représentent indépendamment un atome d'hydrogène, un groupe alkyle en C₁-C₄, un groupe alkyle en C₁-C₄ hydroxylé, un groupe alkyle en C₁-C₄ alcoxylé, un groupe alkyle en C₁-C₄ substitué par un alkylthio, ou un groupe hydroxy, alcoxy, alkylthio, amino, un atome d'halogène ou un conjugué;
C est (CR⁶R⁷)_{y};
D est (CR⁶R⁷)_{z}; où:
R⁶ et R⁷ sont indépendamment un atome d'hydrogène, une chaîne latérale d'un acide alpha-aminé naturel, ou un groupe alkyle en C₂-C₆, aryle, aralkyle, hétéroaryle, hydroxy, alcoxy en C₁-C₆, alkylthio en C₁-C₆, un conjugué, NR³R⁴et SR⁵, ou
R⁶ et R⁷, pris conjointement, complètent un système alicyclique ou hétérocyclique;
R³ et R⁴ sont indépendamment un atome d'hydrogène, un conjugué, un groupe alkyle en C₁-C₄, un groupe alkyle en C₁-C₄ substitué par un groupe hydroxy ou alcoxy ou alkylthio, un groupe hydroxy, alcoxy, alkylthio ou amino; et
R⁵ est un atome d'hydrogène, un conjugué, un groupe alkyle en C₁-C₆, un groupe alkyle en C₁-C₆ substitué par un groupe hydroxy, alcoxy ou alkylthio;
y et z valent chacun zéro ou un nombre entier de 1 à 10, la somme de y + z étant supérieure à 2 mais pas plus de 10;
E est indépendamment COOH, CSOH, SOOH, SO₂OH ou un dérivé activé ou protégé de ceux-ci;
F est indépendamment NHR³ ou NPgR³, où Pg est un groupe protecteur de groupe amino; et
l'un au moins parmi R¹, R², R³, R⁴, R⁵, R⁶, R⁷, R¹² et R¹³ est un conjugué, où ledit conjugué est un terpène, une molécule aromatique lipophile, un phospholipide, une molécule de liaison aux récepteurs cellulaires, un agent réticulant, une vitamine hydrosoluble, une vitamine liposoluble, un complexe de clivage d'ARN/ADN, une porphyrine, ou un composé polymère choisi parmi les amines polymères, les glycols polymères et les polyéthers; et où ledit conjugué comporte éventuellement un groupement de liaison, pourvu que le conjugué ne soit pas une acridine.

14. Conjugué d'acide nucléique peptidique selon la revendication 13, dans lequel au moins un groupe R³ est un conjugué.

15. Conjugué d'acide nucléique peptidique selon la revendication 13 ou 14, dans lequel au moins l'un desdits groupes A ou desdits groupes B inclut un conjugué.

16. Conjugué d'acide nucléique peptidique selon l'une quelconque des revendications 13 à 15, dans lequel au moins l'un des groupes R¹ ou R² est un conjugué.

17. Conjugué d'acide nucléique peptidique selon l'une quelconque des revendications 13 à 16, dans lequel au moins l'un desdits groupes C ou desdits groupes D inclut un conjugué.

18. Conjugué d'acide nucléique peptidique comprenant une pluralité de monomères de PNA où au moins l'un desdits monomères de PNA répond à la formule: 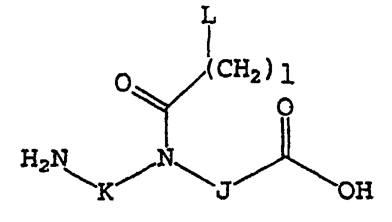 ou à la formule: 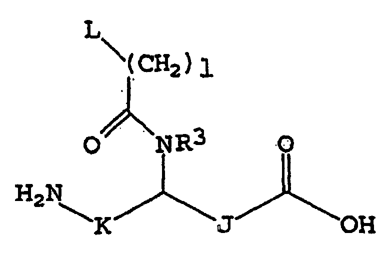 ou à la formule: 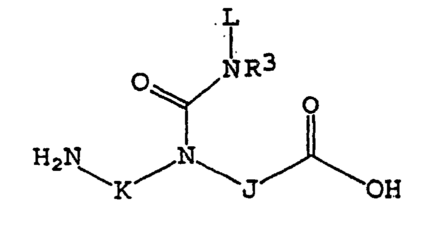 où:
L est R¹²(R¹³)ₐ; où:
R¹² est un atome d'hydrogène, un groupe hydroxy, un groupe alcanoyle en C₁-C₄, une nucléobase d'origine naturelle, une nucléobase d'origine non naturelle, un groupement aromatique, un agent intercalant d'ADN, un groupe de liaison de nucléobase, un groupement hétérocyclique, un ligand indicateur, ou un conjugué; et au moins l'un des R¹² est une nucléobase d'origine naturelle, une nucléobase d'origine non naturelle, un agent intercalant d'ADN ou un groupe de liaison de nucléobase;
R¹³ est un conjugué ; et
a vaut 0 ou 1;
K est (CR⁶R⁷)_{z};
J est (CR⁶R⁷)_{y}; où
R⁶ et R⁷ sont indépendamment un atome d'hydrogène, une chaîne latérale d'un acide alpha-aminé naturel, ou un groupe alkyle en C₂-C₆, aryle, aralkyle, hétéroaryle, hydroxy, alcoxy en C₁-C₆, alkylthio en C₁-C₆, un conjugué, NR³R⁴ et SR⁵, ou
R⁶ et R⁷, pris conjointement, complètent un système alicyclique ou hétérocyclique;
R³ et R⁴ sont indépendamment un atome d'hydrogène, un conjugué, un groupe alkyle en C₁-C₄, ou un groupe alkyle en C₁-C₄ substitué par des groupes hydroxy, alcoxy ou alkylthio, ou un groupe hydroxy, alcoxy, alkylthio ou amino;
R⁵ est un atome d'hydrogène, un conjugué, un groupe alkyle en C₁-C₆, ou un groupe alkyle en C₁-C₆ substitué par des groupes hydroxy, alcoxy ou alkylthio;
y et z valent chacun zéro ou un nombre entier de 1 à 10, la somme de
y + z étant supérieure à 2 mais pas plus de 10;
l est un nombre entier de 1 à 5; et
l'un au moins parmi R¹, R², R³, R⁴, R⁵, R⁶, R⁷, R¹² et R¹³ est un conjugué, où ledit conjugué est un terpène, une molécule aromatique lipophile, un phospholipide, une molécule de liaison aux récepteurs cellulaires, un agent réticulant, une vitamine hydrosoluble, une vitamine liposoluble, un complexe de clivage d'ARN/ADN, une porphyrine, ou un composé polymère choisi parmi les amines polymères, les glycols polymères et les polyéthers et où ledit conjugué comporte éventuellement un groupement de liaison, pourvu que le conjugué ne soit pas une acridine.

19. Conjugué d'acide nucléique peptidique selon la revendication 18, dans lequel au moins l'un parmi ledit groupe K ou ledit groupe J inclut un conjugué.

20. Conjugué d'acide nucléique peptidique selon l'une quelconque des revendications 13 à 19, où R¹² ou R¹³ est un conjugué.

21. Conjugué d'acide nucléique peptidique selon l'une quelconque des revendications 13 à 20, où au moins l'un parmi R³, R⁴, R⁵, R⁶ et R⁷ est un conjugué.
